# EUROPEAN PATENT APPLICATION

(11) **EP 4 636 767 A2**
(43) Date of publication of application: **22.10.2025**
(21) Application number: 25179145.5
(22) Date of filing: 20.12.2019
(51) Int. Cl.: G11C 13/00

(54) **METHOD OF ENCODING DATA ON A POLYNUCLEOTIDE STRAND**

(30) Priority: 21.12.2018 GB 201821155
(62) Divisional of application: 19835473.0
(71) Applicant: Oxford Nanopore Technologies PLC, Oxford OX4 4DQ (GB)
(72) Inventor: BROWN, Clive Gavin, Oxford, OX4 4DQ (GB); HERON, Andrew John, Oxford, OX4 4DQ (GB); GRAHAM, James Edward, Oxford, OX4 4DQ (GB)
(74) Representative: J A Kemp LLP

(57) **Abstract**

Provided herein are methods of encoding data on a polymer. Also provided are methods of reading data encoded on a polymer. Also provided are systems for encoding data on a polymer; systems for reading data encoded on a polymer; and data encoding / data reading platforms.

## Description

### FIELD OF THE INVENTION

The invention relates generally to methods of encoding data on a polynucleotide. The invention also relates to methods of reading data encoded on a polynucleotide. The methods involve moving the polynucleotide strand with respect to a nanoreactor; and selectively modifying portions of the polynucleotide strand as they move through the nanoreactor. The pattern of selective modifications on the polynucleotide strand encodes data on the polynucleotide strand. The invention also relates generally to systems for encoding data on a polynucleotide strand; for reading data encoded on a polynucleotide strand; and to data encoding / data reading platforms.

### BACKGROUND

There is an ongoing need to store data. Some calculations have predicted worldwide data storage needs in 2025 as around 175 zettabytes. Such storage demands will place increasing reliance on new technologies to efficiently encode and retrieve data.

There is further a need to store data efficiently over prolonged time scales. However, the physical media used to store data today are prone to deterioration over time with associated loss of data fidelity.

One approach that has been proposed to address this need is to use polynucleotides such as DNA to encode data for long-term data storage.

The concept of storing data on polynucleotides such as DNA was initially hypothesized as long ago as the 1960s and in recent years there have been a number of efforts to demonstrate feasible systems for reading and writing data onto DNA.

Most attempts to date have focussed on de novo synthesis of DNA sequences which encode data. For example, binary data may be encoded in blocks of DNA. The DNA blocks may be synthesized in vitro by conventional means such as phosphoramidate chemistry. Such DNA blocks may include auxiliary information such as address sequences, amplification or sequencing tags, and the like. To encode large amounts of data, multiple blocks are used with the blocks being ordered according to their address sequences. However, such encoding steps are very slow and costly. In particular, such encoding steps may be limited by the synthesis of the DNA. Various approaches have been proposed for decoding data thus encoded. Some approaches have involved amplification of the DNA following by conventional sequencing technologies, such as Sanger sequencing. However, such approaches are not feasible for large-scale data storage and readout as throughput speed is limited; plus the amplification steps involved can introduce errors. Next-generation sequencing methods have been proposed but do not overcome the inherent limitations in the encoding/synthesis steps.

There is thus a need for improved methods of data storage using polynucleotides such as DNA. There is a particular need for methods which avoid the disadvantages associated with known methods, such as the requirement to synthesize blocks of DNA by de novo synthesis; to combine DNA blocks to allow storage of large amounts of data; or to amplify DNA for data retrieval. The present invention address some or all of these needs.

### SUMMARY

The inventors have recognised that data can be encoded on a polynucleotide strand by controlling the movement of the polynucleotide strand with respect to a nanoreactor, and selectively modifying portions of the polynucleotide strand as they move within the nanoreactor. The modification of the polynucleotide strand determines the characteristics of the signal obtained when the modified strand is analysed. The modifications to the strand can thus encode data on the strand.

The inventors have found that confining the portion of the polynucleotide strand to be modified in a nanoreactor is particularly beneficial. The controlled movement of the portions of the strand through the nanoreactor allows controlled modification of specific regions of the strand with extremely high accuracy. The specific regions thus modified allow data to be encoded on the strand. Furthermore, the polynucleotide strand can be modified reliably and at a high rate.

The methods of the invention thus take advantage of the exquisite control possible by confining the polynucleotide into a nanoreactor. For example, the nanoreactor may confine the polynucleotide into a single dimension so that modification to the polynucleotide is constrained into that dimension. The controlled movement and position of the polynucleotide within the nanoreactor allows data to be encoded on the polynucleotide strand with extremely high precision. The combination of control and rate is typically far superior to prior art techniques that involve de novo synthesis of polynucleotide for encoding data.

Long write lengths are possible using the methods of the invention. Despite some limited improvements over recent years, long strands of DNA can still not be reliably synthesized de novo in contiguous units. Rather, such strands are typically assembled together post-synthesis from smaller pieces. The methods of the invention do not have such limitations. Long polynucleotide strands can be moved with respect to a nanoreactor and portions of the polynucleotide strands can be selectively modified as they move through the nanoreactor. Thus, strands of kilobase, megabase or even tens or hundreds of megabases in length can be addressed in the methods of the invention.

As discussed in more detail below, the polynucleotide strands used to encode data in the invention can be naturally occurring (e.g. extracted from naturally-occurring or modified organisms) or can be synthetically produced. However, even when synthetic polynucleotide strands are used in the methods of the invention, there is no requirements for those strands to be synthesized monomer-by-monomer. By contrast, long polynucleotide strands can be produced by concatenating long repeat units. Such reactions are far simpler and more readily scalable than techniques which require the controlled assembly of different polynucleotide strands. In other words, the methods of the invention are advantageous as they do not require multiple different small units of polynucleotides to be synthesized and subsequently combined, or polynucleotides to be synthesized "base-by-base" in order to encode data.

The methods of the invention allow naturally or synthetically produced polynucleotides to be used as the data storage medium and modifications to be made in order to encode data on the polynucleotide. The methods of the invention allow data to be encoded at extremely high efficiency. The controlled movement of a polynucleotide strand through a nanoreactor can be extremely fast. For example, a polynucleotide strand can be controllably passed through a nanopore at a rate of hundreds or thousands of bases per second, or even faster. Moreover, the process can be scaled almost infinitely, by using an array of individually addressable nanoreactors. For example, an array of nanoreactors may contain from hundreds to thousands or even millions of independently addressable nanoreactors. Furthermore, the ability to use very long polynucleotide strands also means of course that more data can be encoded.

A further advantage of the methods of the invention is that by encoding data onto a polynucleotide strand the density of data recorded can be very high. Embodiments of the invention in which the data encoded on the polynucleotide strand is read at the single molecule level may also allow the data to be read at extremely high accuracy.

Accordingly, provided herein is a method of encoding data on a polynucleotide strand, comprising:
(A) moving the polynucleotide strand with respect to a nanoreactor; and
(B) selectively modifying portions of the polynucleotide strand as they move through the nanoreactor;
wherein the pattern of selective modifications on the polynucleotide strand encodes data on the strand.

The strand is controllably moved with respect to the nanoreactor. The portions of the polynucleotide strand which are selectively modified typically correspond to data units thereby encoded.

Typically, the nanoreactor comprises a nanopore. The nanoreactor may comprise a nanovolume around a nanopore. The nanopore may be a transmembrane protein nanopore, a solid state nanopore, a DNA-origami pore, or a polymer-based plastic pore, for example the nanopore may be a transmembrane β-barrel protein pore.

The invention involves modifying the polynucleotide strand. The modifications made to the polynucleotide strand typically do not alter the overall length of the polynucleotide strand; i.e. data is encoded on the polynucleotide strand backbone rather than involving the addition or removal of monomer units from the polynucleotide strand.

Any suitable modifications can be made to the polynucleotide strand. For example, the modifications may be epigenetic modifications. Selectively modifying portions of the polynucleotide strand may comprise subjecting the portions of the polynucleotide strand in the nanoreactor to reaction conditions comprising (i) the presence, absence or concentration of one or more chemical reagent(s); (ii) the engagement of an enzyme with the polynucleotide strand under conditions that the enzyme modifies the nucleotides within the polynucleotide strand; (iii) the presence or absence of electromagnetic radiation; and/or (iv) the presence or absence of applied heat.

The methods of the invention may further comprise the step of:
(C) determining the pattern of selective modifications on the polynucleotide strand.

In such methods, determining the pattern of selective modifications on the polynucleotide strand often comprises determining the presence, absence, extent or properties of modifications made to the polynucleotide strand. In such a manner, data encoded on the polynucleotide strand can be read. The methods of the invention may comprise characterizing the modified polynucleotide strand. The characteristics of the polynucleotide strand can be determined in addition to the data encoded on the polynucleotide strand. For example the sequence of the polynucleotide strand can be determined. Determining the pattern of selective modifications on the polynucleotide strand and/or characterizing the modified polynucleotide strand typically comprises contacting a detector with the modified polynucleotide strand such that the polynucleotide strand moves with respect to the detector; and taking one or more measurements which are indicative of one or more properties of the modified polynucleotide strand as the polynucleotide strand moves with respect to the detector. The detector is often a transmembrane pore; and in embodiments of the invention in which the nanoreactor is a transmembrane pore the detector may be the same transmembrane pore as the nanoreactor.

Also provided herein is
- A method of modifying a polynucleotide strand, comprising:
   (A) moving the polynucleotide strand with respect to a nanoreactor; and
   (B) selectively modifying portions of the polynucleotide strand as they move through the nanoreactor;
   wherein optionally the method, polynucleotide strand, nanoreactor and/or selective modifications made are as defined in any one of the preceding claims.
- A system for encoding data on a polynucleotide strand, the system comprising a nanoreactor configured to selectively modify portions of a polynucleotide strand as the polynucleotide strand moves through the nanoreactor;
- A system for reading data encoded on a polynucleotide strand; the system comprising a detector configured to determine the pattern of selective modifications on the polynucleotide strand as the polynucleotide strand moves with respect to the detector;
- A data encoding / data reading platform comprising:
   i) a nanoreactor configured to selectively modify portions of a polynucleotide strand as the polynucleotide strand moves through the nanoreactor;
   ii) optional storage for storing the polynucleotide strand once modified in the nanoreactor; and
   iii) a detector configured to determine the pattern of selective modifications on the polynucleotide strand as the polynucleotide strand moves with respect to the detector;
- Use of a nanopore and/or a nanovolume around a nanopore as a nanoreactor for selectively modifying portions of a polynucleotide strand as the polynucleotide strand moves with respect to the nanopore; and
- A data storage medium comprising a selectively modified polynucleotide, wherein said modified polynucleotide is obtainable using a method of the invention.

The systems disclosed herein allow the rapid reading of such modified (encoded) polynucleotides and permit the rapid retrieval of data stored therein.

Also provided herein is a method of selectively modifying a polynucleotide strand within a nanoreactor. In some embodiments the polynucleotide strand and nanoreactor are as described herein.

### DESCRIPTION OF THE FIGURES

It is to be understood that the Figures are for illustration purposes and are not intended to be limiting.

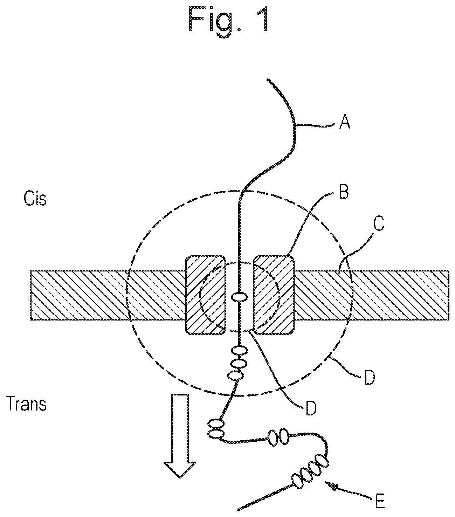

Figure 1 shows a schematic of a nanoreactor for selectively modifying a polymer, such as DNA. The figure shows how a polymer (A) such as a polynucleotide (as described in more detail herein) can be contacted with a nanoreactor under conditions that a region of polynucleotide within the nanoreactor is modified. For example, the nanoreactor may be a nanopore (B) in a membrane (C) separating two compartments labelled cis and trans. As described in more detail herein, a nanopore may be a nanochannel, nanoslit, nanocavity etc in the membrane. The nanoreactor may be the nanovolume around the nanopore. The nanovolume around the nanopore is also known as a reaction region (D). The polymer is moved with respect to the nanoreactor so that different regions of the polymer can be exposed to the reaction region over time. For example, the polymer can be moved with respect to a nanopore by translocating the polymer through the nanopore across the membrane. Controlled movement can be achieved, for example, by various means known in the art, including with an applied chemical potential across the membrane, an applied voltage across the membrane, an enzyme motor (not shown for clarity). The region of the polymer in the reaction region can be modified by chemical means in or near to the channel of the nanopore as it translocates through the nanopore. Therefore, by controlling the movement of the polymer and the timing of the chemical reaction it is possible to selectively modify the polymer to produce a modified polymer (E) (e.g. modified DNA). Control of the modification positions on the polymer can be used to encode data for the purposes of data storage. For example, data can be encoded by selectively modifying a pattern of modifications vs unmodified data into the polymer.

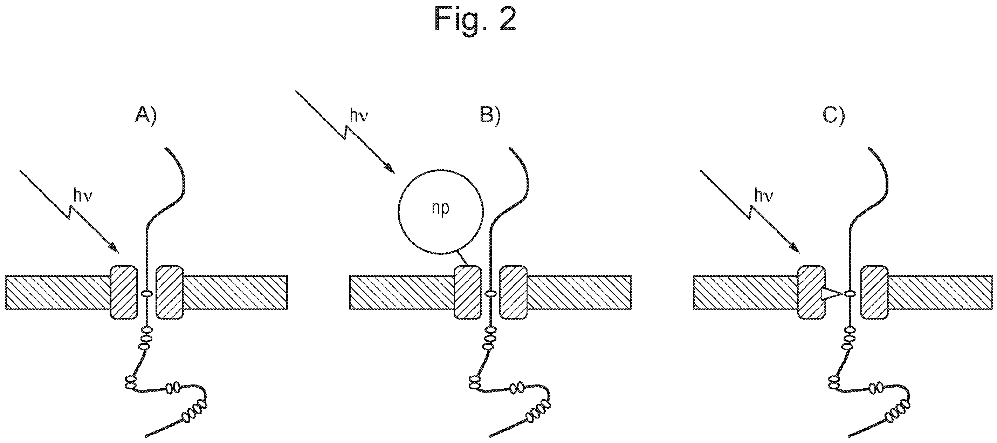

**Figure 2** illustrates some exemplary ways in which electromagnetic radiation such as light can be used to control the chemical reaction to modify a polynucleotide. (A) electromagnetic radiation can be applied to the nanoreactor (e.g. to a nanopore or to a volume around a nanopore), wherein a change of conditions occurs inside the nanoreactor to promote reactivity of the region of polynucleotide therein, thereby modifying the polynucleotide within the nanoreactor. (B) electromagnetic radiation can be applied to the nanoreactor (e.g. to a nanopore or to a volume around a nanopore) with an attached photosensitizer (eg. a nanoparticle, labelled np in figure 2, such as gold or a quantum dot). The activated photosensitizer creates a localised change in conditions enabling reactive modification of the nearby portion of the polynucleotide. (C) electromagnetic radiation can be applied to photosensitizer that is immobilised inside a nanoreactor (e.g. to a nanopore or to a volume around a nanopore).

Irradiation of a nanoreactor, such as those described, can be used to create different changes in the local reaction region. For example, gold particles or gold plasmonic guides can be used to create localised heating. Gold particles or gold plasmonic guides can also be used to change the local electrical conditions, changing the interaction of nearby charged molecules (such as a polynucleotides) and ions in solution. Alternatively, quantum dot particles or many organic molecules can be used to re-emit radiation. Irradiation of quantum dots or many organic molecules can also produce reactive oxygen species (ROS) in the presence or oxidative chemicals. The changes in local conditions can be used directly, or combined in with reactive species in the solution, to control reactivity in the nearby reaction region to selectively modify a portion of the polynucleotide strand.

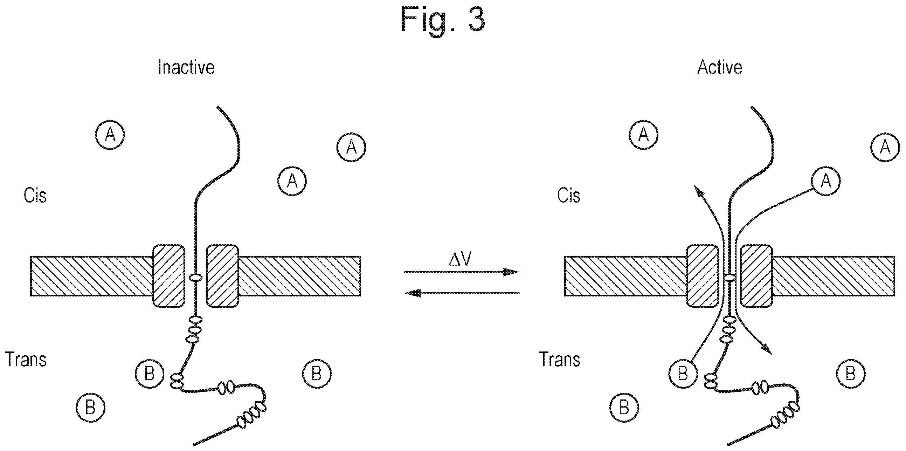

**Figure 3** illustrates one example of using voltage to control the delivery of chemical reagents to or through a nanoreactor (e.g. a nanopore) for the purposes of controlling a chemical reaction to modify the polymer in the nanoreactor. The figure illustrates a simplified example of two cross-reactive reactants (A) and (B) that are initially separated, for example on opposite sides of a membrane, so that no reaction can occur (labelled inactive state). Alteration of the ionic conditions through the nanoreactor (e.g. nanopore), for example by adjusting the applied voltage across the membrane (labelled as active state in Figure 3), can be used to drive the reactants A and B into the nanoreactor (depicted in Figure 3 as a nanopore, although this is not limiting).

Reactants A and B are intended to be illustrative and not limiting. There may a single type of reactive species in the system, or multiple types of reactive species. Some reactive species may be co-located initially outside the nanoreactor (for example oxidants may be colocated with protective scavengers). Each type or reactant may be a single entity (eg. a motor enzyme or a reactive enzyme), or multiple molecules (eg. ions dissolved in relatively high concentration).

Enabling co-location of the reactants with each other in the active state can be used to control the movement of the polynucleotide. For example, voltage can be applied to drive metal cofactor ions (eg. reactant A) through to a motor enzyme on the opposite side of the nanopore (eg. reactant B, preferably as a single enzyme bound to polynucleotide, not shown in figure) to enable NTP hydrolysis to control the translocation of the polynucleotide

Enabling co-location of the reactants with each other in the active state can be used to create reactive conditions to selectively modify the portion of the polynucleotide in the reaction region. Examples of reactants to modify the polynucleotide include for example combining ions (eg. metal cofactor ions) with reactive small molecules (eg. single-electron oxidants), or combining ions (eg. metal cofactor ions) with reactive enzymes (eg. DNA-methyltransferases).

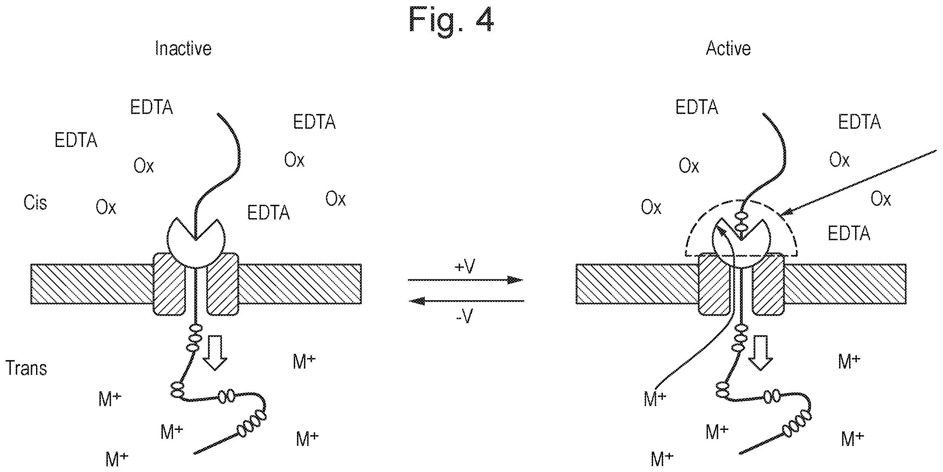

**Figure 4** illustrates one embodiment of the use of voltage used to control the concentration of chemical reactants in a nanoreactor such as a nanopore. In Figure 4, a positive voltage applied to the trans compartment drives the flow of positively charged cofactor metal ions (labelled M⁺, eg. Cu⁺ ions, which are different from any ions that are required for current flow, such as monovalent K⁺, Na⁺ ions, which do not mediate the reactive chemistry) from the trans to the cis chamber (labelled Active state). At low or negative voltage (applied to trans compartment) the M⁺ ions cannot flow from trans to cis, and no chemistry occurs to modify the polymer. At high positive voltage (applied to trans compartment) the M⁺ ions can flow through the nanopore to the cis chamber. The system can be configured so that a threshold voltage is required to begin to drive the reactive cofactor ions (eg. Cu⁺) through the nanopore, so that little or no M⁺ ions flow until a high enough positive voltage is applied to the trans chamber. Similarly, the applied voltage drives the flow of negatively charged oxidants (Ox) from the cis to the trans chamber. At low or negative voltage (applied to trans compartment) the Ox ions cannot flow from cis to trans, and no chemistry occurs to modify the polymer. At high positive voltage (applied to trans compartment) the Ox ions can flow through the nanopore to the trans chamber. As depicted, the M⁺ and Ox ions react with the polynucleotide in the nanoreactor (i.e. in the nanopore) thereby modifying the polynucleotide.

Figure 4 also shows how on passing through to the cis compartment the M⁺ ions may be quenched by protectants in the cis compartment. For example, in the exemplary scheme shown in Figure 4, M⁺ ions can be chelated by a large excess of EDTA, thus limiting the time for which the ions can be involved in reactive chemistries. This creates a reactive hotspot region in the nanopore and near the cis exit of the nanopore where there is an increased probability of the M⁺ ion mediating reaction of the polymer by acting as a required cofactor, eg. as a cofactor for a reactive oxidant to produce single electron oxidation radicals, which can be used for example to selectively oxidise deoxyguanosine bases of DNA. This reactive hotspot region can be identified with a nanovolume as defined herein and which constitutes the nanoreactor for modifying the polynucleotide.

The voltage control of reactivity to selectively modify portions of the polynucleotide in the nanopore can be combined with approximately constant controlled movement of the polynucleotide through the nanopore to encode patterns of modifications into the polynucleotide, for example to encode data. For example, Figure 4 illustrates how a polynucleotide can be moved through the nanopore with a processive enzyme motor, such as a helicase, at an approximately constant speed, and the voltage can be modulated high/low to encode patterns of modified/unmodified respectively into the polynucleotide. In this system it is necessary to ensure there is limited cross reactivity between the reactive cofactors M+/Ox and the enzyme fuel cofactor M+/NTP for NTP hydrolysis. Alternatively, the polynucleotide could be controlled by non-enzymatic, voltage independent, means, such as via magnetic beads.

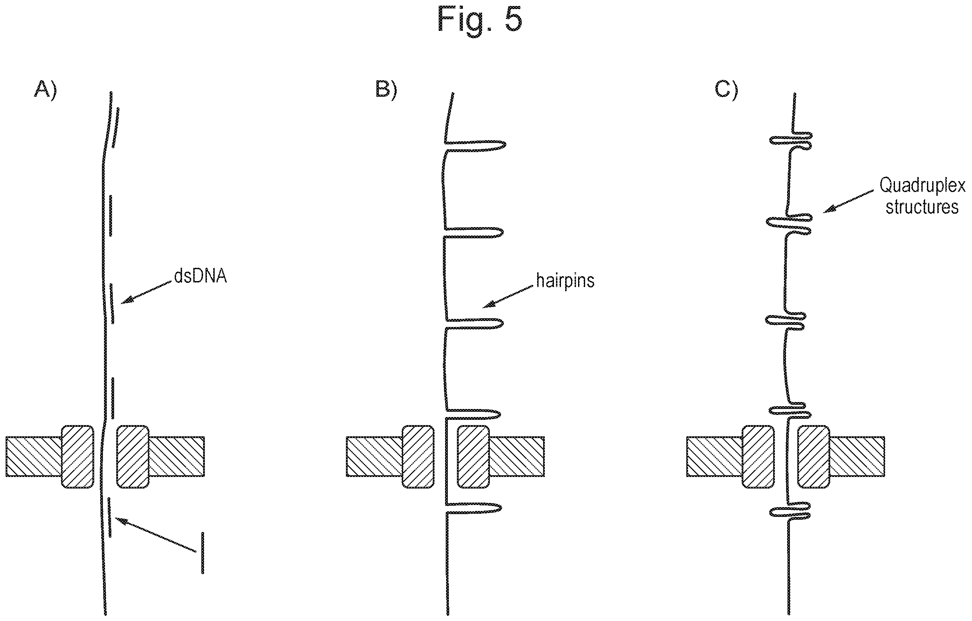

**Figure 5** shows some examples of how polynucleotide movement can be controlled through a nanoreactor such as a nanopore through use of secondary structure that is too large to pass through the pore intact. When encountering the pore the secondary structure causes the strand to pause, until sufficient force, for example by an applied voltage, is applied to overcome hybridisation and unwind the strand, whereupon it can slip to the next secondary structure element in the sequence. Repetitive sequences such as this can be designed using known methods, and constructed by synthetic means, for example by concatenating together units of the same small synthetic oligonucleotide. A) Short sections of double-stranded polynucleotide, where the double-strand is too large to pass, can be used to pause the polynucleotide. B) Hairpins in the polynucleotide can be used to pause the polynucleotide. C) quadruplex structures in the polynucleotide can be used to pause the polynucleotide. The strength of the structure can be altered to control the amount of force required to unwind the structure. Therefore, under low voltage the structure can immobilise the polynucleotide indefinitely. At high voltage the secondary structure can be quickly unwound to advance the strand. In the intermediate voltage regime the secondary structure will pause for a period before advancing.

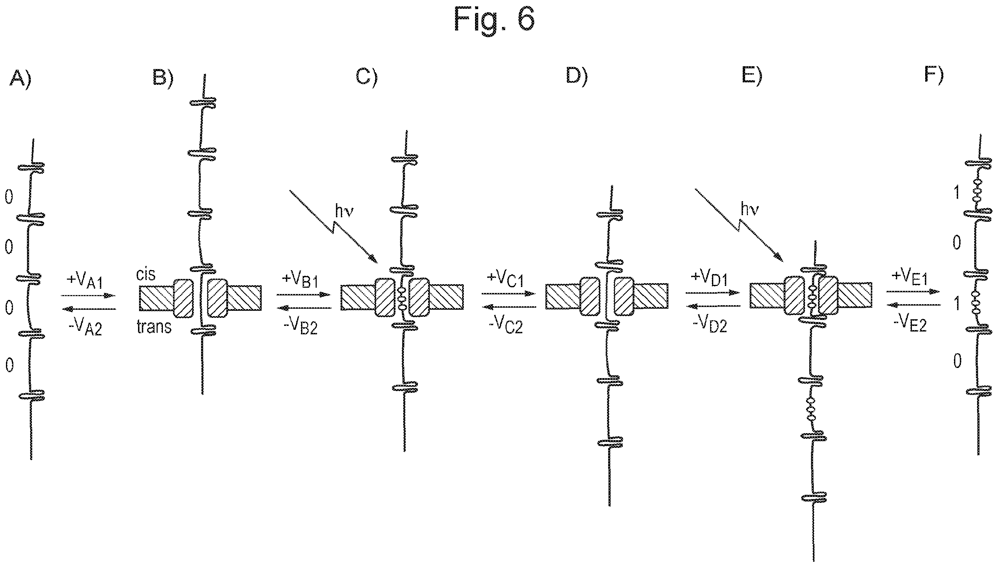

**Figure 6** shows how control of movement with secondary structure (quadruplex), can combined with light based modification to selectively modify regions of a polynucleotide. By feedback control of voltage, combined with careful design of secondary structure elements, it is possible to control the movement of the polynucleotide, advancing the polynucleotide either backwards or forwards between regions of the polynucleotide. When paused these regions can then be selectively modified, by the irradiation with light for example, to create a pattern of modifications in the polynucleotide.

It is also possible in this scheme to move the polynucleotide backwards again. This can be used to repeat a failed modification for example, or to re-read the strand to read the data encoded on it.

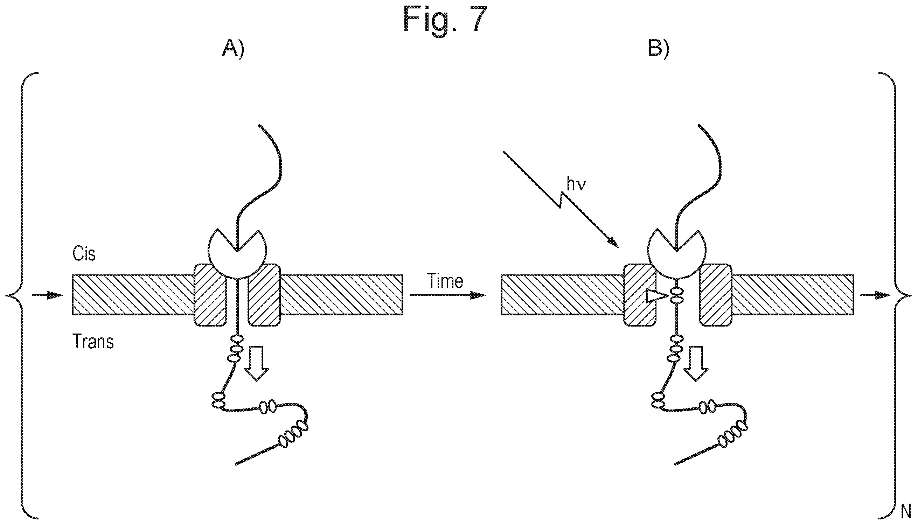

**Figure 7** illustrates an example of where voltage can be used to selectively control movement of the polynucleotide while the modification (by irradiation with light) occurs at a constant regular period. The figure shows a polynucleotide pulled through a nanopore, where the movement is slowed by a passive molecular brake. Under low voltage the movement can be slowed or paused, and at high voltage the polynucleotide can be pulled quickly through the enzyme brake. In this way it is possible to advance and pause the polynucleotide in the nanopore as desired to control the portion of the polynucleotide exposed to the reactive region. The positional control combined with a periodic modification condition at a constant frequency (eg. irradiation with light to create reactive oxygen species in the nanopore that modify nucleotides, labelled as A when the light is off and B when the light is on in figure 7) enables creation of a controlled pattern of modifications along the polynucleotide.

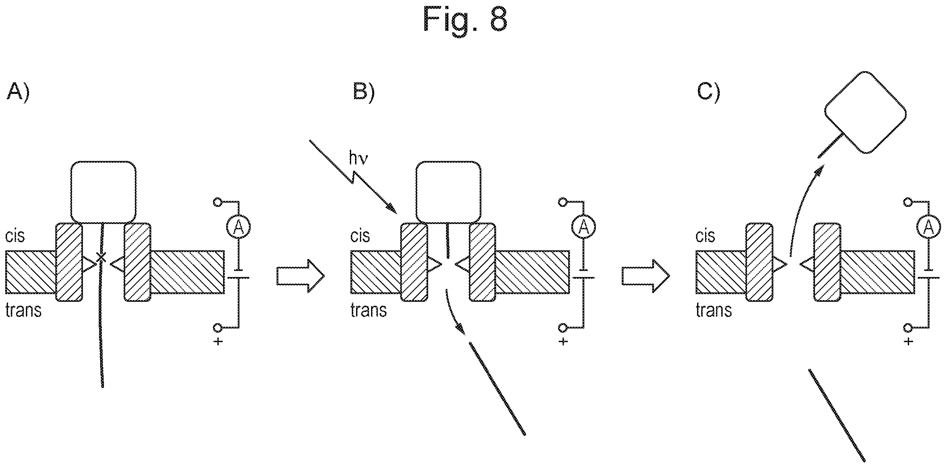

**Figure 8** schematically illustrates the scission of a linker in a nanopore nanoreactor, leading to strand cleavage. **Figure 8A** shows an initial state represented by capture of a capped DNA strand, e.g. a DNA strand terminated by streptavidin, in the nanopore. When irradiated with light at the correct frequency the linker cleaves **(****Figure 8B****),** which leads to spontaneous strand loss shortly afterwards **(****Figure 8C****)** as the strand is too short to remain bound under the applied voltage conditions. The now unoccupied pore then captures a new intact substrate from solution, returning to the state depicted in **Figure 8A****,** and the process can be repeated. Figure 8 demonstrates the light-mediated modification of a polynucleotide strand within a nanopore. Strategies based on light-modified scission of polynucleotide strands, especially scission of side chains of a polynucleotide strand, can be used in the methods of the present disclosure.

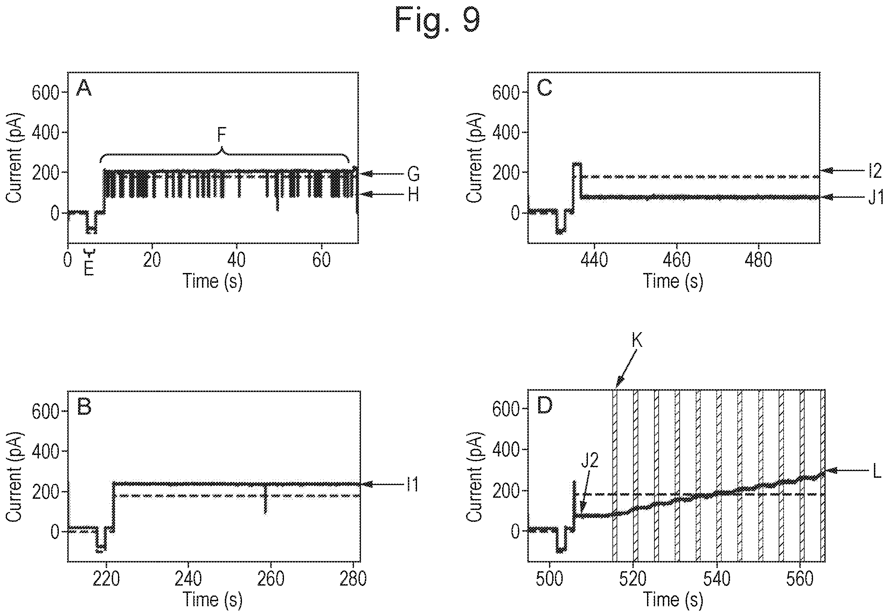

**Figure 9** shows representative experimental ionic current vs time data from the scission process described in Figure 8, but wherein the polynucleotide substrate does not comprise a photo-cleavable linker (e.g. wherein the polynucleotide substrate is a streptavidin-capped poly-T strand, as described in the Examples). **Figure 9A** shows a typical ionic current vs time trace for a single nanopore, capturing a test DNA analyte. G is the open-pore level and H the blockade level for the test analyte. **Figure 9B** shows the pore current (I1) after flushing out the test analyte. **Figure 9C** shows the data after addition of the reaction test substrate, with the current dropping to level J1 representative of the trapping of the capped polynucleotide strand. The strand remains permanently trapped, so the blockage is permanent in the absence of irradiation. **Figure 9D** shows the period of UV irradiation. J2 indicates capture current level of a trapped DNA strand. Lines marked K indicate the timings of the brief UV pulses. L indicates the drift of the trapped strand level as a result of UV irradiation. Figure 9D indicates that there is no loss of the captured strand due to the multiple UV pulses for the control (e.g. polyT) substrate. Data are discussed in the Examples.

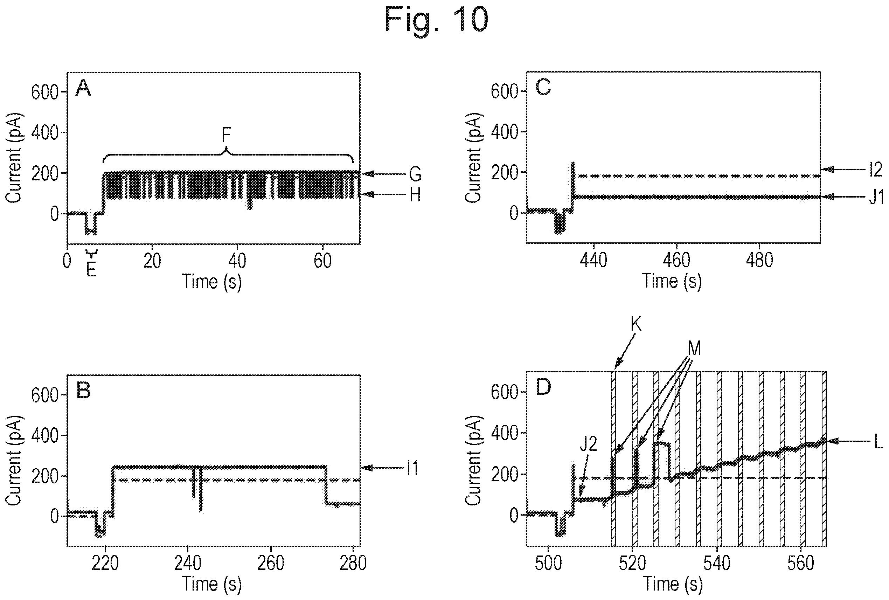

**Figure 10** shows analogous data and labels to Figure 9. However in Figure 10 the polynucleotide strand contains a photocleavable linker. A significant differences as compared to Figure 9 can be seen in Figure 10D. Unlike the non-photocleavable sample of Figure 9, where there is no loss of the strand after irradiation, in this sample with the photocleavable linker there is almost immediate loss of the strand (marked M), followed by capture of a another uncleaved strand from solution, in a high percentage of the UV pulses. Figure 10 demonstrates the successful on-pore (i.e. within the nanoreactor) modification of a polynucleotide strand. Data are discussed in the Examples.

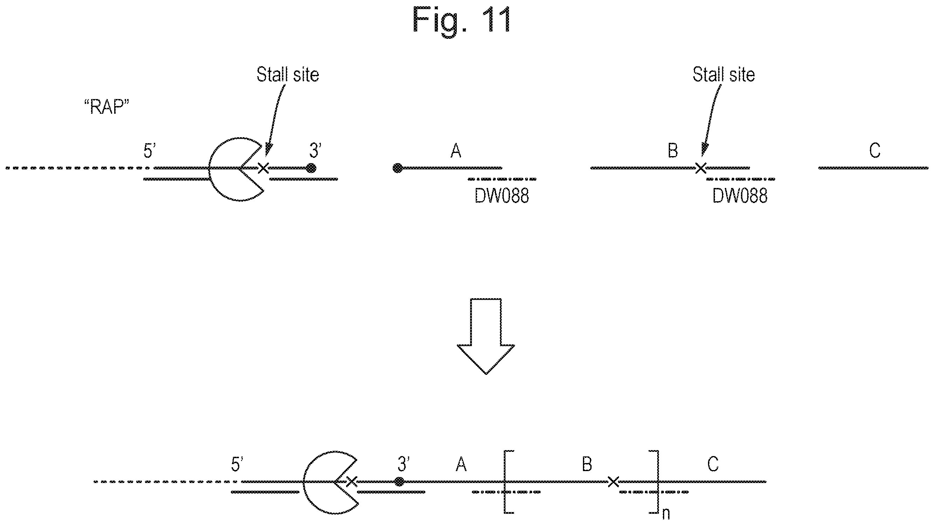

**Figure 11** illustrates the preparation of a polynucleotide substrate for controlled movement through a nanopore by means of a motor protein (notched circle). The figure shows the components conjugated together to form the substrate, comprising a Nanopore Sequencing adapter "RAP", a seed component A, n repeating units of the central main unit B, and a tail unit C. An overlapping complementary strand "DW088" is also shown.

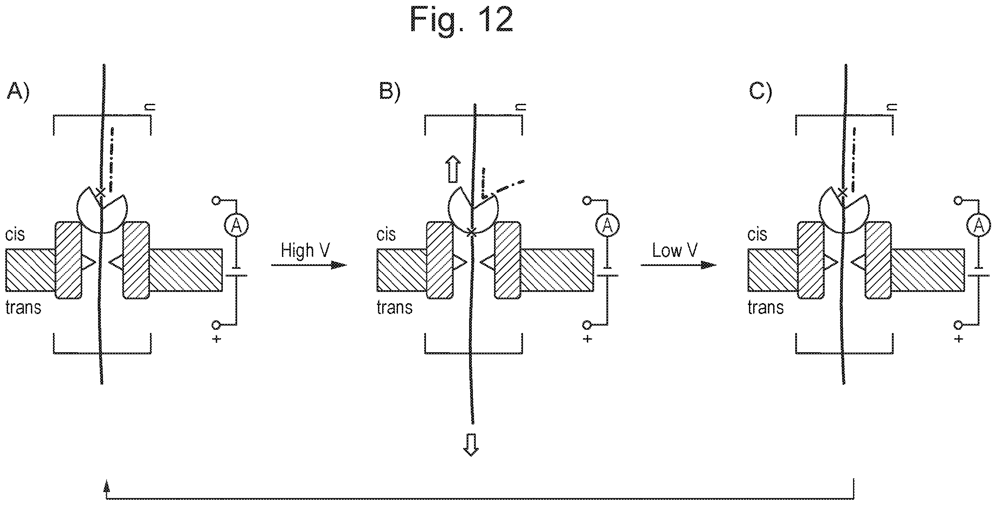

**Figure 12** illustrates motor protein-controlled movement of a polynucleotide substrate (e.g. the substrate shown in Figure 11) through a nanopore nanoreactor. As shown, the motor protein and voltage can be used together to control the polynucleotide translocation through the pore and pause desired sections in the nanopore when required. After initial capture in the nanopore nanoreactor under an applied voltage the polynucleotide will translocate until contacting the motor protein that is stalled at a "stall site" (Figure 12A). Under high applied positive voltage to the trans the motor protein will be pushed over the stall site and proceed to control the movement of the DNA through the nanopore (Figure 12B). The motor protein will continue to pass through all subsequent stall sites it encounters in the repeated B units of the strand while the applied voltage is high. When the voltage is dropped to a lower holding voltage, for example at a desired time point, the motor protein will be unable to overcome the next stall it encounters, thus stalling the translocation of the substrate through the nanopore nanoreactor and pausing that specific B unit in the nanopore nanoreactor for the duration of the low voltage period (Figure 12C > Figure 12A). When required the voltage can be increased once again to cause the motor protein to pass over the stall to continue DNA translocation. Therefore, by control of the voltage it is possible to pause and resume movement of the substrate through the nanopore.

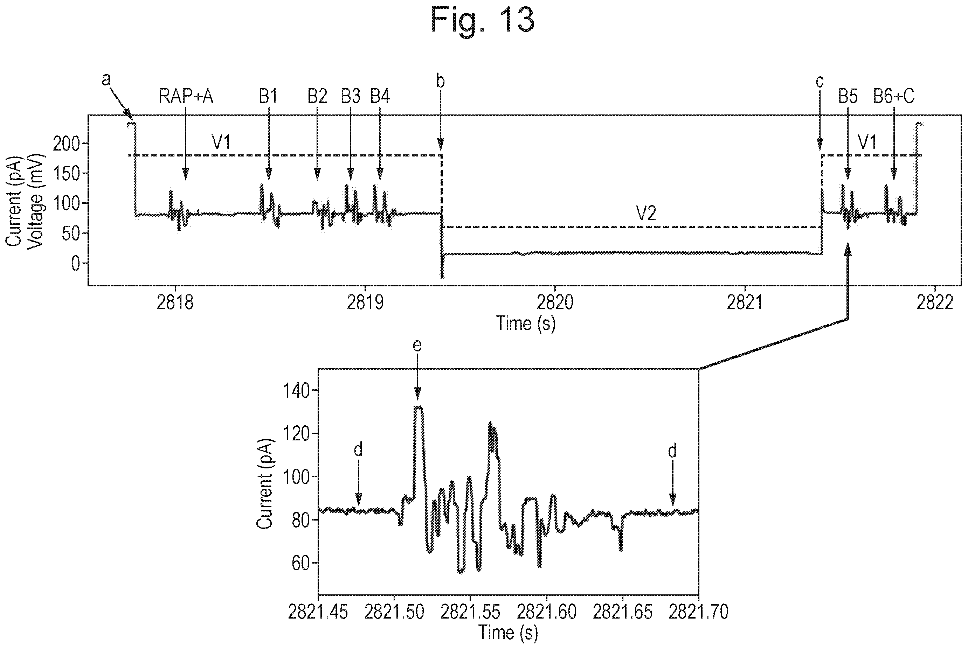

**Figure 13** shows representative experimental ionic current vs time data for movement described in Figure 12 and the concatenated DNA strands composed of RAP + A + (B)n + C units described in Figure 11. The data was acquired on a MinION using baseline sequencing conditions and custom voltage control scripts.

Figure 13 shows a typical example of the capture and subsequent movement control of a concatenated DNA substrate through a nanopore. At (a) the substrate is first captured under high voltage V1 (in this example, + 180m V) until the motor protein contacts the top of the nanopore, resulting in a characteristic reduced current blockade. The motor protein prevents further translocation until it is pushed over the first stall by the force of the applied voltage. After a brief pause as the stall the motor is pushed over the stall and the "RAP+A" signal is observed passing through the nanopore as the motor protein feeds the DNA into the nanopore under ATP fuel controlled movement. While the voltage is held at V1 the motor protein continues to proceed through subsequent stall sites in the "Bn" units after brief pauses at each stall. The stall sites produce characteristic large current deflections (e) as indicated in the zoom below. Pauses are marked (d).

When the voltage is lowered to a holding voltage V2 (e.g. +60 mV) at point (b), the ionic current passing through the pore immediately drops, and the reduced force applied by the lower voltage results in the motor protein being unable to overcome the next stall site it encounters. During the V2 period the lack of change in the current signal clearly indicates that the DNA strand is stalled and no longer being passed through the pore under the control of the motor protein. When the voltage is increased to V1 again at point (c) the motor protein is pushed over the stall after a brief pause and continues to proceed along the strand and through subsequent stall sites in the (B) units. The DNA used here does not have a streptavidin bound to the 3'end of the DNA on the C unit, so the strand exit the pore when reaching the end. Data discussed in the examples.

This figure illustrates how changes in voltage can be used to control movement and stalling of polynucleotides such as DNA in a nanoreactor such as a nanopore, thus enabling control over which units are held in the nanopore for an extended period. This can be used for example to stall the DNA at desired locations along the strand for sufficient time to apply a reaction to modify only the desired unit in or near the nanopore, to for example encode a pattern of modifications to encode data into the DNA strand.

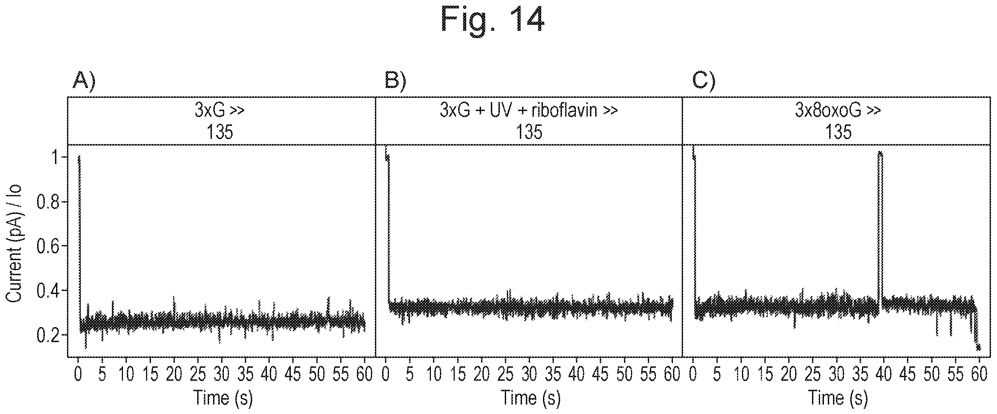

**Figure 14** shows representative current blockades from the capture of single-stranded DNA polynucleotides containing 3 guanine bases in a polyT background in CsgG nanopores. The strands are captured by the 5' end in the nanopore and trapped by a traptavidin bound to a 3' biotin on the DNA so that the guanine bases are located nearer the central constriction of the nanopore. As shown, the magnitude of the current blockade is indicative of the state of the guanine bases. Figure 14 shows representative capture events (ionic current vs time) for 3 samples: Figure 14A: a sample of untreated DNA that contains 3 guanine bases in a polyT background; Figure 14B: the same sample as in Figure 14A, but exposed to UV irradiation in the presence of the sensitizer riboflavin; and Figure 14C: a positive control sample with DNA containing 3 8-oxo-dG bases at equivalent positions to the unmodified dG bases in Figure 14A. As figure 14 illustrates, the blockade levels from the treated sample B) match those of the 8-oxo-dG control C), thus indicating that the irradiation has modified the strand within the nanopore nanoreactor.

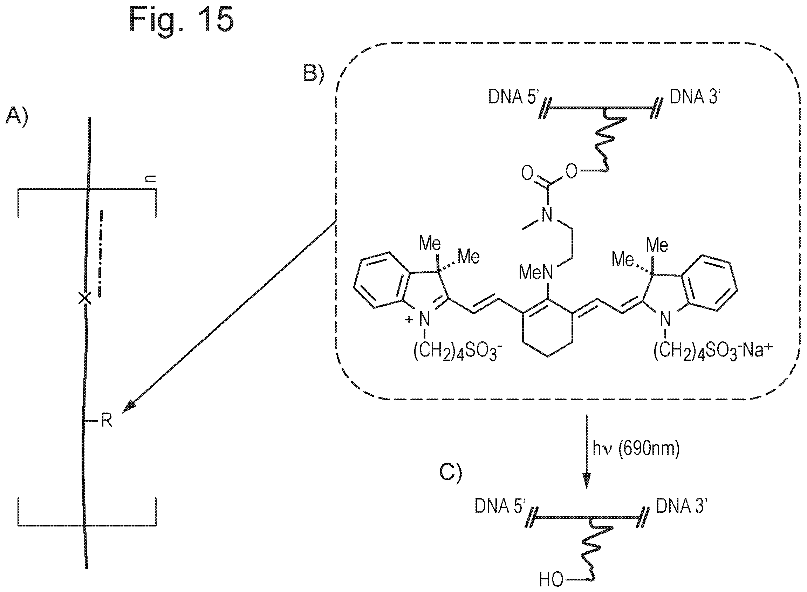

**Figure 15** shows how a polynucleotide substrate with photocleavable side-chain chemistry can be modified by light mediated photocleavage for the purposes of encoding data. Figure 15 shows an example of the "B" portion of a concatemeric polynucleotide substrate, such as the ones as shown in Figure 11, which contains a modifiable side-chain moiety "R" at a given position in the strand (Figure 15A). Figure 15B shows an example of a photocleavable molecule that is attached to the DNA, for example to the backbone of the DNA polynucleotide. Figure 15C shows that upon irradiation with the correct wavelength of light the molecule undergoes photocleavage.

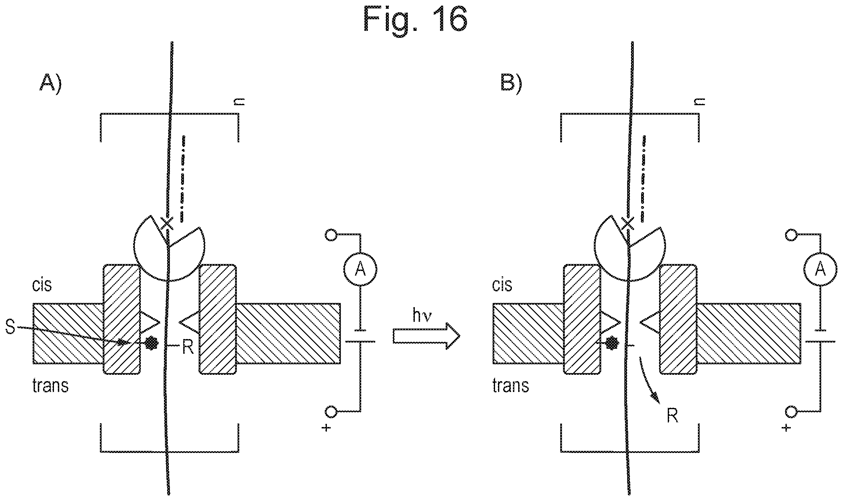

**Figure 16** shows a schematic representation of the selective modification of one unit of a concatemeric DNA substrate, such as the one described in Figure 15, by selective photocleavage of the photocleavable side-chain in the nanopore. Figure 16A shows the substrate held in the nanopore, for example paused at low voltage, with the photocleavable side-chain R positioned nearby a photosensitizer (labelled S). Upon irradiation of the entire nanopore system, the sensitizer absorbs the incoming radiation and re-emits radiation locally at a different wavelength. The photocleavable side-chain does not absorb the incoming global wavelengths and is only sensitive to the local re-emitted radiation from the sensitizer, which results in photocleavage as indicated and loss of the side-chain molecule. (Figure 16B). Other photocleavable R groups on the rest of the substrate outside of this unit in the nanopore (not shown) are much further away from the sensitizer and not exposed to sufficient radiation to enable photocleavage.

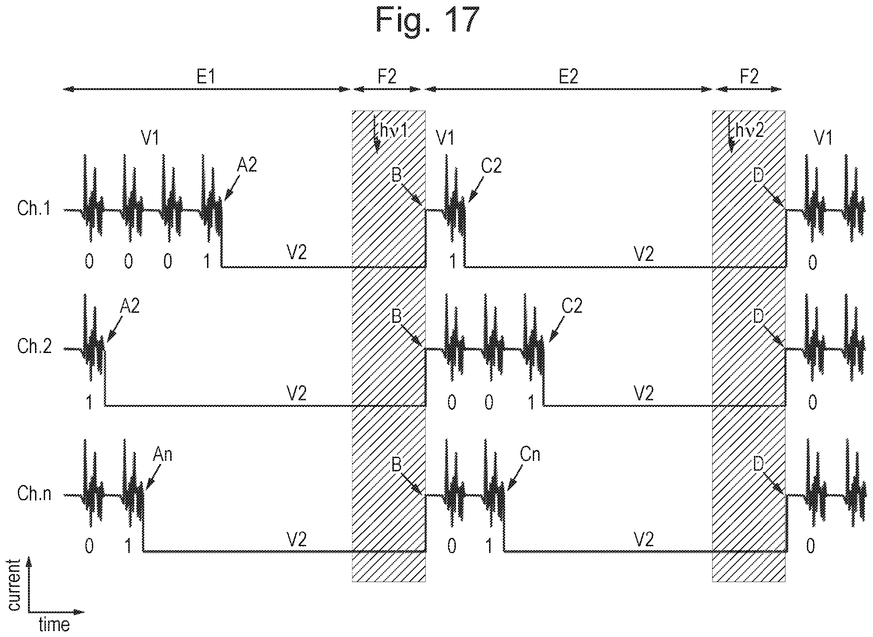

**Figure 17** shows representative ionic current vs time data from 3 channels (Ch.1, Ch.2 , and Ch.n) in an array of channels, for example on a MinION chip. Each channel is independently addressed with electrodes and circuitry that enables individual control of the applied voltage to that channel. For example, in some embodiments MinION ASICs are employed, using unblock circuitry to independently switch each channel between a holding voltage (V2) and the global voltage (V1) as required. In some embodiments, software, such as Oxford Nanopore MinKNOW software with active monitoring and feedback control, is used to independently monitor the current levels in real-time. In some embodiments the software monitors the current levels to sequence and detect the forward movement of the DNA substrates in each channel, and switches the voltage as required such as to encode a pre-determined pattern of data. Channel 1 is set to encode [000110...] into the strand, Channel 2 is set to encode [10010...] and Channel n is set to encode [01010...] as indicated on the figure under the repeating units. This is described in more detail in the examples.

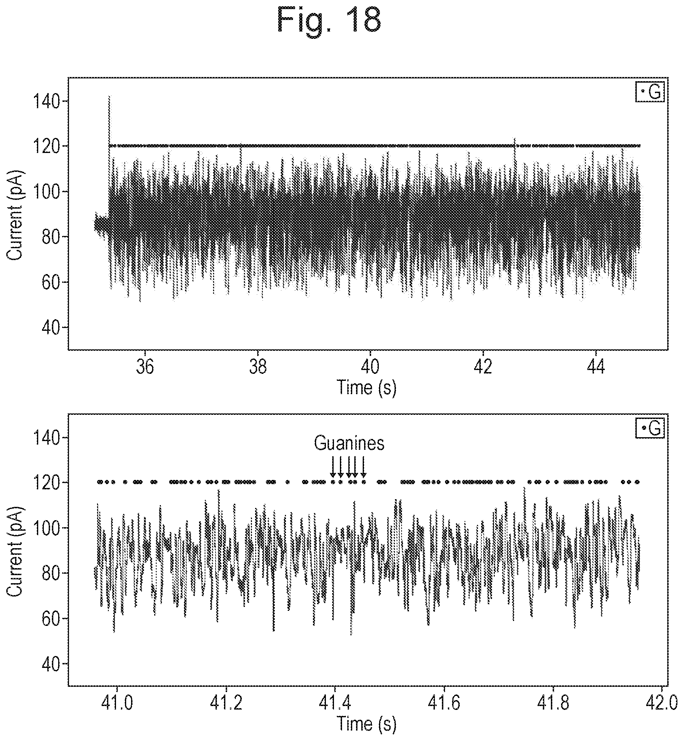

**Figure 18** shows a typical experimental ionic current vs time signal for nanopore sequencing of a 3.5 kilobase section of the lambda phage DNA. The polynucleotide was sequenced using FLO-MIN106 chemistry on a MinION flow cell (Oxford Nanopore Technologies). The figure marks up the locations of the guanine residues, which are spread at high density across the sequence. The guanines can be more easily picked out in the zoom section in the lower panel of Figure 18, which is provided for ease of reference.

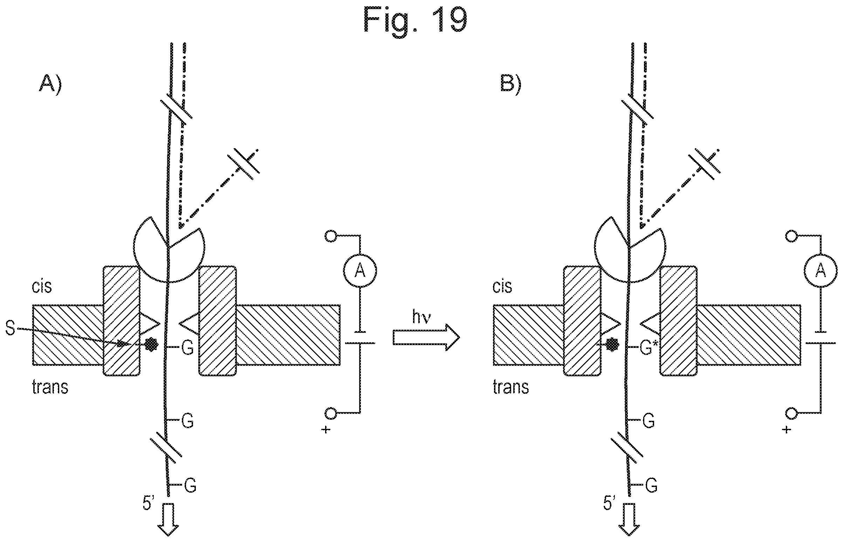

**Figure 19** shows a schematic representation of the selective modification of the portion of the polynucleotide in or near the nanopore as it moves through a nanopore from cis to trans by means of a motor protein. The polynucleotide contains purine bases, for example guanine bases distributed through natural DNA. Only the guanine bases (labelled G) in the nanopore adjacent to a sensitizer (S) are modified by the irradiation of the system to a modified guanine (G*), for example to 8-oxo-guanine or other oxidized guanine products.

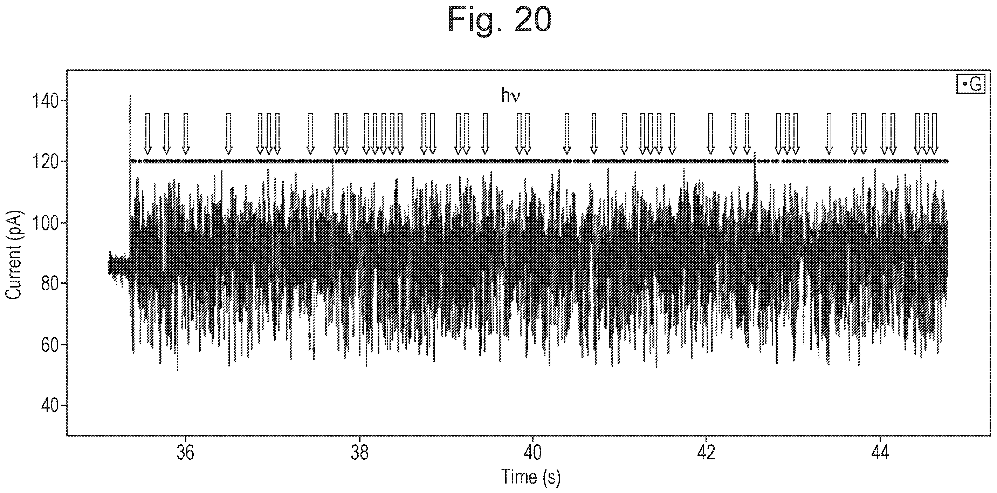

**Figure 20** illustrates a fragment of lambda phage dsDNA moving through a nanopore at an approximately constant rate being modified with an irregular reaction signal to create a unique pattern of modifications in the strand. Reaction conditions, for example irradiation by the appropriate light source, are applied at irregular intervals to modify portions of the marked guanine bases long the strand as indicated, such as to encode a unique pattern to store data.

### DETAILED DESCRIPTION

It is to be understood that different applications of the disclosed methods and products may be tailored to the specific needs in the art. It is also to be understood that the terminology used herein is for the purpose of describing particular embodiments of the methods and products only, and is not intended to be limiting.

**In** addition as used in this specification and the appended claims, the singular forms "a", "an", and "the" include plural referents unless the content clearly dictates otherwise. Thus, for example, reference to "a polynucleotide" includes two or more polynucleotides, reference to "a transmembrane pore" includes two or more pores, etc.

All publications, patents and patent applications cited herein, whether supra or infra, are hereby incorporated by reference in their entirety.

### General method

The inventors have devised a method of encoding data on a polynucleotide strand, comprising:
(A) moving the polynucleotide strand with respect to a nanoreactor; and
(B) selectively modifying portions of the polynucleotide strand as they move through the nanoreactor;
wherein the pattern of selective modifications on the polynucleotide strand encodes data on the strand.

The polynucleotide may be any polynucleotide described in more detail herein.

The polynucleotide may be moved through the nanoreactor using any appropriate method. For example, the polynucleotide is typically charged. Thus, voltage can be used to move the polynucleotide through a nanoreactor. The nanoreactor can be any suitable nanoreactor as described herein. For example, as described in more detail herein, the nanoreactor maybe a nanopore in which case an applied voltage across the nanopore can be used to contact the polynucleotide with the nanopore and to move the polynucleotide with respect to the nanopore.

The method is for encoding data on said polynucleotide. The polynucleotide may be for the storage of data. Any suitable modification can be used. Suitable modifications that can be made to polynucleotides are discussed herein. Any modification which can be used to encode data on a polynucleotide can be used in the invention.

The methods of the invention typically do not alter the overall length of the polynucleotide strand. The overall length of the polynucleotide strand is often expressed in terms of the number of monomer units that makes up the polynucleotide strand. **In** the methods, the number of monomer units in the polynucleotide strand is typically unaltered. For example, selectively modifying portions of the polynucleotide strand typically does not comprise adding or removing monomer units from the polynucleotide strand.

Those skilled in the art will thus appreciate that the methods of the invention can in some embodiments be likened to a ticker tape mechanism. Such an analogy is provided for illustration only and in no way limits the invention. The "tape" in this analogy is the polynucleotide strand and passes through the nanoreactor wherein it is modified in much the same way that data can be encoded on a ticker tape by e.g. punching holes in the tape. The tape length does not alter but the data is encoded onto the tape. In the same way, the backbone of the polynucleotide strand is typically not altered in the methods disclosed herein; rather data is encoded onto the polynucleotide strand. The data encoded onto the polynucleotide strand can be considered as an additional layer of information that is encoded in addition to any data that may be encoded by the unmodified polynucleotide strand. For example, the unmodified polynucleotide strand may be a polynucleotide sequence which could encode one or more polypeptides etc. The modifications made to the polynucleotide strand encode additional data above this which can be read as described herein in the methods of the invention. Thus, the data that is encoded on the polynucleotide strand in the methods of the invention is typically "exogenous" data that is not present in the unmodified polynucleotide strand before it is modified in the methods of the invention.

In the methods of the invention, the nanoreactor typically comprises a nanopore. In such embodiments the nanoreactor may comprise the internal volume of the nanopore or a portion of the internal volume of the nanopore. The nanoreactor may comprise the entire internal volume of the nanopore. The nanoreactor may comprise the internal volume of only a portion of the nanopore. For example, if a protein nanopore is used and the protein nanopore has, for example, a barrel and a lumen region, the nanoreactor may comprise either the barrel or the lumen or both, or a portion of the barrel or a portion of the lumen or both. Those skilled in the art will appreciate that the size of the nanoreactor can be controlled according to the application of the methods and for example according to the size of the portion of the polynucleotide strand which is to be modified (e.g. the size of the data unit to be encoded).

The nanoreactor may comprise a nanovolume around a nanopore. The nanovolume may comprise a volume extending to about 30 nm from one or more openings of the nanopore. For example, the nanovolume may comprise a volume extending to about 20 nm, about 10 nm or about 5 nm from one or more openings of the nanopore. If a nanopore has both a cis and a trans opening, for example, the nanovolume may extend from the cis opening, the trans opening or both the cis and the trans opening. The nanovolume which extends beyond the nanoreactor (e.g. beyond the opening of a nanopore) is also referred to as a reaction region (e.g. shown as (D) in Figure 1).

The nanopore may be a transmembrane protein nanopore, a solid state nanopore, a DNA-origami pore, or a polymer-based plastic pore. The nanopore may be a transmembrane β-barrel protein pore. The nanopore may comprise a well, gap, channel, tube, trench or slit in a membrane or across a membrane. Nanopores are described in more detail herein.

The method may comprise passing the polynucleotide strand through the nanopore. As described in more detail herein, the polynucleotide strand may be passed (translocated) through a nanopore under an applied voltage. Methods and apparatus for translocating polynucleotide strand through nanopores are well known in the art and are described in more detail herein.

As explained above, the methods involve selectively modifying portions of the polynucleotide strand as they move through the nanoreactor. Any suitable modifications can be made. For example, any modifications which allow modified portions of the polynucleotide strand to be distinguished from unmodified (non-modified, or less modified) portions of the polynucleotide strand can be made.

In some embodiments, selectively modifying portions of the polynucleotide strand comprises making epigenetic modifications to the polynucleotide strand. Many epigenetic modifications to polynucleotide strands are known to those skilled in the art and some are described in more detail herein.

The selective modification of portions of the polynucleotide strand as they move through the nanoreactor can be made in any suitable way. Typically, selectively modifying portions of the polynucleotide strand comprises subjecting the portions of the polynucleotide strand in the nanoreactor to reaction conditions comprising (i) the presence, absence or concentration of one or more chemical reagent(s); (ii) the engagement of an enzyme with the polynucleotide strand under conditions that the enzyme modifies the nucleotides within the polynucleotide strand; (iii) the presence or absence of electromagnetic radiation; and/or (iv) the presence or absence of applied heat. Such reaction conditions are described in more detail herein.

The methods of the invention may further comprise the step of:
(C) determining the pattern of selective modifications on the polynucleotide strand.

**In** such methods, determining the pattern of selective modifications on the polynucleotide strand typically comprises determining the presence, absence, extent or properties of modifications made to the polynucleotide strand. As such, determining the presence, absence, extent or properties of modifications made to the polynucleotide strand corresponds to reading data encoded on the polynucleotide strand. Thus, the writing of data onto the polynucleotide strand can be identified with the modifications that are made to the polynucleotide strand as it moves with respect to the nanoreactor and reading the data that is thereby written onto the polynucleotide strand as it moves with respect to the nanoreactor can be identified with determining the presence, absence, extent or properties of modifications made to the polynucleotide strand.

In some embodiments the methods comprise characterizing the modified polynucleotide strand. Such characterisation is distinct from determining the presence, absence, extent or properties of modifications made to the polynucleotide strand. For example, characterizing the modified polynucleotide strand can allow information about the polynucleotide strand "backbone", e.g. its sequence or the percentage content of any specific nucleotide(s) to be determined. Determining the presence, absence, extent or properties of modifications made to the polynucleotide strand can allow the data that is encoded as a separate "layer" above this to be read.

The invention also provides a method of modifying a polynucleotide strand, comprising:
(A) moving the polynucleotide strand with respect to a nanoreactor; and
(B) selectively modifying portions of the polynucleotide strand as they move through the nanoreactor.

In such methods, the polynucleotide strand is typically a polynucleotide strand as described herein. The nanoreactor is typically a nanoreactor as described herein. The selective modifications which are made to portions of the polynucleotide strand as they move through the nanoreactor are typically modifications as described herein. The methods may be a method as described here, for example a method of encoding data on the polynucleotide strand.

### Modification of portions of the polynucleotide

In the methods of the invention, the polynucleotide strand may comprise one or more portion(s) within the nanoreactor and one or more portion(s) external to the nanoreactor. **In** such embodiments, the method may comprise subjecting the portion(s) of the polynucleotide within the nanoreactor to reaction conditions such that (i) the portion(s) of the polynucleotide within the nanoreactor are modified and (ii) the portion(s) of the polynucleotide external to the nanoreactor are not modified or are modified differently to the portions of the polynucleotide within the nanoreactor.

The portions of the polynucleotide strand which are selectively modified are typically sequential portions of the polynucleotide strand. The portions therefor typically follow one another along the polynucleotide strand. The method does not require that all portions that sequentially follow one another along the polynucleotide strand are modified. For example, as the portions of the polynucleotide strand pass through the nanoreactor some portions may be modified and other portions may not be modified. Alternatively all portions may be modified, e.g. to different extents. Some portions may be modified in a first pass of the polynucleotide strand through the nanoreactor and other portions may be modified in a second pass of the polynucleotide strand through the nanoreactor. The pattern of modifications on the polynucleotide strand encodes data on the polynucleotide strand.

Thus, the portion(s) of the polynucleotide within the nanoreactor may be selectively modified whilst the portion(s) of the polynucleotide external to the nanoreactor are not modified. For example, a modified portion of a polynucleotide may represent a 1 and an unmodified portion of a polynucleotide may represent a 0, such that binary data may be encoded on the polynucleotide. Alternatively, a modified portion of a polynucleotide may represent a 0 and an unmodified portion of a polynucleotide may represent a 1, such that binary data may be encoded on the polynucleotide.

The portion(s) of the polynucleotide within the nanoreactor may be selectively modified whilst the portion(s) of the polynucleotide external to the nanoreactor are differently modified. Thus, the portion(s) of the polynucleotide within the nanoreactor may be selectively modified in a first manner (i.e. subject to a first modification) whilst the portion(s) of the polynucleotide external to the nanoreactor are modified in a second manner (i.e. subject to a second modification). The first modification and the second modification may be different modifications i.e. the chemical medication of the polynucleotide may differ between the first and second modifications. The first modification and the second modification may be the same modification but applied to different extents. For example, the first modification may correspond to modification of at least 10%, at least 20%, at least 30%, at least 40%, at least 50%, at least 60%, at least 70%, at least 80%, at least 90%, at least 95%, at least 98%, at least 99% or more of the portion(s) of the polynucleotide within the nanoreactor. The second modification may correspond to modification of at most 99%, at most 98%, at most 95%, at most 90%, at most 80%, at most 70%, at most 60%, at most 50%, at most 40%, at most 30%, at most 20%, at most 10%, or less of the portion(s) of the polynucleotide external to the nanoreactor. The methods of the invention are very sensitive and even small differences between the extent or nature of the modifications made to the portion(s) of the polynucleotide within the nanoreactor and the portion(s) of the polynucleotide external to the nanoreactor can be used to encode data on the polynucleotide.

### Data encoded on the polynucleotide

The portions of the polynucleotide strand which are selectively modified correspond to data units thereby encoded. For example, sequential portions of the polynucleotide may move with respect to the nanoreactor as described herein with units of data encoded on the polynucleotide as sequential portions of the polynucleotide move with respect to the nanoreactor.

The portions can be of any suitable length depending on the application of the method and the nanoreactor used, etc. For example, the portions of the polynucleotide strand which are modified in the nanoreactor may be from 1 to about 1000 nucleotides, for example the portions of the polynucleotide strand which are modified in the nanoreactor may be from 1 to about 100 nucleotides, e.g. the portions of the polynucleotide strand which are modified in the nanoreactor may be from 1 to about 10 nucleotides. Those skilled in the art can determine appropriate portions to use according to the application of the methods e.g. according to the amount and nature of the data being stored on the polynucleotide strand. The size of the unit of the polynucleotide which is modified in the nanoreactor, i.e. the size of the data unit that is encoded, can be controlled by controlling the size of the nanoreactor, the speed of the movement of the polynucleotide strand with respect to the nanoreactor, the duration of time over which modification is allowed to take place, etc. The nanoreactor may be configured to modify any desired number of nucleotides in the polynucleotide as it moves with respect to the nanoreactor. In some methods, the nanoreactor is capable of modifying individual nucleotides in a polynucleotide as they move with respect to the nanoreactor.

For example if the modification of a nucleotide represents a 1 and the lack of modification of a nucleotide represents a 0 then binary data can be encoded on the polynucleotide based on the modification or absence of modification of sequential nucleotides in the polynucleotide. However, the method is not limited to the modification of individual nucleotides. As those skilled in the art will appreciate, blocks of nucleotides within the polynucleotide can be modified as a unit to encode data. The modification of a block of nucleotides within the polynucleotide can represent a 1 or a 0 with the lack of modification of the block of nucleotides representing a 0 or a 1. However, more complex encoding is possible with patterns of modification of nucleotides within the block of nucleotides encoding specific data e.g. words or commands ("data structures").

The methods of the invention typically comprising selectively modifying from about 10¹ to about 10⁹ portions of the polynucleotide strand as they move through the nanoreactor. For example, about 10¹, about 10², about 10³, about 10⁴, about 10⁵, about 10⁶, about 10⁷, about 10⁸ or about 10⁹ portions may be modified in the methods of the invention. The number of portions that are modified can be chosen according to the amount and nature of the data being stored on the polynucleotide strand. When larger amounts of data are required, for example, longer polynucleotide strands can be used and the number of portions which are modified as they pass through the nanoreactor can be increased. In other embodiments, shorter polynucleotide strands are used e.g. when less data needs to be stored.

### Polynucleotides

The methods of the invention involve encoding data on a polynucleotide strand as it moves through a nanoreactor.

A polynucleotide, such as a nucleic acid, is a macromolecule comprising two or more nucleotides. The polynucleotide can be single-stranded or double-stranded. The polynucleotide or nucleic acid may comprise any combination of any nucleotides. The nucleotides can be naturally occurring or artificial.

A nucleotide typically contains a nucleobase, a sugar and at least one phosphate group. The nucleobase and sugar form a nucleoside.

The nucleobase is typically heterocyclic. Nucleobases include, but are not limited to, purines and pyrimidines and more specifically adenine (A), guanine (G), thymine (T), uracil (U) and cytosine (C).

The sugar is typically a pentose sugar. Nucleotide sugars include, but are not limited to, ribose and deoxyribose. The sugar is preferably a deoxyribose. The polynucleotide preferably comprises the following nucleosides: deoxyadenosine (dA), deoxyuridine (dU) and/or thymidine (dT), deoxyguanosine (dG) and deoxycytidine (dC).

The nucleotide is typically a ribonucleotide or deoxyribonucleotide. The nucleotide typically contains a monophosphate, diphosphate or triphosphate. The nucleotide may comprise more than three phosphates, such as 4 or 5 phosphates. Phosphates may be attached on the 5' or 3' side of a nucleotide. Nucleotides include, but are not limited to, adenosine monophosphate (AMP), guanosine monophosphate (GMP), thymidine monophosphate (TMP), uridine monophosphate (UMP), 5-methylcytidine monophosphate, 5-hydroxymethylcytidine monophosphate, cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), deoxycytidine monophosphate (dCMP) and deoxymethylcytidine monophosphate. The nucleotides are preferably selected from AMP, TMP, GMP, CMP, UMP, dAMP, dTMP, dGMP, dCMP and dUMP.

A nucleotide may be abasic (i.e. lack a nucleobase). A nucleotide may also lack a nucleobase and a sugar (i.e. is a C3 spacer).

The nucleotides in the polynucleotide may be attached to each other in any manner. The nucleotides are typically attached by their sugar and phosphate groups as in nucleic acids. The nucleotides may be connected via their nucleobases as in pyrimidine dimers.

The polynucleotide can be a nucleic acid, such as deoxyribonucleic acid (DNA) or ribonucleic acid (RNA). The polynucleotide can comprise one strand of RNA hybridized to one strand of DNA. The polynucleotide may be any synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains. The PNA backbone is composed of repeating N-(2-aminoethyl)-glycine units linked by peptide bonds. The GNA backbone is composed of repeating glycol units linked by phosphodiester bonds. The TNA backbone is composed of repeating threose sugars linked together by phosphodiester bonds. LNA is formed from ribonucleotides as discussed above having an extra bridge connecting the 2' oxygen and 4' carbon in the ribose moiety. The polynucleotide is preferably DNA, RNA or a DNA or RNA hybrid, most preferably DNA. A DNA/RNA hybrid may comprise DNA and RNA on the same strand. Preferably, the DNA/RNA hybrid comprises one DNA strand hybridized to a RNA strand.

The backbone of the polynucleotide can be altered to reduce the possibility of strand scission. For example, DNA is known to be more stable than RNA under many conditions. The backbone of the polynucleotide strand can be modified to avoid damage caused by e.g. harsh chemicals such as free radicals.

DNA or RNA that contains unnatural or modified bases can be produced by amplifying natural DNA or RNA polynucleotides in the presence of modified NTPs using an appropriate polymerase.

The methods of the invention involve the modification of polynucleotides. In addition to modification by the methods of the invention, one or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light and are the primary cause of skin melanomas. One or more nucleotides in the polynucleotide may be modified, for instance with a label or a tag. The polynucleotide may comprise one or more spacers.

The polynucleotide may be single- or double-stranded.

Single-stranded polynucleotide may be advantageous in some methods. For example, in some methods it is desirable to negate the force that is applied by double-stranded DNA/RNA re-annealing external to the nanoreactor, such as external to an internal volume of a nanopore. The re-annealing applies a force that acts to pull the DNA/RNA out of the nanopore.

In some embodiments a single-stranded polynucleotide may contain regions with strong secondary structures, such as hairpins, quadruplexes, or triplex DNA (e.g. see Figure 5). Structures of these types can be used to control the movement of the polynucleotide with respect to the nanoreactor, e.g. as it moves through a nanopore. For example, it is possible to use periodic hairpins or quadruplexes in a single-stranded substrate to control the movement of a polynucleotide through a nanopore. For example, secondary structures can be used to pause the movement of the polynucleotide through a nanopore, where the polynucleotide moves through the nanopores in a stick-slip fashion under an applied voltage, where the polynucleotide briefly pauses in the nanopore upon encountering each successive secondary structure along the strand before they are unwound by the force of the pore and translocated. Such methods are additional beneficial as they allow feedback to be obtained. Thus, for example, successive movements as the polynucleotide strand slips from one position to another may be detected in realtime by the nanoreactor (e.g. by a nanopore as described herein). The movement of the polynucleotide strand with respect to the nanoreactor can then be adjusted accordingly. For example, if an applied voltage is used to control movement, the feedback obtained by such "slippage" can be used to inform the applied voltage used throughout the application of the method. For instance, an applied voltage can be reduced to hold a polynucleotide strand within a nanoreactor such as a nanopore, and increased to progress the movement of the polynucleotide strand with respect to the nanoreactor (e.g. the nanopore).

The polynucleotide may reform secondary structures after it has moved with respect to the nanoreactor and been modified thereby. For example, when the nanoreactor is a nanopore the polynucleotide may form or re-form secondary structures after translocating through nanopore. Such secondary structures can be used to prevent the polynucleotide from moving back through the nanoreactor (nanopore) under low or no applied negative voltages (applied to the trans side of the nanopore).

An applied voltage can be modulated between +V, 0V, and -V to control the movement of a polynucleotide containing successive secondary structures to move the polynucleotide forward, pause (for example to enable a chemical reaction) , or reverse the polynucleotide. When using single-stranded DNA through a sliding molecular brake it is possible to pause the DNA movement in the nanopore at approximately 0mV of applied voltage.

In other embodiments the DNA or RNA is preferably double-stranded. Use of double-stranded polynucleotides can be advantageous in certain methods. For example, double-stranded polynucleotides can be used to reduce the formation of secondary structures in the primary translocated single-strand polynucleotide in cases where secondary structure causes unwanted issues, such as for example in the formation of unwanted quadruplexes that can alter the movement of the molecule through the nanopore, for example by creating unwanted pauses in movement in cases where smooth continuous speed is desirable) (secondary structures have to be unwound to pass through a small nanopore). However a double stranded polynucleotide may comprise single stranded regions and regions with other structures, such as hairpin loops, triplexes and/or quadruplexes. Such secondary structures can be useful as described above in the context of single-stranded polynucleotides.

In some embodiments the polynucleotide is double-stranded, where the two strands of the double-stranded molecule are covalently linked, for example at the ends of the molecules by joining the 5' end of one strand to the 3' end of the other with a hairpin structure. Strands with linked template and complement can be useful for example to compare the modified information (eg. in the template) to the original data (eg. in the original reverse complement). For example, after modifying the template strand of dsDNA strand with linked template and complement components, the DNA can be re-read, by a nanopore sequencer for example, comparing the modified DNA in the template strand to the unaltered DNA in the reverse complement strand, for example to validate the quality of the changes made to the DNA.

In some embodiments the pore is large enough to pass double-stranded polynucleotides, and modifications can be made to one or both strands of the dsDNA.

The polynucleotide can be any length. For example, the polynucleotides can be at least 10, at least 50, at least 100, at least 150, at least 200, at least 250, at least 300, at least 400 or at least 500 nucleotides or nucleotide pairs in length. The target polynucleotide can be 1000 or more nucleotides or nucleotide pairs, 5000 or more nucleotides or nucleotide pairs in length or 100000 or more nucleotides or nucleotide pairs in length or 500,000 or more nucleotides or nucleotide pairs in length, or 1,000,000 or more nucleotides or nucleotide pairs in length, 10, 000,000 or more nucleotides or nucleotide pairs in length, or 100,000,000 or more nucleotides or nucleotide pairs in length, or 200,000,000 or more nucleotides or nucleotide pairs in length, or the entire length of a chromosome. In some embodiments the polynculeotide is preferably very large (kilobase to megabases long) such as to encode large amounts of data into a single strand. In other embodiments the polynucleotide is of short or moderate length to enable faster random access read of the data.

The polynucleotide may be an oligonucleotide. Oligonucleotides are short nucleotide polymers which typically have 50 or fewer nucleotides, such 40 or fewer, 30 or fewer, 20 or fewer, 10 or fewer or 5 or fewer nucleotides. The target oligonucleotide is preferably from about 15 to about 30 nucleotides in length, such as from about 20 to about 25 nucleotides in length. For example, the oligonucleotide can be about 15, about 16, about 17, about 18, about 19, about 20, about 21, about 22, about 23, about 24, about 25, about 26, about 27, about 28, about 29 or about 30 nucleotides in length.

The polynucleotide may be a fragment of a longer target polynucleotide. In this embodiment, the longer polynucleotide is typically fragmented into multiple, such as two or more, shorter polynucleotides.

The polynucleotide may be naturally occurring. For example, the polynucleotide may be sourced from common organisms such as viruses, bacteria, archaea, plants or animals. Such organisms may be selected or altered to adjust the sequence of the source polynucleotide, for example by adjusting the base composition, removing unwanted sequence elements, and the like. The selection and alteration of organisms in order to arrive at desired polynucleotide characteristics is routine for one of ordinary skill in the art.

The source organism for the polynucleotide may be chosen based on desired characteristics of the sequence. Desired characteristics include the ratio of single-stranded vs double-stranded polynucleotides produced by the organism; the complexity of the sequences of polynucleotides produced by the organism, the composition of the polynucleotides produced by the organism (such as the GC composition), or the length of contiguous polynucleotide strands produced by the organism. For example, when a contiguous polynucleotide strand of around 50 kb is required, lambda phage DNA can be used. If longer contiguous strands are required, other organisms can be used to produce the polynucleotide; for example *E. coli* produces around 4.5 Mb of contiguous dsDNA.

In some embodiments the identity of the natural polynucleotide in each nanopore is known by controlling the source material. For example, the source template material could be full length contiguous ~50kbase lambda phage dsDNA for each nanoreactor (e.g. nanopore) in a system containing multiple nanoreactors (e.g. nanopores). However, although the starting polynucleotides may be identical in each case, the modifications made to each individual polynucleotide can be different, for example to encode different information into each polynucleotide. In some embodiments the sequence of the polynucleotide being fed through the nanopore is not known in advance. In other embodiments the sequence of the polynucleotide is known.

In some embodiments when using natural polynucleotide sources the strands used are not known before capture in nanopore for modification. In these cases it is sometimes preferable to sequence the polynucleotide either before or during the modification process. In one embodiment the polynucleotide is first sequenced before being modified, either in part or in its entirety, then aligned against known reference databases to determine the identity of the sequence. Once the sequence is known the modification process can be altered based on knowledge of the composition. For example, if the modification process only acts on G bases, then knowledge of the sequence can be used to control the position of the strand, for example to control the pausing of the desired sequence containing the G bases in the reaction volume to ensure optimal modification of desired bases.

In another embodiment the strand is sequenced and/or identified during the process of modification. This can be used to better control which regions of the strand are modified. In some embodiments the sequence is determined in real-time by aligning real-time signal or basecalling to known references. Exemplary methods of determining a polynucleotide sequence are described in WO 2016/059427, incorporated by reference herein. Pre-determined knowledge of the base composition of the polynucleotide strand can be used to more selectively control when the reaction condition (eg.light) is applied. This can be useful to control the modification to selectively modify single bases or a select number of bases in a known location. Pre-determined knowledge can also be useful when the modification occurs at some distance from a nanopore "reader" location that is sensitive to the bases, in embodiments when such readers are used to characterize the modifications made. For example the modification might occur above or below the nanopore reader, such as >1nm away. Knowledge of the rate of movement and the relative distance between the bases in the reader and those near the sensitizer can be used to better control the selectivity of the reaction region in the sequence.

In some embodiments the base composition is not known, and the modifications are made in longer stretches that cover multiple bases, creating "islands" of modification and unmodified.

Additionally, sequencing can be used to confirm that the modification has been successful. Further actions can then be taken on the strand when unsuccessful modification is detected. For example, in some embodiments where the direction of the strand can be reversed (for example by reversing the applied potential, e.g. causing the threading of a polynucleotide back through a nanopore after its initial movement forwards through the pore), so that the region to be modified can be returned to the reaction volume after unsuccessful modification to repeat the modification process. This process can be repeated until successful modification is detected.

Alternatively, if unsuccessful modification is detected in systems where the process cannot be repeated (e.g. if the direction of the strand cannot be reversed), then the modification criteria can be altered for later sections of the DNA to account for the previous unsuccessful modification. This process could be used, for example, to correct for incorrectly written data by later writing the same data again further along the polynucleotide, thus creating redundancy only when required (vs redundancy that is built into the code from the beginning, which can unnecessarily waste space).

In some embodiments the polynucleotide is synthetic or semi-synthetic. For example, the DNA or RNA may be purely synthetic, synthesised by conventional DNA synthesis methods such as phosphoamidite based chemistries. Synthetic polynucleotides subunits may be joined together by known means, such as ligation or chemical linkage, to produce longer strands. Strands may be synthesized (e.g. joined together) in an array of nanoreactors (e.g. nanopores) before data is encoded on the strands. In some embodiments internal self-forming structures (eg. hairpins, quadruplexes) can be designed into the substrate e.g. by ligating appropriate sequences. Enzymes for controlling movement can also be loaded onto the product substrate.

In some embodiments the polynucleotide has a simplified nucleotide composition for the majority of the region where modifications will be implemented. For example, a DNA strand may be composed of only, or substantially, G and A bases. Such bases do not readily hybridise and can be readily distinguished in modified and unmodified forms.

In some embodiments the polynucleotide has a repeating pattern of the same subunit. For example, a repeating unit may be (AmGn)q, wherein m, n and q are positive integers. For example, m is often from 1 to 20, such as from 1 to 10 e.g. from 1 to 5, e.g. 1, 2, 3, 4 or 5. n is often from 1 to 20, such as from 1 to 10 e.g. from 1 to 5, e.g. 1, 2, 3, 4 or 5. m and n may be the same or different. q is often from 1 to about 100,000. A typical repeating unit may be for example (AAAAAAGGGGGG)q.

Repeating polynucleotides can be made by many means known in the art, for example by concatenating together synthetic subunits with sticky ends that enable ligation. In some embodiments the polynucleotide may therefore be a concatenated polynucleotide. Methods of concatenating polynucleotides are described in PCT/GB2017/051493.

Synthetic polynucleotides can be copied and scaled up for production by means known in the art, including PCR, incorporation into bacterial factories, and the like.

In some embodiments the polynucleotide is altered to either increase or decrease reactivity to external agents. For example, in a mixed base system it can be desirable to control which bases are capable of being modified and which background bases should not be modified. Background bases can be selected so that they have reduced reactivity relative to canonical bases to limit the possibility of unwanted reactions. For example, bases ATC are less reactive than G, so it is possible to adjust the composition of the polynucleotide strand to have runs of less reactive ATC bases in regions where modification is not required, and regions with high G content where modification is to be made. Alternatively, bases that are intended to be modified can be selected to have higher reactivity when exposed to the appropriate reactions conditions.

In some embodiments, the polynucleotide can comprise bases which contain a reactive side-chain. Any suitable reactive functional groups can be incorporated on the side chain as required. Suitable examples of reactive functional groups include click chemistry reagents.

Click chemistry is a term first introduced by Kolb *et al.* in 2001 to describe an expanding set of powerful, selective, and modular building blocks that work reliably in both small- and large-scale applications (Kolb HC, Finn, MG, Sharpless KB, Click chemistry: diverse chemical function from a few good reactions, Angew. Chem. Int. Ed. 40 (2001) 2004-2021). They have defined the set of stringent criteria for click chemistry as follows: "The reaction must be modular, wide in scope, give very high yields, generate only inoffensive by-products that can be removed by non-chromatographic methods, and be stereospecific (but not necessarily enantioselective). The required process characteristics include simple reaction conditions (ideally, the process should be insensitive to oxygen and water), readily available starting materials and reagents, the use of no solvent or a solvent that is benign (such as water) or easily removed, and simple product isolation. Purification if required must be by non-chromatographic methods, such as crystallization or distillation, and the product must be stable under physiological conditions".

Suitable examples of click chemistry include, but are not limited to, the following:
(a) copper-free variant of the 1,3 dipolar cycloaddition reaction, where an azide reacts with an alkyne under strain, for example in a cyclooctane ring;
(b) the reaction of an oxygen nucleophile on one linker with an epoxide or aziridine reactive moiety on the other; and
(c) the Staudinger ligation, where the alkyne moiety can be replaced by an aryl phosphine, resulting in a specific reaction with the azide to give an amide bond.

Any reactive group may be used in the invention. The reactive group may be one that is suitable for click chemistry. The reactive group may be any of those disclosed in WO 2010/086602, particularly in Table 4 of that application.

In some embodiments, the polynucleotide, both before and after modification, is capable of being controlled by a polynucleotide motor to control movement with respect to a nanoreactor, e.g. to control translocation through a nanopore. Some polynucleotide motors, such as helicases, are tolerant of significant changes to natural polynucleotides, including unnatural backbones, altered sugar, altered bases with small or large adducts attached, abasic sites, etc. Some exemplary polynucleotide motors are described herein.

Preferably both the modified and unmodified forms of the polynucleotide are stable under appropriate storage conditions for extended periods of time. For example, the polynucleotide in its modified and/or its unmodified form may be stable for in excess of 1 day, 1 month, 1 year, 10 years, etc, when stored under appropriate conditions. The necessary stability of a polynucleotide can be determined based on its application and can be controlled using methods known in the art, including the use of high purity reagents and storage under appropriate conditions. In some embodiments the polynucleotide has to be processed after modification in the nanoreactor to alter the bases, stabilise the chemistry, store the polynucleotide, etc. In some embodiments the final modified strand can be copied by conventional polymerase amplification methods, while preserving the modification information in the copying process.

### Movement of the polynucleotide with respect to the nanoreactor

In the methods of the invention, the polynucleotide is modified under appropriate reaction conditions in the nanoreactor. The reaction conditions are applied to the polynucleotide as the polynucleotide moves with respect to the nanoreactor. For example, the polynucleotide may pass through the nanoreactor, such as through a nanopore. In other embodiments the polynucleotide may pass across the nanoreactor, such as across the opening of a nanopore. The movement of the polynucleotide with respect to the nanoreactor may be as described in more detail herein. In some embodiments of the invention the nanoreactor comprises an internal volume within a nanopore and/or a nanovolume around a nanopore. For example, the nanoreactor may be a nanopore. The polynucleotide may move with respect to the nanopore; for example the polynucleotide may move through (translocate) the nanopore. The nanopore may be any suitable nanopore as described herein.

In the methods of the invention, the polynucleotide strand may be continuously moved through the nanoreactor whilst portions of the polynucleotide strand are selectively modified as they move through the nanoreactor. In some embodiments, the methods comprise moving the polynucleotide strand at a constant rate with respect to the nanoreactor, and applying reaction conditions at a regular or irregular frequency to the polynucleotide strand as it moves through the nanoreactor. **In** other embodiments, the methods comprise moving the polynucleotide strand at a variable rate with respect to the nanoreactor, and applying reaction conditions at a regular or irregular frequency to the polynucleotide strand as it moves through the nanoreactor. By controlling the manner in which the reaction conditions are applied to the polynucleotide strand as it moves through the nanoreactor the way in which data is encoded onto the polynucleotide strand can be controlled. Examples of such methods are shown in the Figures.

Other embodiments of the methods of the invention comprise interrupting the movement of the polynucleotide strand with respect to the nanoreactor whilst selectively modifying the portions of the polynucleotide strand as they move through the nanoreactor. Examples of such methods are shown in the Figures. For example, by interrupting the movement of the polynucleotide strand with respect to the nanoreactor whilst selectively modifying the portions of the polynucleotide strand within the nanoreactor the extent of the modifications that are made to the portion of the polynucleotide strand within the nanoreactor can be finely controlled.

Thus, in one embodiment, the polynucleotide may move at a constant rate through the nanoreactor, e.g. through a nanopore or a volume around a nanopore. In another embodiment, the polynucleotide may move at a variable rate through the nanoreactor, e.g. through a nanopore or a volume around a nanopore. Whilst the polynucleotide moves through the nanoreactor the polynucleotide is subjected to modification by application of appropriate reaction conditions. The reaction conditions can be applied periodically. For example, application of the reaction conditions can lead to modification of portions of the polynucleotide within the nanoreactor and the modified portions of the polynucleotide can correspond to a signal such as a 1 or 0 to encode data. When the reaction conditions are not applied the portions of the polynucleotide within the nanoreactor are not modified and the unmodified portions of the polynucleotide can correspond to a different signal such as 0 or 1 thereby allowing data to be encoded.

The reaction conditions can be applied at a regular or irregular frequency. A regular frequency can be achieved by e.g. regularly pulsing a light or heat source, or by regular addition of reagents into the nanoreactor (e.g. into the environment around the nanoreactor leading to diffusion into the nanoreactor). An irregular frequency can be achieved by e.g. irregularly pulsing a light or heat source, or by irregular addition of reagents into the nanoreactor (e.g. into the environment around the nanoreactor leading to diffusion into the nanoreactor). A regular application of the reaction conditions in order to modify the polynucleotide as it moves with respect to the nanoreactor can be beneficial as it may permit a simpler setup or allow more predictable modifications to be made to the polynucleotide, which can reduce the complexity of subsequent data analysis to decode the data. An irregular application of the reaction conditions can be beneficial as it may allow more complex data to be recorded in the polynucleotide as it moves with respect to the nanoreactor.

Any suitable reaction condition to encode data on the polynucleotide can be used in the methods of the invention. Some suitable reaction conditions for encoding data on a polynucleotide are described herein.

### Nanoreactor

In the methods of the invention, any suitable nanoreactor can be used.

Often, the nanoreactor comprises an internal volume within a nanopore and/or a nanovolume around a nanopore. The nanopore can be any suitable nanopore. Various suitable nanopores are described in more detail herein.

Usually, when the nanoreactor comprises a nanopore or a nanovolume around a nanopore, the nanopore is a transmembrane protein nanopore, a solid state nanopore, a DNA-origami pore or a polymer-based plastic pore. Most often the nanopore is a transmembrane β-barrel protein pore.

A nanopore is a hole or channel through a membrane that permits hydrated ions driven by an applied potential to flow across or within the membrane. The nanopore may be a protein pore that crosses the membrane to some degree, or may be a non-protein pore that has a structure that crosses the membrane to some degree, such as a polynucleotide pore or solid state pore. The pore may be a DNA origami pore. The pore may be biological or artificial.

Membranes are described in more detail herein.

In the invention, the nanoreactor may comprise the internal volume of a nanopore or a portion of the internal volume of a nanopore. Any suitable nanopore as described herein may be used. Sometimes, the nanovolume comprises a volume extending to about 30 nm from one or more openings of the nanopore. For example, the nanovolume may comprise a volume extending to about 20 nm from one or more openings of the nanopore, such as to about 15 nm, e.g. to about 10 nm for example to about 5 nm from one or more openings of the nanopore. The nanovolume may extend around the cis opening of the nanopore. The nanovolume may extend around the trans opening of the nanopore. The nanovolume may extend around the cis opening and the trans opening of the nanopore.

A transmembrane pore suitable for use in the invention may be a solid state pore. A solid-state nanopore is typically a nanometer-sized hole formed in a synthetic membrane. Suitable solid state pores include, but are not limited to, silicon nitride pores, silicon dioxide pores and graphene pores. Solid state nanopores may be fabricated e.g. by focused ion or electron beams, so the size of the pore can be tuned freely. Suitable solid state pores and methods of producing them are discussed in US Patent No. 6,464,842, WO 03/003446, WO 2005/061373, US Patent No. 7,258,838, US Patent No. 7,466,069, US Patent No. 7,468,271 and US Patent No. 7,253,434.

A transmembrane pore may be a DNA origami pore (Langecker et al., Science, 2012; 338: 932-936). DNA origami pores are disclosed in WO2013/083983.

A transmembrane pore may be a polymer-based pore. Suitable pores can be made from polymer-based plastics such as a polyester e.g. polyethylene terephthalate (PET) via track etching.

A transmembrane pore suitable for use in the invention may be a transmembrane protein pore. A transmembrane protein pore is a polypeptide or a collection of polypeptides that permits ions driven by an applied potential to flow from one side of a membrane to the other side of the membrane. A transmembrane protein pore is a structure that crosses the membrane to some degree and may have a structure that extends beyond the membrane on one or both sides. A transmembrane protein pore may be a single or multimeric protein that permits hydrated ions to flow from one side of a membrane to the other side of the membrane. However, the transmembrane protein pore does not have to cross the membrane. It may be closed at one end. For instance, the pore may be a well, gap, channel, tube, trench or slit in the membrane along which or into which hydrated ions may flow.

A transmembrane protein pore typically comprises a barrel or channel through which the ions may flow. The subunits of the pore typically surround a central axis and contribute strands to a transmembrane β-barrel or channel or a transmembrane α-helix bundle or channel. A transmembrane protein pore typically comprises a channel that allows a polynucleotide, such as DNA or RNA, to move, or be moved, into and/or through the pore.

A transmembrane protein pore may be a monomer or an oligomer. An oligomer is preferably made up of several repeating subunits, such as at least 6, at least 7, at least 8, at least 9, at least 10, at least 11, at least 12, at least 13, at least 14, at least 15, or at least 16 subunits. For example, the pore may be a hexameric, heptameric, octameric or nonameric pore. The pore may be a homo-oligomer in which all of the subunits are identical, or a hetero-oligomer comprising two or more, such as 3, 4, 5 or 6, different subunits.

The barrel or channel of a transmembrane protein pore typically comprises amino acids that facilitate interaction with polynucleotides. These amino acids are preferably located near a constriction (such as within 1, 2, 3, 4 or 5 nm) of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

Transmembrane protein pores suitable for use in accordance with the invention can be derived from β-barrel pores or α-helix bundle pores.

β-barrel pores comprise a barrel or channel that is formed from β-strands. Suitable β-barrel pores include, but are not limited to, β-toxins, such as α-hemolysin (αHL), anthrax toxin and leukocidins, and outer membrane proteins/porins of bacteria, such as *Mycobacterium smegmatis* porin (Msp), for example MspA, MspB, MspC or MspD, CsgG, outer membrane porin F (OmpF), outer membrane porin G (OmpG), outer membrane phospholipase A and *Neisseria* autotransporter lipoprotein (NalP) and other pores, such as lysenin. Other β-barrel pores include SP1, hemolytic protein fragaceatoxin C (FraC), secretins such as InvG or GspD, aerolysin, NetB, VdaC (voltage dependent anion channel), VCC (vibrio cholerae cytolysin), anthrax protective antigen, or an ATPase rotor such as C10 Rotor ring of the Yeast Mitochondrial ATPase, K ring of V-ATPase from Enterococcus hirae, C11 Rotor ring of the Ilycobacter tartaricus ATPase, or C13 Rotor ring of the Bacillus pseudofirmus ATPase. Thus, in some embodiments, the transmembrane protein nanopore is selected from MspA, α-hemolysin, CsgG, lysenin, InvG, GspD, leukocidin, FraC, aerolysin, NetB, and functional homologues and fragments thereof. Structures for the transmembrane protein pores are available in protein data banks, for example MspA, α-HL and CsgG are protein data bank entries 1UUN, 7AHL and 4UV3, respectively.

α-helix bundle pores comprise a barrel or channel that is formed from α-helices. Suitable α-helix bundle pores include, but are not limited to, inner membrane proteins and α outer membrane proteins, such as WZA and ClyA toxin.

The nanopore may be a CsgG pore, such as for example CsgG from *E. coli* Str. K-12 substr. MC4100, or a homologue or mutant thereof. Mutant CsgG pores may comprise one or more mutant monomers. The CsgG pore may be a homopolymer comprising identical monomers, or a heteropolymer comprising two or more different monomers. Suitable pores derived from CsgG are disclosed in WO 2016/034591, WO2017/149316, WO2017/149317, WO2017/149318 and International patent application nos. PCT/GB2018/051191 and PCT/GB2018/051858.

The transmembrane protein pore may be derived from lysenin. Suitable pores derived from lysenin are disclosed in WO 2013/153359.

The transmembrane pore may be derived from or based on α-hemolysin (α-HL). The wild type α-hemolysin pore is formed of 7 identical monomers or sub-units (i.e., it is heptameric). An α-hemolysin pore may be α-hemolysin-NN or a variant thereof. The variant may comprise N residues at positions E111 and K147.

The transmembrane protein pore may be derived from Msp, more specifically, from MspA. Suitable pores derived from MspA are disclosed in WO 2012/107778.

In one embodiment, the nanopore is a secretin pore, such as for example GspD or InvG, or a homologue or mutant thereof. Secretin nanopores are described in WO2018/146491.

The transmembrane pore may be a variant of a pore described herein, such as (for example) a variant of Msp, α-hemolysin (α-HL), lysenin, CsgG, ClyA, Sp1 or haemolytic protein fragaceatoxin C (FraC). A variant of a given ("reference") polypeptide is a polypeptide that has an amino acid sequence which varies from that of the reference polypeptide and which retains its ability to form a pore. The ability of a variant to form a pore can be assayed using any method known in the art. For instance, the variant may be inserted into an amphiphilic layer along with other appropriate subunits and its ability to oligomerise to form a pore may be determined. Methods are known in the art for inserting subunits into membranes, such as amphiphilic layers. For example, subunits may be suspended in a purified form in a solution containing a triblock copolymer membrane such that it diffuses to the membrane and is inserted by binding to the membrane and assembling into a functional state. Alternatively, subunits may be directly inserted into the membrane using the "pick and place" method described in M.A. Holden, H. Bayley. J. Am. Chem. Soc. 2005, 127, 6502-6503 and WO 2006/100484.

Over the entire length of a given reference sequence, a variant may be at least 50% homologous to that sequence based on amino acid similarity or identity. More preferably, the variant may be at least 55%, at least 60%, at least 65%, at least 70%, at least 75%, at least 80%, at least 85%, at least 90% and more preferably at least 95%, 97% or 99% homologous based on amino acid similarity or identity to the amino acid reference sequence over the entire sequence. There may be at least 80%, for example at least 85%, 90% or 95%, amino acid similarity or identity over a stretch of 100 or more, for example 125, 150, 175 or 200 or more, contiguous amino acids ("hard homology").

Standard methods in the art may be used to determine homology. For example the UWGCG Package provides the BESTFIT program which can be used to calculate homology, for example used on its default settings (Devereux et al (1984) Nucleic Acids Research 12, p387-395). The PILEUP and BLAST algorithms can be used to calculate homology or line up sequences (such as identifying equivalent residues or corresponding sequences (typically on their default settings)), for example as described in Altschul S. F. (1993) J Mol Evol 36:290-300; Altschul, S.F et al (1990) J Mol Biol 215:403-10. Software for performing BLAST analyses is publicly available through the National Center for Biotechnology Information (http://www.ncbi.nlm.nih.gov/). Similarity can be measured using pairwise identity or by applying a scoring matrix such as BLOSUM62 and converting to an equivalent identity. Since they represent functional rather than evolved changes, deliberately mutated positions would be masked when determining homology. Similarity may be determined more sensitively by the application of position-specific scoring matrices using, for example, PSIBLAST on a comprehensive database of protein sequences. A different scoring matrix could be used that reflect amino acid chemico-physical properties rather than frequency of substitution over evolutionary time scales (e.g. charge).

Amino acid substitutions may be made to the sequences of pores such as Msp, α-hemolysin (α-HL), lysenin, CsgG, ClyA, Sp1 and haemolytic protein fragaceatoxin C ( FraC). For example up to 1, 2, 3, 4, 5, 10, 20 or 30 substitutions may be made. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid.

In the case of an oligomeric transmembrane protein pore, the mutations may be made in each monomeric polypeptide subunit, or any one or more of the monomers. Suitably, in one embodiment of the invention the mutations described are made to all monomers within the oligomeric protein. A mutant monomer is a monomer whose sequence varies from that of a wild-type pore monomer and which retains the ability to form a pore. Methods for confirming the ability of mutant monomers to form pores are well-known in the art.

Any of the proteins described herein, such as the transmembrane protein pores, may be modified to assist their identification or purification, for example by the addition of histidine residues (a his tag), aspartic acid residues (an asp tag), a streptavidin tag, a flag tag, a SUMO tag, a GST tag or a MBP tag, or by the addition of a signal sequence to promote their secretion from a cell where the polypeptide does not naturally contain such a sequence. An alternative to introducing a genetic tag is to chemically react a tag onto a native or engineered position on the pore or construct. An example of this would be to react a gel-shift reagent to a cysteine engineered on the outside of the pore. This has been demonstrated as a method for separating hemolysin hetero-oligomers (Braha et al. (1997) Chem Biol. 4(7): 497-505).

The pore may be labelled with a revealing label. The revealing label may be any suitable label which allows the pore to be detected. Suitable labels include, but are not limited to, fluorescent molecules, radioisotopes, e.g. ¹²⁵I, ³⁵S, enzymes, antibodies, antigens, polynucleotides and ligands such as biotin.

Any of the proteins described herein, such as the transmembrane protein pores, may be made synthetically or by recombinant means. For example, the pore may be synthesised by *in vitro* translation and transcription (IVTT). The amino acid sequence of the pore may be modified to include non-naturally occurring amino acids or to increase the stability of the protein. When a protein is produced by synthetic means, such amino acids may be introduced during production. The pore may also be altered following either synthetic or recombinant production.

Any of the proteins described herein, such as the transmembrane protein pores, can be produced using standard methods known in the art. Polynucleotide sequences encoding a pore or construct may be derived and replicated using standard methods in the art. Polynucleotide sequences encoding a pore or construct may be expressed in a bacterial host cell using standard techniques in the art. The pore may be produced in a cell by *in situ* expression of the polypeptide from a recombinant expression vector. The expression vector optionally carries an inducible promoter to control the expression of the polypeptide. These methods are described in Sambrook, J. and Russell, D. (2001). Molecular Cloning: A Laboratory Manual, 3rd Edition. Cold Spring Harbor Laboratory Press, Cold Spring Harbor, NY.

The pore may be produced in large scale following purification by any protein liquid chromatography system from protein producing organisms or after recombinant expression. Typical protein liquid chromatography systems include FPLC, AKTA systems, the Bio-Cad system, the Bio-Rad BioLogic system and the Gilson HPLC system.

### Movement of the polynucleotide with respect to the nanoreactor

As explained above, the methods of the invention typically involve the movement of a polynucleotide with respect to the nanoreactor, such as with respect to an internal volume within a nanopore and/or a nanovolume around a nanopore.

Movement of a polynucleotide with respect to a nanoreactor can be controlled in any suitable manner.

Movement of the polynucleotide with respect to the nanoreactor (e.g. through a nanopore) may be controlled by means known in the art to enable sufficient time to for example identity regions of the polynucleotide, allow sufficient time for a reaction to modify a desired region. Reactions are discussed herein.

In the methods, the average speed of the polynucleotide movement with respect to the nanoreactor (e.g. as it moves through a nanopore or a nanovolume around a nanopore) may be from about 0.1 nt/s to about 10,000nt/s range, e.g. from about 0.5 to about 5000 nt/s, for example from about 1 to about 1000 nt/s, such as from about 50 to about 500 nt/s, e.g. from about 100 to about 250 nt/s. The speed of the polynucleotide with respect to the nanoreactor can be controlled to allow time for a desired unit of the polynucleotide to be modified as described herein as the polynucleotide moves with respect to the nanoreactor.

The movement of the polynucleotide with respect to the nanoreactor may be smooth, i.e. regular over time. The movement of the polynucleotide with respect to the nanoreactor may be irregular. For example, the polynucleotide may move with respect to the nanoreactor (e.g. may move through a nanopore or through a nanovolume around a nanopore) in a slip-stick stochastic manner, pausing at desired regions for a desired amount of time (eg. to undergo a reaction to modify the polynucleotide), and then moving forwards or backwards to the next region before pausing once again. When moving forwards or backwards between successive pause locations the polynucleotide may move very quickly, for example at greater than 10,000nt/s, as it may not be necessary to limit the speed between pause locations. This form of movement may be commonly employed in methods of the invention which use secondary structures or blockages, discussed further herein, to control the movement.

The speed of the movement of the polynucleotide with respect to the nanoreactor (e.g. as it moves through the nanopore or through a nanovolume around a nanopore) may be controlled by external means, such as by an applied voltage, by heat or by light, or by controlling the amount of enzyme fuel available.

### Secondary structures

The movement of polynucleotides with respect to the nanoreactor may be controlled by secondary structures or hybridized polynucleotide structures within the polynucleotide. Such structures may be particularly useful in embodiments of the invention in which the nanoreactor is a nanopore or a nanovolume around a nanopore.

Examples of secondary structures well known in the art to control the movement of polynucleotides through nanopores include double-stranded regions, hairpin structures, triplex regions, quadruplex structures, etc. When the structures are too large to pass through the nanopore intact some amount of force is required to unwind the structures and translocate the unwound or semi-unwound form through the nanopore.

The amount of force required to unwind the secondary structures depends on factors such as the nature of the polynucleotide, the conditions of the solution (e.g. pH and ionic strength, etc), and the shape and structure of the nanopore. These factors can be altered to control the stability of the secondary structures and in turn to control the duration of pauses in movement.

The amount of force required to unwind the structures for a given system can be calibrated with control experiments. Secondary structures can be designed using common polynucleotide design tools known in the art to control the size and shape of the structure, and the stability of the structure under an applied force.

Figure 5 illustrates how various examples of secondary structures can be used to control the movement of a polynucleotide through a nanopore.

Secondary structures enable slip-stick control of polynucleotide movement through a nanoreactor, such as a nanopore, under the application of an applied voltage. Modulation of the applied voltage can be used to control the movement of the polynucleotide. For example, in a low or zero applied voltage regime, the force will be insufficient to overcome the hybridisation of the secondary structure, so the polynucleotide translocation will pause indefinitely until the voltage is increased above the threshold required for dehybridisation. A low voltage regime may be achieved by the application of a low voltage, for example a voltage below about 60 mV, for example below about 50 mV, more often below about 40 mV such as 30 mV or below, 20 mV or below, or lower such as 10 mV or below. The voltage can be set to a voltage below that required to dehybridise the structure. Such voltages can be experimentally determined, or can be predicted based on knowledge of the secondary structure and the degree of hybridisation therein.

At higher applied voltages above the threshold for dehybridisation, the structures unwind under the force of the applied voltage and translocate through the nanopore. The threshold voltage required for dehybridisation can be experimentally determined, or can be predicted based on knowledge of the secondary structure and the degree of hybridisation therein. Typically, the threshold voltage required for dehybridisation may be at least 30 mV, such as at least 40 mV, at least 50 mV, at least 60 mV, at least 80 mV, at least 100 mV, or more.

Above the threshold voltage, the duration of the pause upon encountering each secondary structure can be controlled by controlling the applied voltage. For example, under moderate applied voltage, for example 40-80mV, the secondary structures may pause for a few seconds on average before unwinding. At much higher voltages, for example greater than 100mV, the structures may pause for less than 1 second before unwinding.

The direction of movement of the polynucleotide with respect to the nanoreactor can be controlled by varying the applied voltage. For example, reversing the polarity of the applied voltage can be used to reverse translocate the polynucleotide back through the nanoreactor (e.g. through a nanopore), for example to revisit a previous region of the polynucleotide to repeat modification to characterise the region. Characterisation is described in more detail herein.

Voltage routines that alter the voltage in response to a scripted protocol or detection of desired change in properties (such as successful modification for example) can be used to control the movement of polynucleotides, for example successively pausing at selected regions to make modifications or detect the characteristics of the polynucleotide using zero or low voltage, then moving the polynucleotide sequence forward or backwards by applying sufficiently high voltage to unwind the secondary structures. Voltage control can therefore be used to pause only at desired locations in a polynucleotide to selectively modify the polynucleotide, for example to encode a pattern of modified and unmodified regions for the purposes of encoding information.

### Trapping

In some methods it may be desirable to temporarily trap the polynucleotide after its movement with respect to the nanoreactor (e.g. after its translocation through a nanopore or a nanovolume around a nanopore) to control the movement of the polynucleotide.

For example, some methods involve the movement of a polynucleotide with respect to a nanoreactor (e.g. through a nanopore) from a cis to a trans compartment. Trapping elements on polynucleotide regions trans of the nanopore can be used to retain the polynucleotide in the nanopore and prevent its loss from the nanopore or reversal of direction in cases where the applied voltage is turned off or the voltage polarity is reversed so that it is acting to pull the polynucleotide back out of the nanopore.

Secondary structures can be used to trap the polynucleotide movement. For example, secondary structures, such as quadruplexes or hairpins, can spontaneously reform after being unwound in order to allow movement of a polynucleotide with respect to a nanoreactor (e.g. through a nanopore). When reformed they can again provide a steric blockage to reverse translocation until sufficient force or other dehybridizing conditions are applied.

Trapping of single-stranded polynucleotides can also be achieved by use of binding molecules. For example, in methods which involve the movement of a polynucleotide from a cis compartment to a trans compartment via a nanoreactor (e.g. by movement through a nanopore), binding molecules can be used to bind to the polynucleotide once translocated through the nanoreactor (e.g. the nanopore) to create a steric blockage to prevent reverse translocation until sufficient conditions are applied to unbind. Examples of suitable binding molecules include hybridising polynucleotides that create sections of double-stranded polynucleotide; proteins such as streptavidin that bind to biotinylated polynucleotide bases, or polynucleotide binding proteins such as single-strand binding proteins (SSBs), recA, etc. Polynucleotide binding proteins are described in more detail herein.

### Rotaxanes

**In** the methods of the invention, the polynucleotide being modified may be trapped in the nanoreactor, such as in a nanopore. Such trapping can be achieved, for example, by attaching large blocking moieties to the ends of the polynucleotide to prevent translocation. Large blocking moieties can be inter-strand crosslinked polynucleotide, large proteins (eg. streptavidin attached to biotinylated polynucleotide), large molecules etc.

Trapping can be useful for storing a modified polynucleotide in a nanoreactor, such as in a nanopore. For example, after modification a polynucleotide might be trapped in a nanopore and stored until the data encoded within the strand needs to be read, then the strand can be re-read by moving it back through the nanopore and characterising the encoded data, as described in more detail herein.

### Polynucleotide binding proteins

Some methods of the invention involve the use of polynucleotide-binding proteins as described herein.

A polynucleotide binding protein may be capable of controlling movement of the polynucleotide with respect to the nanoreactor. It is straightforward in the art to determine whether or not a protein binds to a polynucleotide.

A polynucleotide binding protein (e.g., polynucleotide unwinding enzyme) may be derived from a polynucleotide handling enzyme. A polynucleotide handling enzyme is a polypeptide that is capable of interacting with and modifying at least one property of a polynucleotide. In some disclosed methods, the polynucleotide handling enzyme does not need to display enzymatic activity as long as it is capable of binding the polynucleotide and controlling its movement through the pore. For instance, in such methods, the enzyme may be modified to remove its enzymatic activity or may be used under conditions which prevent it from acting as an enzyme.

The movement of the polynucleotide with respect to the nanoreactor can be controlled with polynucleotide motors, for example polymerases, helicases, translocases, exonucleases, etc. Polynucleotide motors are also referred to herein as polynucleotide handling enzymes.

The polynucleotide handling enzyme is preferably one that is able to process long polynucleotide strands without unbinding from the polynucleotide. Typically, the polynucleotide handling enzyme is capable of moving along a polynucleotide strand of from 500 nucleotide base pairs up to 250 million nucleotide base pairs, such as from 1,000, 2,000, 5,000, 10,000, 50,000 or 100,000 nucleotide base pairs up to 200 million, 100 million, 10 million or 1 million nucleotide base pairs.

Polynucleotide handling enzymes may be derived from a nucleolytic enzyme. Polynucleotide handling enzymes may be derived from a member of any of the Enzyme Classification (EC) groups 3.1.11, 3.1.13, 3.1.14, 3.1.15, 3.1.16, 3.1.21, 3.1.22, 3.1.25, 3.1.26, 3.1.27, 3.1.30 and 3.1.31. The enzyme may be any of those disclosed in WO 2010/086603.

Preferred enzymes are polymerases, exonucleases, helicases, translocases and topoisomerases, such as gyrases.

The nucleic acid handling enzyme may be a polymerase. A polymerase will typically synthesize a complementary polynucleotide strand as it moves along a polynucleotide. Otherwise, a polymerase may be used in a similar manner to a translocase. The polymerase may be a modified polymerase which retains its ability to move along a polynucleotide, but which does not synthesize a complementary strand. Where the nucleic acid handling enzyme is an unmodified polymerase, the enzyme is typically capable of moving along a polynucleotide of up to 30 kb. The distance of movement may be increased by modifying the polymerase to close an opening from which the polynucleotide is able to unbind when the enzyme is part way along the polynucleotide. For such a modified polymerase, the longer polynucleotide lengths specified above may be processed by the polymeraseThe polymerase may be PyroPhage^{®} 3173 DNA Polymerase (which is commercially available from Lucigen^{®} Corporation), SD Polymerase (commercially available from Bioron^{®}) or variants thereof. The polymerase is preferably Bst3.0 or Phi29 DNA polymerase or a variant thereof.

Synthesis of a complementary strand may be advantageous in that it increases the amount of polynucleotide. Increasing the amount of polynucleotide may improve sensitivity of any subsequent assay using the polynucleotide selected by the method. Where the polynucleotide contains modified bases, the polymerase may be used to synthesize a complementary strand that contains normal bases, which can also be advantageous for subsequent assays using the polynucleotide.

Using a polymerase may have the advantage that it can be used to distinguish a damaged polynucleotide from an undamaged polynucleotide. For example, the polymerase may be unable to pass through an abasic nucleotide in DNA or through thymadine dimers. Accordingly, a method using a polymerase may be used to separate damaged polynucleotides from undamaged polynucleotides.

A topoisomerase may be a member of any of the Moiety Classification (EC) groups 5.99.1.2 and 5.99.1.3.

The helicase may, for example, be a member of superfamily 1 or superfamily 2. The helicase is preferably a member of one of the following families: Pif1-like, Upf1-like, UvrD/Rep, Ski-like, Rad3/XPD, NS3/NPH-II, DEAD, DEAH/RHA, RecG-like, REcQ-like, T1R-like, Swi/Snf-like and Rig-I-like. The first three of those families are in superfamily 1 and the second ten families are in superfamily 2. The helicase is more preferably a member of one of the following subfamilies: RecD, Upf1 (RNA), PcrA, Rep, UvrD, Hel308, Mtr4 (RNA), XPD, NS3 (RNA), Mss116 (RNA), Prp43 (RNA), RecG, RecQ, T1R, RapA and Hef (RNA). The first five of those subfamilies are in superfamily 1 and the second eleven subfamilies are in superfamily 2. Members of the Upf1, Mtr4, NS3, Mss116, Prp43 and Hef subfamilies are RNA helicases. Members of the other subfamilies are DNA helicases.

The helicase may be a multimeric or oligomeric helicase. In other words, the helicase may need to form a multimer or an oligomer, such as a dimer, to function. In such embodiments, the two or more parts cannot be on different monomers. The helicase is preferably monomeric. In other words, the helicase preferably does not need to form a multimer or an oligomer, such as a dimer, to function. For example, Hel308, RecD, TraI and XPD helicases are all monomeric helicases. These are discussed in more detail below. Methods for determining whether or not a helicase is oligomeric/multimeric or monomeric are known in the art. For instance, the kinetics of radiolabelled or fluorescently-labelled polynucleotide unwinding using the helicase can be examined. Alternatively, the helicase can be analysed using size exclusion chromatography.

Monomeric helicases may comprise several domains attached together. For instance, TraI helicases and TraI subgroup helicases may contain two RecD helicase domains, a relaxase domain and a C-terminal domain. The domains typically form a monomeric helicase that is capable of functioning without forming oligomers.

Particular examples of suitable helicases include Hel308, NS3, Dda, UvrD, Rep, PcrA, Pif1 and TraI. These helicases typically work on single stranded DNA. Examples of helicases that can move along both strands of a double stranded DNA include FtsK and hexameric enzyme complexes, or multi subunit complexes such as RecBCD.

The helicase may, for example, be any of the helicases, modified helicases or helicase constructs disclosed in WO 2013/057495, WO 2013/098562, WO2013098561, WO 2014/013260, WO 2014/013259, WO 2014/013262 and WO/2015/055981. The Hel308 helicase preferably comprises any one or more of the modifications disclosed in WO 2014/013260. The Dda helicase preferably comprises any one or more of the modifications disclosed in WO 2015/055981 and/or WO 2016/055777.

The nucleic acid handling enzyme may be an exonuclease. Typically, if the nucleic acid handling enzyme is an exonuclease the exonuclease is in an inactive form so that it does not digest the polynucleotide as it moves along it. Any exonuclease enzyme may be used in the method. Preferred enzymes for use in the method include exonuclease III enzyme from *E. coli,* exonuclease I from *E. coli,* bacteriophage lambda exonuclease and enzymes derived from exonuclease III enzyme from *E. coli,* exonuclease I from *E. coli,* bacteriophage lambda exonuclease. An enzyme derived from one of these exonucleases preferably comprises the domains responsible for binding to the nucleic acid and for digesting the nucleic acid (catalytic domain).

The motors can be either single-stranded motors or double-stranded motors such as FtsK, phage pumps.

Movement can be controlled with individual motors, multiple units of the same motor, or combinations of motors. In some methods disclosed herein, any number of polynucleotide motors may be used. For instance, 1, 2, 3, 4, 5, 6, 7, 8, 9, 10 or more polynucleotide motors may be used. In some disclosed methods, different numbers of polynucleotide motors may be used. Any combination of two or more of the polynucleotide motors mentioned above may be used. Preferred polynucleotide motors are helicases as discussed above. For example, the two or more helicases may be two or more Dda helicases. The two or more helicases may be one or more Dda helicases and one or more TrwC helicases. The two or more helicases may be different variants of the same helicase. The two or more helicases may be preferably attached to one another. The two or more helicases may more preferably be covalently attached to one another. The helicases may be attached in any order and using any method. Preferred helicase constructs for use in such methods are described in WO 2014/013260, WO 2014/013259, WO 2014/013262 and WO2015/055981.

The type of motor, modifications to the motors, and running conditions can be selected by means well known in the art to control the speed of the movement. Conditions that can be altered include mutations in the motors, pH, salt conentrations, fuel or cofactor concentration, etc. The speed of the movement is preferably from 0.1 to 10,000nt/s, is most preferably from 1 to 1000nt/s.

The direction of polynucleotide movement through the nanopore can be controlled through selection of the motor and orientation of the polynucleotide in the nanopore. For example, see the discussion in PCT/GB2012/052579. For example, the polynucleotide can be orientated so that either the 5' end or the 3' enters the pore first. This is termed 5'down or 3'down respectively. The motor/s can be selected based on their direction of movement, 5' to 3' or 3' to 5' along the polynucleotide. By selecting the desired motor and capturing the polynucleotide in the nanopore in the desired direction it is possible to either feed the polynucleotide into the nanopore in the same direction as the applied voltage, or pull the polynucleotide back out of the nanopore against the applied voltage.

For example, proteins may be modified such that they bind polynucleotides *(i.e.* retain polynucleotide binding ability) but do not function as a translocase (*i.e*. do not move along polynucleotides when provided with all the necessary components to facilitate movement, (e.g. ATP and Mg²⁺). Such modifications are known in the art. For instance, modification of the Mg²⁺ binding domain in helicases typically results in variants which do not function as helicases. These types of variants may act as molecular brakes. As used herein, a molecular brake is a protein or molecule that interacts strongly enough with the polynucleotide (eg. by means of steric blockages that distort the polynucleotide, or other strong binding interactions including electrostatic binding, hydrogen bonding, Pi-stacking) such as to prevent free movement of the polynucleotide when a force is applied to it.

When a helicase is not provided with the necessary components to facilitate movement or is modified to hinder or prevent its movement, it can bind to the polynucleotide and act as a brake slowing the movement of the polynucleotide when it is pulled into the pore by the applied field. In the inactive mode, it does not matter whether the polynucleotide is captured either 3' or 5' down, it is the applied field which pulls the polynucleotide into the pore towards the trans side with the enzyme acting as a brake. When in the inactive mode, the movement control of the polynucleotide by the helicase can be described in a number of ways including ratcheting, sliding and braking. Helicase variants which lack helicase activity can also be used in this way.

A polynucleotide handling enzyme may be covalently attached to a nanoreactor e.g to a pore. The polynucleotide may be attached to the pore at more than one, such as two or three, points. Attaching the pore to the enzyme at more than one point can be used to constrain the mobility of the enzyme. For instance, multiple attachments may be used to constrain the freedom of the enzyme to rotate or its ability to move away from the pore. A nanopore can be covalently attached to the enzyme using any method known in the art. The nanopore and enzyme may be produced separately and then attached together. The two components may be attached in any configuration. For instance, they may be attached via their terminal (i.e. amino or carboxy terminal) amino acids. Suitable configurations include, but are not limited to, the amino terminus of the enzyme being attached to the carboxy terminus of the subunit and *vice versa.* Alternatively, the two components may be attached via amino acids within their sequences. For instance, the enzyme may be attached to one or more amino acids in a loop region of a subunit of the pore. In a preferred embodiment, terminal amino acids of the enzyme are attached to one or more amino acids in the loop region of a subunit of the pore.

A nanopore may be genetically fused to the polynucleotide handling enzyme. A nanopore is genetically fused to an enzyme if the whole construct is expressed from a single polynucleotide sequence. The coding sequences of the nanopore and enzyme may be combined in any way to form a single polynucleotide sequence encoding the construct. The nanopore and enzyme may be genetically fused in any configuration. The nanopore and enzyme may be fused via their terminal amino acids. For instance, the amino terminus of the enzyme may be fused to the carboxy terminus of the nanopore and *vice versa.* The amino acid sequence of the enzyme is preferably added in frame into the amino acid sequence of the nanopore. In other words, the enzyme is preferably inserted within the sequence of the nanopore. In such embodiments, the nanopore and enzyme are typically attached at two points, i.e. via the amino and carboxy terminal amino acids of the enzyme. If the enzyme is inserted within the sequence of the nanopore, it is preferred that the amino and carboxy terminal amino acids of the enzyme are in close proximity and are each attached to adjacent amino acids in the sequence of the nanopore or variant thereof. In a preferred embodiment, the enzyme is inserted into a loop region of the nanopore.

A nanopore may be chemically fused to the polynucleotide handling enzyme. A nanopore is chemically fused to an enzyme if the two parts are chemically attached, for instance via a linker molecule.

A nanopore may be transiently attached to the polynucleotide handling enzyme by a hex-his tag or Ni-NTA (e.g. as discussed in WO 2010/004265). The nanopore and enzyme may also be modified such that they transiently attach to each other.

A nanopore may be attached directly to the polynucleotide handling enzyme. The nanopore is preferably attached to the enzyme using one or more, such as two or three, linkers. The one or more linkers may be designed to constrain the mobility of the enzyme. The linkers may be attached to one or more reactive cysteine residues, reactive lysine residues or non-natural amino acids in the nanopore and/or enzyme. Suitable linkers are well-known in the art. Suitable linkers include, but are not limited to, chemical crosslinkers and peptide linkers. Preferred linkers are amino acid sequences (i.e. peptide linkers). The length, flexibility and hydrophilicity of the peptide linker are typically designed such that it does not to disturb the functions of the nanopore and enzyme. Preferred flexible peptide linkers are stretches of 2 to 20, such as 4, 6, 8, 10 or 16, serine and/or glycine amino acids. More preferred flexible linkers include (SG)₁, (SG)₂, (SG)₃, (SG)₄, (SG)₅ and (SG)₈ wherein S is serine and G is glycine. Preferred rigid linkers are stretches of 2 to 30, such as 4, 6, 8, 16 or 24, proline amino acids. More preferred rigid linkers include (P)₁₂ wherein P is proline.

Linkers may be attached to the nanopore first and then the polynucleotide handling enzyme, the enzyme first and then the nanopore or the enzyme and nanopore at the same time. When the linker is attached to the nanopore, it may be a monomeric nanopore, part of an oligomer of two or more monomers or part of complete oligomeric pore. It is preferred that the linker is reacted before any purification step to remove any unbound linker.

### Molecular brake

As discussed above, a molecular brake may be any compound or molecule which binds to the polynucleotide and slows the movement of the polynucleotide through a pore. A molecular brake may be any of those discussed above. A molecular brake preferably comprises a compound which binds to the polynucleotide. The compound is preferably a macrocycle. Suitable macrocycles include, but are not limited to, cyclodextrins, calixarenes, cyclic peptides, crown ethers, cucurbiturils, pillararenes, derivatives thereof or a combination thereof. The cyclodextrin or derivative thereof may be any of those disclosed in Eliseev, A. V., and Schneider, H-J. (1994) J. Am. Chem. Soc. 116, 6081-6088. The cyclodextrin is more preferably heptakis-6-amino-β-cyclodextrin (am₇-βCD), 6-monodeoxy-6-monoamino-β-cyclodextrin (am₁-βCD) or heptakis-(6-deoxy-6-guanidino)-cyclodextrin (gu₇-βCD). Other suitable molecular brakes include polynucleotide binding proteins, or inactivated helicases/polymerases/exonucleases etc that would normally act on or move along the polynucleotide and which prevent or retard the movements of the polynucleotide strand through a pore.

### Closed-complexes

The enzyme may be modified or unmodified. The enzyme may be modified to form a closed-complex. A closed-complex is an enzyme in which the polynucleotide binding site is modified such that the enzyme is closed around the polynucleotide in such a way that the enzyme does not fall off the polynucleotide other than when it reaches the end of the polynucleotide. Closed structures can prevent unwanted unbinding of the motors, enabling longer polynucleotides to be processed. Closed structures also enable motors to pass through unnatural polynucleotide regions, such as abasic sites, without unbinding. Examples of suitable closed-complex enzymes and methods for modifying enzymes to produce closed complexes are disclosed in, for example, WO 2014/013260 and WO 2015/055981.

### Enzyme-pore constructs

In some embodiments, for either active or inactive enzyme control, the enzyme is linked to the nanopore on either or both of the cis and trans entrances to the nanopore. If the nanopore is large enough to accommodate the motor in the internal channel, then the motor may be linked inside the nanopore.

### Controlling polynucleotide movement using heat

The movement of the motor can be controlled by various means while on the nanopore. For example, in some embodiments it may be desirable to pause or slow the motor, so as to undertake reactions to modify the polynucleotide, and then restart or speed up the motor again to move to successive regions along the polynucleotide.

The speed of the motor can be controlled by modifying the local environment around the nanopore, for example by altering the fuel or cofactor conditions, the pH, the temperature etc.

For example, local temperature in or near a nanopore can be controlled by illuminating the nanopore directly or by exciting a nearby nanoparticle or plasmonic guide, eg. a gold nanoparticle linked to the nanopore (see Yamazaki et al, Nano Letters 2017 17 (11), 7067-7074 and Reiner et al, J Am Chem Soc. 2013 Feb 27;135(8):3087-94). These methods can be used enhance temperature changes in a localised region when a system is illuminated over a broad area. When illuminated the systems can create a localised heating, the extent and range of which can be controlled by design of the system and strength of illumination, while the ambient temperature of the rest of the bulk environment is not significantly altered. When the light is switched off the local temperature increase quickly returns to ambient (eg. in less than 1 second) as the heat diffuses away. By controlling aspects such as the ambient temperature, the properties of the nanopore system with or without optical enhancers, and the illumination conditions, it is possible to tightly control the local temperature that the nanoreactor experiences. This can be used to control, for example, to movement of polynucleotide or the reaction conditions for modifying the polynucleotide.

Local temperature changes can be used to control the movement of the polynucleotide through a nanopore. For example, temperature control can be used to control the reaction kinetics of enzymes that process a fuel such as ATP to move along a polynucleotide. It is known in the art that the kinetic rates of various steps in the enzymatic cycle (including steps of NTP binding, NTP hydrolysis, enzyme isomerisation, product unbinding) of polynucleotide processing enzymes are dependent on temperature. If not known beforehand, the rates of substeps in an enzymatic cycle and/or the average rate of full NTP turnover cycle can be determined by various experimental means known in the art. Since NTP hydrolysis tightly regulates the speed at which many polynucleotide enzymes move along polynucleotides, temperature changes can be used to control the average speed of that a polynucleotide motor moves along the polynucleotide and therefore the speed that the polynucleotide moves through the nanopore. Therefore, for example, a plasmonic guide near to an enzyme motor can be illuminated to control the local temperature to control the speed of the enzyme. For example, when not illuminated the ambient conditions can be set so that the speed of the motor is slow or stopped. The illuminated state can be configured to heat the motor to then start or increase the speed of movement. The extent and duration of illumination can be used to control the extent of speed change and the approximate length of polynucleotide the enzyme moves along respectively.

Temperature control can also be used to control the binding or sliding of active or inactive motors. For example, under low temperature the motor may bind strongly to the polynucleotide, and under high temperature the motor may bind weakly to the polynucleotide. Therefore under a fixed applied voltage that applies an approximately constant force on the polynucleotide, the movement of the polynucleotide can be regulated by adjusting the strength of the enzyme-polynucleotide binding interaction. For example, the conditions can be controlled such that under low ambient temperature the enzyme will bind strongly and the movement of the polynucleotide can be slowed or stopped. Increasing the temperature via illumination of a plasmonic guide can then be used to reduce the strength of enzyme-polynucleotide interaction, allowing the polynucleotide to slip faster through the nanopore.

Temperature can also be used to melt secondary structures. Therefore, in cases where secondary structures are used to control movement the application of heat can be used to melt secondary structures to move the polynucleotide forwards in an applied voltage field.

A system can be configured to illuminate an array of nanopores with attached plasmonic guides, illuminating parts of the array or the entire array at regular or irregular intervals to control polynucleotide movement. For example, an array of nanopores can be fully illuminated at a fixed periodic interval, so that each polynucleotide in each polynucleotide-nanopore complex moves forward synchronously. This can be coupled with asynchronous modification (for example by chemical means by altering the voltage) to selectively modify each polynucleotide in the desired region.

### Controlling polynucleotide movement using voltage

Voltage can be used to control the movement of polynucleotides through a nanopore. The magnitude of the applied voltage is proportional to the amount of force applied to a charged polynucleotide. The amount of force applied depends on the magnitude of the voltage, the system conditions (salt concentration, pH, temperature), and nanopore.

### Reaction Conditions

The methods of the invention comprise selectively modifying portions of the polynucleotide strand. In some embodiments, such methods comprise selectively controlling the number of nucleotides that are modified within each portion of the polynucleotide strand. For example, in some embodiments 1 nucleotide may be selectively modified. In other embodiments more than one nucleotide may be selectively modified; for example from 1 to about 1000 nucleotides, such as from about 10 to about 500, e.g. from about 50 to about 250 nucleotides may be selectively modified. Different portions of the polynucleotide strand may have different numbers of modified nucleotides and thereby by distinguished.

In other embodiments the methods comprise selectively controlling the extent of the modifications made to the nucleotides that are modified within each portion of the polynucleotide strand. For example, in some embodiments extensive modifications may be made to nucleotides within the portion of the polynucleotide strand which is within the nanoreactor when the portion is within the nanoreactor for a prolonged time period, whereas other portions of the polynucleotide strand may be within the nanoreactor for a shorter period and be less extensively modified. Different portions of the polynucleotide strand may thus be modified to different extents and thereby by distinguished.

In some embodiments, selectively modifying portions of the polynucleotide strand comprises (i) scission of part of the polynucleotide strand, e.g. scission of one or more side chains on the polynucleotide strand; (ii) modification of the strand, e.g. modification of one or more side chains on the strand; and/or (iii) addition to the strand, e.g. additional of pendant chemical groups to the backbone or sidechains of the residues on the polynucleotide strand. When selectively modifying the polynucleotide strand comprises a scission reaction, the reaction is typically not scission of the polynucleotide backbone such that the overall strand length remains unaltered by the modification reaction.

In the invention, selectively modifying portions of the polynucleotide strand typically comprises subjecting the portions of the polynucleotide strand in the nanoreactor to reaction conditions comprising (i) the presence, absence or concentration of one or more chemical reagent(s); (ii) the engagement of an enzyme with the polynucleotide strand under conditions that the enzyme modifies the nucleotides within the polynucleotide strand; (iii) the presence or absence of electromagnetic radiation; and/or (iv) the presence or absence of applied heat.

In some embodiments, modification of the polynucleotide strand in the nanoreactor can be controlled by controlling the presence, absence or concentration of one or more chemical reagent(s) in the nanoreactor by applying an electrical or chemical potential across the nanoreactor. For example, when charged reagents are used then a chemical potential can be used to control the movement of such species into the nanoreactor and thus to control the concentration of such reagents in the nanoreactor. Ionic flux conditions through a nanopore can be altered by changing the applied voltage, so a chemical potential or voltage control can be used regardless of whether the chemical reagents are charged or not. Both a chemical and electrical potential can be used.

Typically, such chemical reagents comprise at least a first reagent and a second reagent. The first and second reagents react with the portion of the polynucleotide strand in the nanoreactor. However, the invention is not limited to embodiments in which multiple reagents are used. The invention may involve the use of only one chemical reagent to modify the polynucleotide, or multiple reagents may be used. In an exemplary embodiment two reagents are used.

There are many suitable combinations of reagents which react to modify polynucleotides, such as combinations of metal ions and oxidising agents such as peroxide etc. In the invention, any suitable modification can be detected. For example, the modification can be made to the backbone, the sugar, and/or the bases of polynucleotides in the nanoreactor.

In some embodiments a polynucleotide in the nanoreactor is modified by a click chemical reaction. In some embodiments the click reaction is between a reactive group on the polynucleotide and a reactive molecule in solution or attached to the nanoreactor. Click chemistry conducted within nanopore nanoreactors has been described in e.g. Haugland et al, "Synthetically Diversified Protein Nanopores: Resolving Click Reaction Mechanisms" ACS Nano 2019. Examples of click chemical reactions and reagents are described in more detail herein.

In the methods of the invention, it is typically desired that the polynucleotide external to the nanoreactor is not modified whereas the portion of the polynucleotide within the nanoreactor is modified as described herein. This can be achieved by localising chemical reagents in the nanoreactor. Localisation of chemical reagents in the nanoreactor can be achieved by providing the reagents precisely to the nanoreactor or by synthesizing the reagents in the nanoreactor. Localisation of chemical reagents in the nanoreactor can also be achieved by the use of protectants to remove the chemical reagents from the environment external to the nanoreactor. One or more protectant(s) is thus typically provided external to the nanoreactor to minimise or prevent modification of the polynucleotide by chemical reagent(s) external to the nanoreactor.

Suitable protectants are known in the art and any suitable protectant can be used, depending on the nature of the chemical reagent which it is desired to target, for example chelating agents, antioxidants, oxygen scavengers, reducing agents, etc. Particularly suitable protectants include chelating agents. Suitable chelating agents are known for a wide variety of chemical reagents including both cations and anions. For example, chelating agents such as EDTA are useful in removing metal ions from the environments external to a nanoreactor (e.g. from the enviroments outside the internal cavity of a nanopore). Other suitable chelating agents include vitamin B12, citric acid, crown ethers, calixarenes, and the like.

When a protectant is used in the methods of the invention, the protectant is typically used in excess relative to the amount of the chemical reagent at issue. The protectant is often present in an amount at least twice, such as at least 5 times, at least 10 times, at least 100 times, or at least 1000 times the concentration of the chemical reagent at issue.

The protectant is often located in a different compartment of a system to the bulk of the chemical reagent it is intended to sequester. For example, in methods of the invention in which metal ions are used to modify the polynucleotide, the metal ions are often located trans of a nanoreactor such as a nanopore in a membrane, such that when a positive voltage is applied to the trans side of the nanopore the metal ions flow from the trans compartment to the cis compartment through the nanopore. **In** such cases, a protectant such as EDTA is typically present in the cis compartment in order to sequester metal ions that flow through the nanopore such that polynucleotide in the cis compartment is not modified apart from when it is within the nanopore.

In some embodiments a chemical sensitizer is used to facilitate the redox modification of the polynucleotide strand in the nanoreactor. **In** some embodiments a redox mediator is used as the chemical sensitizer. Examples include peroxide, ascorbate, etc.

In other embodiments, the polynucleotide may be modified in the nanoreactor by using a polynucleotide-modifying enzyme. Many different polynucleotide-modifying enzymes are known in the art and are suitable for use in the methods disclosed herein. Polynucleotide modifying enzymes are discussed herein in more detail.

Typically, in embodiments of the invention which use a polynucleotide-processing enzyme, the polynucleotide-processing enzyme modifies the base portion of nucleotides within the polynucleotide strand. However, the modification can be made to the "backbone" of the polynucleotide strand e.g. to the phosphodiester linkages and/or to the sugars.

In some embodiments, modification of the polynucleotide by the polynucleotide-processing enzyme is controlled by controlling the presence, absence or concentration of fuel and/or substrate for the enzyme. For example, for an NTP-driven nucleic acid modifying enzyme controlling the concentration of NTPs (e.g. ATP) in the nanoreactor can allow the modification of the polynucleotide by the polynucleotide-processing enzyme.

Some polynucleotide-processing enzymes respond to conformational changes induced with physical force is exerted on the polynucleotide substrate. The modification or otherwise of the polynucleotide can thus be controlled by controlling a force exerted on the polynucleotide. Suitable forces include electrophoretic forces applied by applying a voltage across the nanoreactor e.g. across a nanopore in a membrane. Varying the voltage applied leads to a variation in the force applied on the polynucleotide-processing enzyme and controls the activity of the enzyme in modifying the polynucleotide.

Some polynucleotide processing enzymes function selectively on either only single- or double-stranded polynucleotide. The nanoreactor can be used to control the hybridised structure of the polynucleotide to enable selective binding and modification when desired. For example, a DNA strand translocating through a nanopore can be paused as desired long enough for a short secondary polynucleotide to hybridise to form a dsDNA region, to which a ds-DNA selective enzyme can bind to modify the DNA (eg. a DNA-methyltransferase that modifies specific DNA bases).

In some embodiments the methods comprise contacting the portions of the polynucleotide strand in the nanoreactor with electromagnetic radiation in the form of light, preferably visible or ultraviolet light. Light may be applied to the nanoreactor using any suitable means. Fibre optics may be used to deliver the light to the nanoreactor. A laser may be used to irradiate the nanoreactor.

In some embodiments the methods involve irradiating a photosensitizer in the nanoreactor for modification of the polynucleotide strand. Any suitable photosensitizer can be used. Suitable photosensitizers include porphyrins and the like. In some embodiments the sensitizer is a light-excited molecule. In some embodiments the sensitizer is stable under excitation.

In some embodiments the sensitizer is an inorganic based nanoparticle or organic based dye. The sensitizer may be chosen for its specific properties including: size, excitation wavelengths, emission wavelengths, reactive properties upon exposure, surface chemistry, etc. The sensitizer may be any re-emitting light source known in the art, that when illuminated globally will re-emit light locally. Well known examples include organic dyes and fluorophores, particles such nanodots, etc.

In some embodiments the methods involve irradiating a sensitizer in the nanoreactor and transferring radiation from the sensitizer to the polynucleotide strand in the nanoreactor for modification of the polynucleotide strand. For example, the sensitizer may be a metal nanoparticle, and/or wherein said radiation is electromagnetic radiation or thermal radiation.

In some embodiments a photosensitizer may comprise a redox enzyme or cofactor thereof. In some embodiments a photosensitizer may comprise a nanoparticle. In some embodiments a photosensitizer may comprise an organic or inorganic dye. Suitable dyes include acridine orange, methylene blue, etc.

In some embodiments the sensitizer is an organic molecule such as riboflavin.

In some embodiments the sensitizer is attached to the nanoreactor. In some embodiments the nanoreactor is a nanopore and the sensitizer is attached to a surface of the nanopore.

In some embodiments the sensitizer is attached to the outer portions of the nanopore, thus in the vicinity of polynucleotide entering or exiting the nanopore.

In some embodiments the sensitizer is attached to an internal surface of the nanopore, e.g. a surface of the channel running through the nanopore. This can be beneficial as it increasing the chance of selectively modifying only the photocleavable element nearby and not other photocleavable elements positioned along the typically very flexible polynucleotide molecules that might otherwise transiently come near to the sensitizer unless occluded. An additional advantage of placing the sensitizer inside the nanopore is that the reaction can occur at or near a reader if placed correctly, so that a change in current upon modification can be detected in situ.

In some embodiments the sensitizer is placed near the entrance or exit to nanopore, or in constriction regions. For example, many biologically derived nanopores with a wide range of internal dimensions are known, with internal cavities ranging from <1nm to >10nm in diameter. Well known examples of include alpha-hemolysin, MspA, CsgG, Aerolysin, Phi29 portal, etc. Likewise a range of small sensitizers are well known in the art (typically <10nm, preferably 1-4nm in diameter).

In some embodiments the sensitizer is attached directly to the nanopore, e.g. by covalent attachment to reactive amino-acids, for example employing disulphide or maleimide chemistry.

In some embodiments the sensitizer is a nanoparticle and is attached to a nanopore nanoreactor. Attachment of nanoparticles to alpha-hemolysin is demonstrated in Reiner et al, J. Am. Chem. Soc., 27 (135) 2013. Internalisation of small nanoparticles in nanopores is demonstrated in Chavis et al, ACS Sensors, 2017 (doi: 10.1021/acssensors.7b00362)

In some embodiments the sensitizer is attached to the nanopore in a position to engage with a photocleavable group on the polynucleotide strand being modified. For example, in some embodiments the sensitizer is positioned within about 10 nm, e.g. within about 5 nm, such as within about 3 nm, e.g. within about 2nm, such as within about 1 nm of a photocleavable group on the strand.

In some embodiments the sensitizer is attached to the nanoreactor, e.g. to a nanopore, by maleimide chemistry, e.g. by attachment to cysteines in a nanopore. For example, in some embodiments the nanoreactor is a CsgG nanopore and the sensitizer is conjugated to a cysteine mutation introduced at a suitable residue in the CsgG nanopore. Suitable positions on other protein nanopores can also be readily identified by those skilled in the art.

The modification of the polynucleotide in the nanoreactor can be achieved using heat. The heat can be applied using any suitable means. For example, local heat can be applied by transferring heat to the polynucleotide from an irradiated sensitizer. The sensitizer may be a plasmonic guide, such as a gold nanoparticle linked to the nanopore. As discussed above, plasmonic guides can be used enhance temperature changes in a localised region when a system is illuminated over a broad area. When illuminated a plasmonic guide creates a localised heating (within a few 10s of nanometers of the plasmonic guide), while the ambient temperature of the rest of the bulk environment is not significantly altered. When the light is switched off the local temperature increase quickly returns to ambient (often in less a few seconds) as the heat diffuses away. Plasmonic guides linked to or near to a nanopores or motor can therefore be used to alter the local temperature, e.g. by up to tens of degrees. The control of the local temperature in the nanoreactor can thus be used to control the modification of polynucleotides within the nanoreactor.

In some embodiments a nanoreactor setup, e.g. an array of nanoreactors may be globally subjected to reaction conditions to modify the portions of the polynucleotide within each nanoreactor in the array. The global reaction conditions might include for example (as described in more detail herein) irradiation with light, heating, contact with one or more chemical reagents (e.g. by flushing a flow cell with buffer containing reactive components). The duration and pattern of each reaction exposure can be varied to control the pattern of modifications.

In some embodiments the reaction condition is a pulse of irradiation at a specific frequency. For example, as described in more detail herein, a light pulse may be used to excite a sensitizer on the nanopore that re-emits radiation locally to cleave an adjacent photocleavable side-chain on the DNA unit in the nanopore.

### Data reading

The methods of the invention may further comprise the step of:
(C) determining the pattern of selective modifications on the polynucleotide strand.

As explained above, such methods typically involve determining the pattern of selective modifications on the polynucleotide strand by determining the presence, absence, extent or properties of modifications made to the polynucleotide strand.

In some embodiments the methods comprise characterizing the modified polynucleotide strand. Such characterisation is distinct from determining the presence, absence, extent or properties of modifications made to the polynucleotide strand. The methods of the invention may comprise determining the presence, absence, extent or properties of modifications made to the polynucleotide strand but not further characterizing the modified polynucleotide strand. The methods of the invention may comprise characterizing the modified polynucleotide strand but not determining the presence, absence, extent or properties of modifications made to the polynucleotide strand. The methods of the invention may beneficially comprise both determining the presence, absence, extent or properties of modifications made to the polynucleotide strand and characterizing the modified polynucleotide strand.

As explained above, characterizing the modified polynucleotide strand can allow information about the polynucleotide strand "backbone", e.g. its sequence or the percentage content of any specific nucleotide(s) to be determined. Determining the presence, absence, extent or properties of modifications made to the polynucleotide strand can allow the data that is encoded as a separate "layer" above this to be read. Characterizing the modified polynucleotide strand, e.g. by sequencing it can be useful in terms of allowing the positions of modifications that are made to the polynucleotide strand to be determined. This can be useful when decoding data that is encoded on the strand.

Typically, in the methods, determining the pattern of selective modifications on the polynucleotide strand and/or characterizing the modified polynucleotide strand comprises:
i) contacting a detector with the modified polynucleotide strand such that the polynucleotide strand moves with respect to the detector; and
ii) taking one or more measurements which are indicative of one or more properties of the modified polynucleotide strand as the polynucleotide strand moves with respect to the detector.

In the methods of the invention, the detector can be any suitable detector. For example, electrical or optical detection of the modified polynucleotides can be used as described in more detail herein. Often, the detector is a transmembrane pore. Advantageously, the use of a nanopore system for reading data encoded on the polynucleotide strand allows the use of polynucleotide modifications that cannot be read using non-nanopore sequencing methods.

In such embodiments, the methods often comprise:
a) contacting the pore with the modified polynucleotide strand;
b) applying a potential difference across the pore; and
c) taking one or more measurements which are indicative of one or more
properties of the modified polynucleotide strand moving with respect to the pore and thereby determining the pattern of selective modifications on the polynucleotide strand and/or characterizing the modified polynucleotide strand.

Any suitable properties can be measured during such methods. Often, the measurements which are indicative of the properties of the modified polynucleotide are current measurements. The methods of the invention may therefore comprise measuring the current passing through the transmembrane pore as the modified polynucleotide strand moves with respect to the transmembrane pore.

Often, in methods of the invention, the nanoreactor comprises a nanopore; and determining the pattern of selective modifications on the polynucleotide strand and/or characterizing the modified polynucleotide comprises (i) contacting the *same* nanopore with the modified polynucleotide strand such that the polynucleotide strand moves with respect to the nanopore; and (ii) taking one or more measurements which are indicative of one or more properties of the modified polynucleotide strand as the polynucleotide strand moves with respect to the nanopore. Those skilled in the art will thus recognise that in these embodiments the same nanopore is used as the nanoreactor and as the detector. **In** other words, a nanopore (the same nanopore) can act as both a "write head" for encoding data on a polynucleotide strand as it moves with respect to the nanopore, and as a "read head" for reading the data thereby encoded on the polynucleotide strand. The data can be read as the modifications are made to the polynucleotide strand (i.e. as the data is encoded) or at a subsequent stage, for example after storing the modified polynucleotide strand.

It is understood that the modified polynucleotide may have to be processed using means well known in the art prior to detection. For example, for some nanopore readout methods it may be necessary to attach sequencing adapters with motors for controlling the movement of the polynucleotide through the nanopore.

Other sequencing technologies can also be used to characterise a modified polynucleotide in accordance with the invention. For example, the modified polynucleotide can be characterised using Sanger sequencing. More often the modified polynucleotide is characterised using parallelised next-generation sequencing technologies. Any suitable sequencing technique can be used. The polynucleotide may have to be processed in advance of read-out with some technologies. For example the polynucleotide may need to be amplified using conventional polymerase amplification techniques. If amplified or copied the information encoded in the modified polynucleotide needs to directly or indirectly propagate to the copies of the original strand.

### Processing of DNA

In some embodiments the polynucleotide remains unaltered, and remains trapped in the nanopore for read-out in the same nanopore at a later point. Alternatively the modified polynucleotide is removed from the nanopore, and then stored without further processing in a small volume, for example in an aqueous droplet or a microwell.

In some embodiments the modified polynucleotide undergoes further processing steps. For example, the polynucleotide might be amplified, cut in segments, linked to other polynucleotides, changed from single-stranded into double-stranded form (Eg. by polymerase fill-in), etc.

The polynucleotide can be processed to further modify the strand. For example, by exposing the polynucleotide to conditions that react either the modified or unmodified portions. Processing the polynucleotide may be for the purposes of stabilising the modification chemistry for example, such as in the case that the initial modification is not stable for extended long-term storage.

The polynucleotide may be processed by amplification using conventional polymerase amplification techniques well known in the art. If amplified or copied the information encoded in the modified polynucleotide should directly or indirectly propagate to the copies of the original strand. Amplification may be employed for the purposes of creating a copy of the original information encoded in the modified strand. Alternatively amplification may be employed to alter the polynucleotide or magnify the quantity so that it is in a state suitable for downstream processes, for example readout on some technologies such as Sanger sequencing.

### Systems and uses

Also provided is a system for encoding data on a polynucleotide strand, the system comprising a nanoreactor configured to selectively modify portions of a polynucleotide strand as the polynucleotide strand moves through the nanoreactor. In some embodiments, the system comprises a nanopore for controlling the movement of the polynucleotide strand through the nanoreactor and/or a polynucleotide for modification in the nanoreactor

Also provided is a system for reading data encoded on a polynucleotide strand; the system comprising a detector configured to determine the pattern of selective modifications on the polynucleotide strand as the polynucleotide strand moves with respect to the detector. In some embodiments the system comprises a modified polynucleotide strand for analysis by said detector.

Also provided is a data encoding / data reading platform comprising:
i) a nanoreactor configured to selectively modify portions of a polynucleotide strand as the polynucleotide strand moves through the nanoreactor;
ii) optional storage for storing the polynucleotide strand once modified in the nanoreactor; and
iii) a detector configured to determine the pattern of selective modifications on the polynucleotide strand as the polynucleotide strand moves with respect to the detector.

In some embodiments the nanoreactor and/or the detector comprise a nanopore. Often, the nanoreactor and the detector comprise the same nanopore.

Also provided is the use of a nanopore and/or a nanovolume around a nanopore as a nanoreactor for selectively modifying portions of a polynucleotide strand as the polynucleotide strand moves with respect to the nanopore. Typically, selectively modifying the polynucleotide strand in such use is for encoding data on said polynucleotide strand.

Also provided is a data storage medium comprising a selectively modified polynucleotide, wherein said modified polynucleotide is obtainable using a method as described herein.

### Platforms

Also provided herein is a data encoding / data reading platform comprising:
i) a nanoreactor;
ii) means for subjecting a polynucleotide positioned in the nanoreactor to reaction conditions to modify the polynucleotide;
iii) an optional storage means for storing the polynucleotide once modified in the nanoreactor; and
iii) a detector configured to detect the presence, absence, extent or properties of modifications made to a modified polynucleotide.

In the platform, the nanoreactor is typically a nanoreactor as described herein. The platform includes means for subjecting a polynucleotide in the nanoreactor to reaction conditions to modify the polynucleotide. The reaction conditions can be any of those described herein. The means may include means for applying light, heat, voltage or chemical reagents to the nanoreactor.

The platforms preferably contain an array of individually or globally addressable nanopores or nanoreactors. Suitable examples of nanoreactors arrays include arrays of zero mode waveguides, arrays of nanopores, or arrays of aqueous droplets.

The platform may comprise storage means for storing the polynucleotide once modified in the nanoreactor. Any suitable storage means can be used. The modified polynucleotides may be stored in the nanopore in which they were also modified, and may be read-out in the same nanopore or released at a later stage as required. The modified polynucleotide can be stored by fully translocating them to a trans well volume on an chip. The modified polynucleotide can be for instance be stored in an aqueous droplet array. Preferably the modified polynucleotides are stored in droplets that can be moved, enabling them to moved to locations for storage, and/or processed as desired, and/or re-linked to new droplets with nanopores for later read-out. Any single storage volume may contain a single modified polynucleotide, or multiple differentially modified polynucleotides.

### Membrane

In methods of the invention in which a nanopore is present in a membrane, any suitable membrane may be used.

The membrane is preferably an amphiphilic layer or a solid state layer.

An amphiphilic layer is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450).

Block copolymers are polymeric materials in which two or more monomer sub-units that are polymerized together to create a single polymer chain. Block copolymers typically have properties that are contributed by each monomer sub-unit. However, a block copolymer may have unique properties that polymers formed from the individual sub-units do not possess. Block copolymers can be engineered such that one of the monomer sub-units is hydrophobic (i.e. lipophilic), whilst the other sub-unit(s) are hydrophilic whilst in aqueous media. In this case, the block copolymer may possess amphiphilic properties and may form a structure that mimics a biological membrane. The block copolymer may be a diblock (consisting of two monomer sub-units), but may also be constructed from more than two monomer sub-units to form more complex arrangements that behave as amphipiles. The copolymer may be a triblock, tetrablock or pentablock copolymer. The membrane is preferably a triblock copolymer membrane.

Archaebacterial bipolar tetraether lipids are naturally occurring lipids that are constructed such that the lipid forms a monolayer membrane. These lipids are generally found in extremophiles that survive in harsh biological environments, thermophiles, halophiles and acidophiles. Their stability is believed to derive from the fused nature of the final bilayer. It is straightforward to construct block copolymer materials that mimic these biological entities by creating a triblock polymer that has the general motif hydrophilic-hydrophobic-hydrophilic. This material may form monomeric membranes that behave similarly to lipid bilayers and encompass a range of phase behaviours from vesicles through to laminar membranes. Membranes formed from these triblock copolymers hold several advantages over biological lipid membranes. Because the triblock copolymer is synthesised, the exact construction can be carefully controlled to provide the correct chain lengths and properties required to form membranes and to interact with pores and other proteins.

Block copolymers may also be constructed from sub-units that are not classed as lipid sub-materials; for example a hydrophobic polymer may be made from siloxane or other non-hydrocarbon based monomers. The hydrophilic sub-section of block copolymer can also possess low protein binding properties, which allows the creation of a membrane that is highly resistant when exposed to raw biological samples. This head group unit may also be derived from non-classical lipid head-groups.

Triblock copolymer membranes also have increased mechanical and environmental stability compared with biological lipid membranes, for example a much higher operational temperature or pH range. The synthetic nature of the block copolymers provides a platform to customise polymer based membranes for a wide range of applications.

The membrane is most preferably one of the membranes disclosed in International Application No. WO2014/064443 or WO2014/064444.

The amphiphilic molecules may be chemically-modified or functionalised to facilitate coupling of the polynucleotide. The amphiphilic layer may be a monolayer or a bilayer. The amphiphilic layer is typically planar. The amphiphilic layer may be curved. The amphiphilic layer may be supported.

Amphiphilic membranes are typically naturally mobile, essentially acting as two dimensional fluids with lipid diffusion rates of approximately 10⁻⁸ cm s⁻¹. This means that the pore and coupled polynucleotide can typically move within an amphiphilic membrane.

The membrane may be a lipid bilayer. Lipid bilayers are models of cell membranes and serve as excellent platforms for a range of experimental studies. For example, lipid bilayers can be used for *in vitro* investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome. The lipid bilayer is preferably a planar lipid bilayer. Suitable lipid bilayers are disclosed in WO 2008/102121, WO 2009/077734 and WO 2006/100484.

Methods for forming lipid bilayers are known in the art. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface. The lipid is normally added to the surface of an aqueous electrolyte solution by first dissolving it in an organic solvent and then allowing a drop of the solvent to evaporate on the surface of the aqueous solution on either side of the aperture. Once the organic solvent has evaporated, the solution/air interfaces on either side of the aperture are physically moved up and down past the aperture until a bilayer is formed. Planar lipid bilayers may be formed across an aperture in a membrane or across an opening into a recess.

The method of Montal & Mueller is popular because it is a cost-effective and relatively straightforward method of forming good quality lipid bilayers that are suitable for protein pore insertion. Other common methods of bilayer formation include tip-dipping, painting bilayers and patch-clamping of liposome bilayers.

Tip-dipping bilayer formation entails touching the aperture surface (for example, a pipette tip) onto the surface of a test solution that is carrying a monolayer of lipid. Again, the lipid monolayer is first generated at the solution/air interface by allowing a drop of lipid dissolved in organic solvent to evaporate at the solution surface. The bilayer is then formed by the Langmuir-Schaefer process and requires mechanical automation to move the aperture relative to the solution surface.

For painted bilayers, a drop of lipid dissolved in organic solvent is applied directly to the aperture, which is submerged in an aqueous test solution. The lipid solution is spread thinly over the aperture using a paintbrush or an equivalent. Thinning of the solvent results in formation of a lipid bilayer. However, complete removal of the solvent from the bilayer is difficult and consequently the bilayer formed by this method is less stable and more prone to noise during electrochemical measurement.

Patch-clamping is commonly used in the study of biological cell membranes. The cell membrane is clamped to the end of a pipette by suction and a patch of the membrane becomes attached over the aperture. The method has been adapted for producing lipid bilayers by clamping liposomes which then burst to leave a lipid bilayer sealing over the aperture of the pipette. The method requires stable, giant and unilamellar liposomes and the fabrication of small apertures in materials having a glass surface.

Liposomes can be formed by sonication, extrusion or the Mozafari method (Colas et al. (2007) Micron 38:841-847).

In a preferred embodiment, the lipid bilayer is formed as described in International Application No. WO 2009/077734. Advantageously in this method, the lipid bilayer is formed from dried lipids. In a most preferred embodiment, the lipid bilayer is formed across an opening as described in WO2009/077734.

A lipid bilayer is formed from two opposing layers of lipids. The two layers of lipids are arranged such that their hydrophobic tail groups face towards each other to form a hydrophobic interior. The hydrophilic head groups of the lipids face outwards towards the aqueous environment on each side of the bilayer. The bilayer may be present in a number of lipid phases including, but not limited to, the liquid disordered phase (fluid lamellar), liquid ordered phase, solid ordered phase (lamellar gel phase, interdigitated gel phase) and planar bilayer crystals (lamellar sub-gel phase, lamellar crystalline phase).

Any lipid composition that forms a lipid bilayer may be used. The lipid composition is chosen such that a lipid bilayer having the required properties, such surface charge, ability to support membrane proteins, packing density or mechanical properties, is formed. The lipid composition can comprise one or more different lipids. For instance, the lipid composition can contain up to 100 lipids. The lipid composition preferably contains 1 to 10 lipids. The lipid composition may comprise naturally-occurring lipids and/or artificial lipids.

The lipids typically comprise a head group, an interfacial moiety and two hydrophobic tail groups which may be the same or different. Suitable head groups include, but are not limited to, neutral head groups, such as diacylglycerides (DG) and ceramides (CM); zwitterionic head groups, such as phosphatidylcholine (PC), phosphatidylethanolamine (PE) and sphingomyelin (SM); negatively charged head groups, such as phosphatidylglycerol (PG); phosphatidylserine (PS), phosphatidylinositol (PI), phosphatic acid (PA) and cardiolipin (CA); and positively charged headgroups, such as trimethylammonium-Propane (TAP). Suitable interfacial moieties include, but are not limited to, naturally-occurring interfacial moieties, such as glycerol-based or ceramide-based moieties. Suitable hydrophobic tail groups include, but are not limited to, saturated hydrocarbon chains, such as lauric acid (*n*-Dodecanolic acid), myristic acid (*n-*Tetradecononic acid), palmitic acid (*n*-Hexadecanoic acid), stearic acid (*n*-Octadecanoic) and arachidic (*n*-Eicosanoic); unsaturated hydrocarbon chains, such as oleic acid (*cis*-9-Octadecanoic); and branched hydrocarbon chains, such as phytanoyl. The length of the chain and the position and number of the double bonds in the unsaturated hydrocarbon chains can vary. The length of the chains and the position and number of the branches, such as methyl groups, in the branched hydrocarbon chains can vary. The hydrophobic tail groups can be linked to the interfacial moiety as an ether or an ester. The lipids may be mycolic acid.

The lipids can also be chemically-modified. The head group or the tail group of the lipids may be chemically-modified. Suitable lipids whose head groups have been chemically-modified include, but are not limited to, PEG-modified lipids, such as 1,2-Diacyl-sn-Glycero-3-Phosphoethanolamine-N -[Methoxy(Polyethylene glycol)-2000]; functionalised PEG Lipids, such as 1,2-Distearoyl-sn-Glycero-3 Phosphoethanolamine-N-[Biotinyl(Polyethylene Glycol)2000]; and lipids modified for conjugation, such as 1,2-Dioleoyl-sn-Glycero-3-Phosphoethanolamine-N-(succinyl) and 1,2-Dipalmitoyl-sn-Glycero-3-Phosphoethanolamine-N-(Biotinyl). Suitable lipids whose tail groups have been chemically-modified include, but are not limited to, polymerisable lipids, such as 1,2-bis(10,12-tricosadiynoyl)-sn-Glycero-3-Phosphocholine; fluorinated lipids, such as 1-Palmitoyl-2-(16-Fluoropalmitoyl)-sn-Glycero-3-Phosphocholine; deuterated lipids, such as 1,2-Dipalmitoyl-D62-sn-Glycero-3-Phosphocholine; and ether linked lipids, such as 1,2-Di-O-phytanyl-sn-Glycero-3-Phosphocholine. The lipids may be chemically-modified or functionalised to facilitate coupling of the polynucleotide.

The amphiphilic layer, for example the lipid composition, typically comprises one or more additives that will affect the properties of the layer. Suitable additives include, but are not limited to, fatty acids, such as palmitic acid, myristic acid and oleic acid; fatty alcohols, such as palmitic alcohol, myristic alcohol and oleic alcohol; sterols, such as cholesterol, ergosterol, lanosterol, sitosterol and stigmasterol; lysophospholipids, such as 1-Acyl-2-Hydroxy-sn- Glycero-3-Phosphocholine; and ceramides.

In another preferred embodiment, the membrane comprises a solid state layer. Solid state layers can be formed from both organic and inorganic materials including, but not limited to, microelectronic materials, insulating materials such as Si₃N₄, A1₂O₃, and SiO, organic and inorganic polymers such as polyamide, plastics such as Teflon^{®} or elastomers such as two-component addition-cure silicone rubber, and glasses. The solid state layer may be formed from graphene. Suitable graphene layers are disclosed in WO 2009/035647. If the membrane comprises a solid state layer, the pore is typically present in an amphiphilic membrane or layer contained within the solid state layer, for instance within a hole, well, gap, channel, tube, trench or slit within the solid state layer. The skilled person can prepare suitable solid state/amphiphilic hybrid systems. Suitable systems are disclosed in WO 2009/020682 and WO 2012/005857. Any of the amphiphilic membranes or layers discussed above may be used.

The method is typically carried out using (i) an artificial amphiphilic layer comprising a pore, or (ii) an isolated, naturally-occurring lipid bilayer comprising a pore. The method is typically carried out using an artificial amphiphilic layer, such as an artificial triblock copolymer layer. The layer may comprise other transmembrane and/or intramembrane proteins as well as other molecules in addition to the pore.

### Measurement Equipment and Processes

The methods disclosed herein may involve detecting the presence, absence or modifications made to a polynucleotide, or for example characterising a modified polynucleotide obtained in the methods disclosed herein.

A variety of different types of measurements may be made when detecting the characteristics of the polynucleotide. This includes without limitation: electrical measurements and optical measurements. A suitable optical method involving the measurement of fluorescence is disclosed by J. Am. Chem. Soc. 2009, 131 1652-1653. Possible electrical measurements include: current measurements, impedance measurements, tunnelling measurements (Ivanov AP et al., Nano Lett. 2011 Jan 12; 11(1):279-85), and FET measurements (International Application WO 2005/124888). Optical measurements may be combined with electrical measurements (Soni GV et al., Rev Sci Instrum. 2010 Jan; 81(1):014301). The measurement may be a transmembrane current measurement such as measurement of ionic current flowing through the pore.

Electrical measurements may be made using standard single channel recording equipment as describe in Stoddart D et al., Proc Natl Acad Sci, 12;106(19):7702-7, Lieberman KR et al, J Am Chem Soc. 2010; 132(50): 17961-72, and International Application WO 2000/28312. Alternatively, electrical measurements may be made using a multi-channel system, for example as described in International Application WO 2009/077734 and International Application WO 2011/067559.

Methods of characterising a modified polynucleotide are preferably carried out with a potential applied across the membrane. The applied potential may be a voltage potential. Alternatively, the applied potential may be a chemical potential. An example of this is using a salt gradient across a membrane, such as an amphiphilic layer. A salt gradient is disclosed in Holden et al., J Am Chem Soc. 2007 Jul 11; 129(27):8650-5.

The methods of characterising a modified polynucleotide may involve the measuring of a current passing through the pore as the polynucleotide moves with respect to the pore. Suitable conditions for measuring ionic currents through transmembrane protein pores are known in the art and disclosed in the Example. The method is typically carried out with a voltage applied across the membrane and pore. The voltage used is typically from +5 V to -5 V, such as from +4 V to -4 V, +3 V to -3 V or +2 V to -2 V. The voltage used is typically from -600 mV to +600mV or -400 mV to +400 mV. The voltage used is preferably in a range having a lower limit selected from -400 mV, -300 mV, -200 mV, -150 mV, -100 mV, -50 mV, -20mV and 0 mV and an upper limit independently selected from +10 mV, + 20 mV, +50 mV, +100 mV, +150 mV, +200 mV, +300 mV and +400 mV. The voltage used is more preferably in the range 100 mV to 240 mV and most preferably in the range of 120 mV to 220 mV. It is possible to increase discrimination between different nucleotides by a pore by using an increased applied potential.

The methods are typically carried out in the presence of any charge carriers, such as metal salts, for example alkali metal salt, halide salts, for example chloride salts, such as alkali metal chloride salt. Charge carriers may include ionic liquids or organic salts, for example tetramethyl ammonium chloride, trimethylphenyl ammonium chloride, phenyltrimethyl ammonium chloride, or 1-ethyl-3-methyl imidazolium chloride. In the exemplary apparatus discussed above, the salt is present in the aqueous solution in the chamber. Potassium chloride (KCl), sodium chloride (NaCl), caesium chloride (CsCl) or a mixture of potassium ferrocyanide and potassium ferricyanide is typically used. KCl, NaCl and a mixture of potassium ferrocyanide and potassium ferricyanide are preferred. The charge carriers may be asymmetric across the membrane. For instance, the type and/or concentration of the charge carriers may be different on each side of the membrane.

The salt concentration may be at saturation. The salt concentration may be 3 M or lower and is typically from 0.1 to 2.5 M, from 0.3 to 1.9 M, from 0.5 to 1.8 M, from 0.7 to 1.7 M, from 0.9 to 1.6 M or from 1 M to 1.4 M. The salt concentration is preferably from 150 mM to 1 M. The method is preferably carried out using a salt concentration of at least 0.3 M, such as at least 0.4 M, at least 0.5 M, at least 0.6 M, at least 0.8 M, at least 1.0 M, at least 1.5 M, at least 2.0 M, at least 2.5 M or at least 3.0 M. High salt concentrations provide a high signal to noise ratio and allow for currents indicative of the presence of a nucleotide to be identified against the background of normal current fluctuations.

The methods are typically carried out in the presence of a buffer. In the exemplary apparatus discussed above, the buffer is present in the aqueous solution in the chamber. Any buffer may be used in the method of the invention. Typically, the buffer is phosphate buffer. Other suitable buffers are HEPES and Tris-HCl buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The methods may be carried out at from 0 °C to 100 °C, from 15 °C to 95 °C, from 16 °C to 90 °C, from 17 °C to 85 °C, from 18 °C to 80 °C, 19 °C to 70 °C, or from 20 °C to 60 °C. The methods are typically carried out at room temperature. The methods are optionally carried out at a temperature that supports enzyme function, such as about 37 °C.

The methods may comprise the use of an array of nanoreactors, e.g. nanopores, present in membranes. The systems described herein may comprise an array of nanoreactors, e.g. an array of nanopores in membranes.

In a preferred embodiment, each membrane in the array comprises one nanopore. Due to the manner in which the array is formed, for example, the array may comprise one or more membrane that does not comprise a nanopore, and/or one or more membrane that comprises two or more nanopores. The array may comprise from about 2 to about 1000, such as from about 10 to about 800, from about 20 to about 600 or from about 30 to about 500 membranes. In other embodiments, each individually addressable membrane can contain multiple nanopores, eg. more than hundreds or more than thousands of nanopores. In such embodiments, each nanopore encodes a single strand with the same information. Such embodiments enables multiple copies of data to be encoded on multiple polynucleotide strands without requiring one or more separate amplification steps.

The membrane may be comprised in an apparatus having an array of electrically isolated membranes, each individually addressed using its own electrode, such that the array is equivalent to many individual sensors measuring in parallel from a test sample. The membranes may be relatively densely packed, allowing a large number of membranes to be used for a given volume of test sample. Suitable arrays of membranes and apparatuses are described in the art, for example in WO 2009/077734 and WO2012/042226. WO 2009/077734, for example, discloses a plurality of individually addressable lipid bilayers formed across an array of microwell apertures, each microwell containing an electrode and an aqueous medium in contact with the lipid bilayer.

Systems described herein are typically provided to the end user in a 'ready to use' state wherein the membranes and transmembrane pores are pre-inserted. Systems are also referred to herein as apparatus. A typical apparatus provided in a 'ready to use' state comprises an array of amphiphilic membranes, each membrane comprising a transmembrane pore and being provided across a well containing a liquid. Such an apparatus and method of making it are disclosed by WO2014/064443. Test liquid to be analyzed is applied to the upper surface of the amphiphilic membranes.

Providing an apparatus in a 'ready to use' state however has additional considerations in that care needs to be taken that the sensor does not dry out, namely that liquid is not lost from the well by passage through the amphiphilic membrane, which may result in a loss of performance or damage the sensor. One solution to address the problem of drying out of the sensor is to provide the device with a buffer liquid over the surface of the amphiphilic membrane such that any evaporation through the surface of the membrane is minimized and the liquids provided on either side of the membrane may have the same ionic strength so as to reduce any osmotic effects. In use the buffer liquid may be removed from the surface of the amphiphilic membrane and a test liquid to be analyzed is introduced to contact the surface.

Some applications may use measurement of electrical properties across the membranes, for example ion current flow. To provide for such measurements, the apparatus may further comprise respective electrodes in each compartment making electrical contact with the volumes comprising polar medium. Other types of measurements may be carried out for example optical measurements such as fluorescence measurements and FET measurements. Optical measurements and electrical measurements may be carried out simultaneously (Heron AJ et al., J Am Chem Soc. 2009;131(5):1652-3).

The apparatus may further comprise a common electrode. The apparatus may further comprise an electrical circuit connected between the common electrode and the respective electrodes in each compartment, the electrical circuit being arranged to take electrical measurements. Such electrical measurements may be dependent on a process occurring at or through the membranes.

The apparatus may comprise a FET array for making measurements of the nanopore array.

In the methods provided herein, the detector may be selected from (i) a zero-mode waveguide, (ii) a field-effect transistor, optionally a nanowire field-effect transistor; (iii) an AFM tip; (iv) a nanotube, optionally a carbon nanotube; and (v) a nanopore. Preferably, the detector is a nanopore.

In one embodiment, the apparatus comprises:
a sensor device that is capable of supporting the plurality of pores and membranes and being operable to perform analyte characterisation using the pores and membranes; and
at least one port for delivery of the material for performing the characterisation.
In one embodiment, the apparatus comprises:
   a sensor device that is capable of supporting the plurality of pores and membranes being operable to perform analyte characterisation using the pores and membranes; and
   at least one reservoir for holding material for performing the characterisation.

In one embodiment, the apparatus comprises:
a sensor device that is capable of supporting the membrane and plurality of pores and membranes and being operable to perform analyte characterising using the pores and membranes;
at least one reservoir for holding material for performing the characterising;
a fluidics system configured to controllably supply material from the at least one reservoir to the sensor device; and
one or more containers for receiving respective samples, the fluidics system being configured to supply the samples selectively from one or more containers to the sensor device.

The apparatus may also comprise an electrical circuit capable of applying a potential and measuring an electrical signal across the membrane and pore complex.

The apparatus may be any of those described in WO 2008/102120, WO 2009/077734, WO 2010/122293, WO 2011/067559 or WO 00/28312.

Also provided herein is a method of selectively modifying a polynucleotide strand within a nanoreactor. In some embodiments the polynucleotide strand is modified as it moves with respect to the nanoreactor. In some embodiments the pattern of selective modifications on the polynucleotide strand encodes data on the strand. In some embodiments the polynucleotide strand and nanoreactor are as described herein.

It is to be understood that although particular embodiments, specific configurations as well as materials and/or molecules, have been discussed herein for methods according to the present invention, various changes or modifications in form and detail may be made without departing from the scope and spirit of this invention. The preceding embodiments and following examples are provided for illustration only, and should not be considered limiting the application.

### EXAMPLES

### Example 1

This example demonstrates modification of a semi-rotaxane polynucleotide strand ("static strand") by photocleavage of a photocleavable linker moiety while held under potential in a nanopore. This example demonstrates that a polynucleotide strand within a nanopore nanoreactor can be modified in real time.

Three-prime-biotinylated polynucleotide strands containing a photocleavable spacer moiety ("Int PC Spacer"; Integrated DNA Technologies (IDT); available at https://eu.idtdna.com/site/Catalog/Modifications/Product/1707) were synthesised by Integrated DNA Technologies (IDT). Synthesized strands are shown below:

| Name | Sequence (5'->3') |
|---|---|
| DW052_PC_map_static | TTTTTTTTTTTTTTTTTTTTTTTTTTTTTT/3Bio/ |
| DW053_PC_map_static | TTTTTTTTTTTTTTTTTTTTTTTTTTTTTT/iSpPC//3Bio/ |

**"Static strands"** were prepared by incubating 500 nM of each polynucleotide strand with 500 nM monomeric traptavidin (a 3:1 mix of streptavidin-N23A/S27D/S45A : traptavidin-S52G/R53D) in buffer containing HEPES-KOH (pH 8.0), KCl at room temperature.

Experiments were performed using FLO-MIN106 flow cells (Oxford Nanopore Technologies). Each well contained a solution of potassium phosphate (pH 8.0), potassium ferricyanide, potassium ferrocyanide **("mediator buffer"),** and the cis chamber contained mediator buffer + 10 nM thrombin binding aptamer. Electrical data were collected using Oxford Nanopore Technologies' MinION using a preprogrammed script, which applies potential across the membrane in the following sequence: [0 mV, 5 sec; -100 mV, 2 sec; 0 mV, 2 sec; +180 mV, 60 sec; 0 mV, 2 sec]. The voltage sequence in the square bracket was repeated up to 50 times.

As shown schematically in Figure 8, the static strand was initially prevented from translocating fully through the nanopore owing to the biotin-traptavidin linkage. Positive electrical potential was used to capture the strand in the nanopore, whilst negative potential was used to eject the captured static strand from the nanopore. The capture of a polynucleotide resulted in a characteristic current blockade, which could be used to identify the polynucleotide dependent on the nucleotide bases sitting in the constriction of the nanopore. Photocleavage of the polynucleotide using 365 nm light in situ while held in the nanopore at positive potential was expected to cleave the polynucleotide into two fragments, causing the non-biotinylated part of the polynucleotide to translocate through the pore, whereas the biotinylated fragment of DNA remaining in the cis chamber would be expected either to remain captured (*i.e.,* no change in current blockade) or spontaneously dissociate (*i.e.,* return to open-pore current) from the nanopore depending on the length of the fragment, which in turn depends on the position of the photocleavable bond.

The above voltage script was run according to the following schedule, introducing reagents into the flow cell as follows, where one "cycle" is a repeat of the voltage sequence:
- Cycle 1: mediator buffer + 10 nM thrombin binding aptamer, used to positively identify the presence of a nanopore in the selected channel.
- Cycle 2: flush cis chamber with 2 mL running buffer via inlet port and prime SpotON port according to manufacturer's instructions.
- Cycle 5: add 75 µL preparation of "static strand". A different static strand from the table above was added to each flow cell.
- Cycle 8: pulse-irradiate MinION flow cell throughout cycle with 365 nm UV light (1 sec pulse of ~0.17 J/cm², 4 sec off, 11 times).

Each "static strand" was run on a separate MinION flow cell, but timings of the additions and UV pulses were identical between flow cells.

Electrical current vs. time data was analysed from the raw sequencing files. Representative traces from the cycles described above are plotted in Figures 9 and 10, with the timings of UV pulses superimposed. The data show a blockade of the capture strand that is distinct from open pore. UV irradiation of the flow cell causes a drift upwards of the measured current owing to photooxidation/photoaquation of electrochemical mediator, which acts as an internal control for whether a particular channel on the flow cell received UV light.

In the absence of UV irradiation the captured strands for any sample remained permanently held in the nanopore unless ejected by voltage reversal. Figure 9 shows that samples containing no photocleavable moiety do not release upon UV irradiation, indicating that no reaction has occurred. By contrast, Figure 10 shows that, for samples containing a photocleavable moiety, upon brief irradiation of the sample there is a high probability of immediate return to open-pore current, which indicates strand loss as a result of a photocleavage reaction. This return to open-pore is then shortly followed by capture of another un-cleaved strand from solution. This therefore indicates detection of a photocleavable moiety inside a nanopore.

Example 1 thus shows that photo-modification of polynucleotides within a nanopore can be achieved in real time, indicating that photomodification of polynucleotide strands can be achieved to encode data on the polynucleotide strand.

### Example 2

This example demonstrates how ssDNA oligonucleotides can be assembled via splint ligation to generate DNA concatemer strands, which can be used in a nanopore system for further applications in DNA data storage.

There are many well-known methods for creating long DNA substrates from smaller defined segments of DNA, for example from small sections of synthetic DNA of less than 100nt. For example, it is possible to concatenate small sections of DNA or RNA together, by ligation for example, to create a longer molecule with many repeats of the smaller units. A combination of smaller units can be connected in defined manners by controlling the sticky ends for ligation. For example, a strand (A)*ₙ* can be created by concatenating together multiple (A) units. Alternatively, a strand (A-B)*ₙ* can be created by concatenating together multiple (A) and (B) units with appropriate sticky end sequence complementarity to coordinate the which units engage together.

Figure 11 shows an example of a long DNA strand with defined repeats that was created by concatenating together smaller segments of DNA by ligation and splinting. Concatemer assembly was generated by incubating 0.25 µL of **DW089** and **DW111** (at 10 µM), 2.5 µL **DW141** (at 100 µM), 5 µL **DW088** (100 µM) and 2 µL nuclease-free duplex buffer (Integrated DNA Technologies, Inc., Cat # 11-01-03-01) at 95°C for 5 min, followed by a slow cooling to room temperature (-0.1°C/sec).

| Name | Sequence (5' → 3') |
|---|---|
| DW088 | gtcgtctcg/iBNA-A//iBNA-A//iBNA-T//iBNA-A//3BNA-A/ |
| DW089 (unit A) | |
| DW111 (unit C) | /5Phos/agacgacTTTTTTTTTT/3BiotinTEG/ |
| DW141 (unit B) | /5Phos/AGACGACAGACGACCCCCCCCCCCTCTCTCTCTCTTTTTTTTT TTTCCCTTTT/iSp18//iSp18//iSp18//iSp18/TTATTCG |

The concatemer assembly was ligated together by incubating 5 µL of concatemer assembly (125 nM final concentration) with 2.5 µL LNB (from Oxford Nanopore LSK-109), 1 µL T4 Ligase (from NEB Quick Ligation Kit, # M2200) in a final volume of 10 µL. The mixture was incubated for 30 minutes at room temperature. The sequencing adapter (("RAP" from SQK-RAD004)) was clicked to the seed strand via adding 1 µL of RAP to each tube and the mixture was incubated for 15 mins at room temperature.

Monomeric Avidin Agarose (ThermoFisher Scientific Cat # 20267) was prepared according to the manufacturer's instructions. The concatemer strands were purified using 50µL of 50% slurry monomeric agarose with the kilobaseBINDER binding buffers (ThermoFisher Scientific Cat # 60101) following the manufacturer's instructions. The sample was eluted with 1x Biotin Elution Buffer (IBA Lifesciences cat #2-1019-025) for 15 minutes at room temperature.

25µL SQB (from Oxford Nanopore LSK-109) were added to the sample. Loading on the flowcell was done according to Oxford Nanopore instructions and sequencing was carried out for 6h.

Ionic current vs time data was acquired while applying custom voltage changes to the platform, switching the voltage between a high voltage V1 (+180mV) and a lower holding voltage V2 (+60mV). The electrical data of ionic current vs time was acquired for all nanopore channels on the chip and analysed. Representative data from the movement of the concatenated DNA sample through the nanopores is shown in Figure 13. The data shows capture and controlled movement of the substrate through the nanopore, clearly showing multiple B units in the substrate passing through the pore. When the voltage is dropped to the lower holding voltage V2 the movement through the nanopore stops as the motor protein is unable to overcome the stall site. Movement only resumes after the voltage is increased to V1. This data illustrates how voltage can be used to control the movement of a DNA substrate through a nanopore, pausing and resuming movement as required by adjusting the magnitude of the applied voltage. Such controlled movement allows the directed modification of polynucleotide strands within a nanopore nanoreactor as the modification can be carried out on specific portions of the polynucleotide within the nanoreactor as required to encode data on the strand.

### Example 3

This example demonstrates the oxidation and detection of guanine DNA bases in a semi-rotaxane polynucleotide strand ("static strand") by irradiation with ultraviolet light.

Three-prime-biotinylated polynucleotide strands containing G bases in a poly(dT) context were synthesised as follows.

| Name | Sequence (5'->3') |
|---|---|
| DW080 | TTTTTTTTTTTTTTTTTTTTTTTTTGGGTTTTT/3Bio/ |
| DW113 | TTTTTTTTTTTTTTTTTTTTTTT/8oxoG//8oxoG//8oxoG/TTTTT/3Bio/ TT |

**"Static strands"** were prepared by incubating 500 nM of the polynucleotide strand with 500 nM monomeric traptavidin in buffer containing HEPES-KOH (pH 8.0), KCl at room temperature. Optionally, 50 µM riboflavin (as UV-sensitiser) was included, and the "static strands" (75 µL, in a 1.5 mL Eppendorf tube) were optionally irradiated by treatment with 365 nm UV light (~6.4 J/cm², as measured using a UV meter).

Experiments were performed using FLO-MIN106 flow cells (Oxford Nanopore Technologies). Electrical data were collected using Oxford Nanopore Technologies' MinION using a preprogrammed script, which applies potential across the membrane in the following sequence: [0 mV, 5 sec; -100 mV, 2 sec; 0 mV, 2 sec; +180 mV, 60 sec; 0 mV, 2 sec]. The voltage sequence in the square bracket was repeated up to 50 times. The static strand was prevented from translocating fully through the nanopore owing to the biotin-traptavidin linkage. Positive electrical potential was used to capture and test the strand in the nanopore, whilst negative potential was used to eject the captured static strand from the nanopore. The capture of a polynucleotide resulted in a characteristic current blockade, which could be used to identify the polynucleotide dependent on the nucleotide bases sitting in the constriction of the nanopore.

An experiment was performed by running the above voltage script, introducing reagents into the flow cell as follows:
- Static strand (DW080) containing three bases in the nanopore constriction;
- Control static strands (DW113, DW114 or DW115) containing three, two or one 8-oxo-2'-deoxyguanosine (8-oxo-dG) bases respectively in the nanopore constriction;
- The above static strand (DW080) irradiated with UV (6.4 J/cm²) in the presence of 50 µM riboflavin sensitiser;
- Control static strand (DW113) containing three 8-oxo-2'-deoxyguanosine (8-oxo-dG) bases respectively in the nanopore constriction;

Electrical current vs. time data was analysed from the raw sequencing files. Representative traces from the cycles described above are plotted in Figure 14, showing similar blockade levels between a strand containing three dG bases irradiated with UV + sensitiser and a strand containing 8-oxo-dG bases at equivalent positions.

### Example 4

This example describes the real time photocleavage of side chains of a polynucleotide strand in order to encode data on the strand.

A polynucleotide substrate comprising a photocleavable side-chain is synthesized. The photocleavable sidechain chemistry is shown in Figure 15 and can be synthesized as using known chemical reactions (Angew Chem Int Ed Engl. 2015 November 9; 54(46): 13635-13638). The polynucleotide strand comprises repeating photocleavable groups as shown on the strand.

The strand is held in a CsgG nanopore by being paused at low voltage as described above. The strand is contacted with a motor protein to control the movement of the strand through the nanopore. The strand is held with photocleavable side-chain R positioned within 3 nm, typically within 1 nm of a nearby photosensitizer (labelled S) attached to the CsgG nanopore by maleimide chemistry.

The entire nanopore system is irradiated, e.g. by UV light. Upon irradiation of the entire nanopore system, the sensitizer absorbs the incoming radiation and re-emits radiation locally at a different wavelength. The photocleavable side-chain does not absorb the incoming global wavelengths and is only sensitive the local re-emitted radiation from the sensitizer, which results in photocleavage as indicated and loss of the side-chain molecule. Other photocleavable R groups on the rest of the substrate outside of this unit in the nanopore (not shown) are much further away from the sensitizer and not exposed to sufficient radiation to enable photocleavage. The selective removal of the side-chain molecule therefore modifies the polynucleotide at selective sites, to for example encodes a pattern of modifications to store data.

The modified DNA produces a different signal to the unmodified starting material when characterised e.g. by being sequenced on a nanopore system. In this way the modifications are detected and identified with data encoded on the strand.

### Example 5

This example demonstrates 1) the controlled movement of a DNA strand through a nanopore by means of a motor protein; 2) the control of which portion of the DNA is contained in the nanopore by means of voltage, and 3) selective modification of the chosen portion of DNA in the nanopore. The example demonstrates all elements combined to modify selected portions of the DNA in a defined pattern to encode data, which can be read back in situ on the same nanopore. Here, light is used to excise a branched bulky group extending from the DNA backbone, thereby yielding a detectable change while the group is held in a protein nanopore.

### Strand preparation

A phosphoramidite is prepared based on heptamethine cyanine dye **R** (Angew Chem Int Ed Engl. 2015 November 9; 54(46): 13635-13638). DNA oligonucleotide **DW141R** is prepared containing four internal Spacer 18 **(****Figure 15****),** with R incorporated between 4-10 bases to the 5' side of the proximal Spacer 18. Scission of **R** is expected to occur upon illumination with 690 nm light, yielding a hydroxyl group at the same position **(****Figure 15C****).**

| Name | Sequence |
|---|---|
| DW089 | /5DBCOTEG/GCTTGGGTGTTTAACCTTTTTTTTTTTTTTTTTTTT ttattcg |
| DW111 | /5Phos/agacgacTTTTTTTTTT/3BiotinTEG/ |
| DW141R | /5Phos/AGACGACAGACGACCCCCCCCCCCTCTCTCTCTCTTTTT TTTTRRRRRRRTTT/iSp18//iSp18//iSp18//iSp18/TTATTCG |
| | R = position of scissionable heptamethine cyanine dye, or T base |
| DW141R2 | /5Phos/AGACGACAGACGACCCCCCCCCCCTCTCTCTCTCTTTTT TTTTTTTRTTTTTT/iSp18//iSp18//iSp18//iSp18/TTATTCG |
| | R = position of scissionable heptamethine cyanine dye, or T base |
| DW088 | gtcgtctcg/iBNA-A//iBNA-A//iBNA-T//iBNA-A//3BNA-A/ |

A "concatemeric assembly" polynucleotide strand containing multiple repeats of **DW141R** is generated as follows: 0.25 µL of **DW089** ("A unit") and **DW111** ("C unit"), both at 10 µM, 2.5 µL **DW141R** ("B unit", at 100 µM), 5 µL **DW088** ("splint", at 100 µM) and 2 µL nuclease-free duplex buffer (Integrated DNA Technologies, Inc., Cat # 11-01-03-01) at 95 °C for 5 min, followed by a slow cooling to room temperature (-0.1 °C/sec).

The above is ligated by incubating 5 µL of concatemeric assembly (125 nM final concentration) with 2.5 µL LNB (from Oxford Nanopore LSK-109), 1 µL T4 Ligase (from NEB Cat # M2200) in a final volume of 10 µL. The mixture is incubated for 30 minutes at room temperature. The sequencing adapter ("RAP" from SQK-RAD004) is clicked to the seed strand by adding 1 µL of RAP to each tube and the mixture incubated for 15 mins at room temperature.

Monomeric avidin agarose (ThermoFisher Scientific Cat # 20267) is prepared according to the manufacturer's instructions. The concatemer strands are purified using 50 µL of 50% slurry monomeric agarose with the kilobaseBINDER binding buffers (ThermoFisher Scientific, Cat # 60101) following the manufacturer's instructions. The sample is eluted with 25 µL of 1x Biotin Elution Buffer (IBA Lifesciences cat #2-1019-025) for 15 minutes at room temperature.

### Nanopore preparation

CsgG protein nanopores bearing cysteine mutations at one or more residues are purified and labelled with biotin maleimide (Merck Cat # B 1267), incubated with QDot 705 streptavidin conjugate (ThermoFisher Scientific, Cat # Q10161MP) and inserted into Oxford Nanopore Technologies' FLO-MIN106 flow cells.

An Oxford Nanopore MinION FLO-MIN106 flow cell is flushed and the SpotON port primed with FLB and FLT from SQK-LSK109, according to the manufacturer's instructions. 25 µL SQB is added to the sample and the resulting 50 µL loaded via the SpotON port. The light guide of a CoolLED pE-4000 LED light source is positioned ~0.5 cm above the SpotON port. Electrical data are collected using Oxford Nanopore Technologies' MinION using a preprogrammed script, which applies potential across the membrane in the following sequence: [V1 = +180 mV, 10 sec; V2 = +60 mV, 10 sec]. The emission of the LED source (16 selectable wavelengths) is activated and deactivated rapidly by computer control. In this case, 405 nm or 435 nm light excites the photosensitiser (quantum dot) but not the target molecule. The voltage sequence in the square bracket is repeated until the experiment is stopped.

### Set-up

Figure 16 shows the system of protein nanopore labelled with photosensitiser (S), as the motor protein encounters the stall site. Until this point, a potential of +180 mV (V1) is applied across the membrane, but as the motor encounters the stall, a change in the recorded current is detected, and the voltage is reduced to holding potential V2 (+60 mV). Irradiation of the photosensitiser (S) at this point yields photocleavage of bulky group R, yielding a detectable change in the current if successful.

Figure 17 shows how movement between subsequent "B units", each containing a single modifiable group ("R"), may be controlled by a combination of the motor protein and the holding potential. The applied voltage across the membrane of each channel in the nanopore array (Ch.1, Ch.2, ..., Ch.n) is individually addressable. The pattern of voltage is used to advance the motor protein any desired number of units along the concatemeric polynucleotide until it encounters the desired modification position, whereupon the entire array is synchronously illuminated. The motor protein prevents further translocation until it is pushed over the first stall by the force of the applied voltage. After a brief pause as the stall the motor is pushed over the stall and the "RAP+A" signal is observed passing through the nanopore as the motor protein feeds the DNA into the nanopore under ATP fuel-controlled movement. While the voltage is held at V1 the motor protein continues to proceed through subsequent stall sites in the "Bₙ" units after brief pauses at each stall. The figure shows how, for example, the motor protein in Ch.1 may be advanced four positions before making the modification, then one (encoding 00011...); whereas that of Ch.2 may be advanced one position, then three (encoding 1001...).

### Data writing

Three representative channels in an array of channels in a MinION chip (Oxford Nanopore Technologies) are selected. Each channel is independently addressed with electrodes and circuitry that enables individual control of the applied voltage to that channel. Unblock circuitry is used to independently switch each channel between a holding voltage (V2) and the global voltage (V1) as required. Software, such as Oxford Nanopore MinKNOW software with active monitoring and feedback control, is used to independently monitor the current levels in real-time. The software monitors the current levels to sequence and detect the forward movement of the DNA substrates in each channel, and switches the voltage as required such as to encode a pre-determined pattern of data.

To demonstrate the writing of data to the polynucleotide strand, channel 1 is set to encode [000110...] into the polynucleotide strand. Channel 2 is set to encode [10010...] and Channel n is set to encode [01010...].

Each channel is contacted with a DNA substrate as shown in Figure 15 moving through the nanopore under the control of a motor protein. The movement of the substrate through the nanopore produces characteristic current signals as the repetitive units pass through the nanopore. The software monitoring each channel measures the characteristic current in real-time under applied global voltage V1 (eg. +180mV) and determines whether to write a modification to the next unit. After the unit to be written to begins and before the next unit begins the software drops the voltage to the holding voltage V2 to stall the motor protein at the next stall site, which holds the unit to be modified adjacent to the sensitizer for performing the modification reaction (Figure 17).

For channel 1 the 4^{th} unit needs to be modified, so after it begins the software drops the voltage as indicated at Figure 17-A1. For channel 2 and channel n the 1^{st} and 2^{nd} units respectively need modification, so the voltage is dropped at points A2 and An as indicated. In this manner each channel is independently controlled to move through the required number of unmodified units (coding 0) and pause on the next unit to modify (coding for 1). The modification is cause by a pulse of irradiation at a desired frequency, the radiation exciting a sensitizer on the nanopore that re-emits radiation locally to cleave an adjacent photocleavable side-chain on the DNA unit in the nanopore.

The timings of the reaction condition are illustrated in Figure 17. The duration of the wait period between pulses (E1, E2, ...En) is set to enable all channels to pass the requisite number of unmodified strands and stack the next one to be modified. The period can be fixed, or variable and controlled by the global software so allow every single channel to reach the desired stage, including those with long sections of unmodified units. The duration of the reaction (F1, F2, ..Fn) is set to enable a high percentage modification success. This might be fixed and determined in advance based on separate experiments in the case that the modification cannot be observed in situ. Alternatively, if the modification is positioned in the nanopore such that a change in current is observed upon modification then the software cannot monitor, record and control the system as required to ensure successful writing.

After the modification phase the voltage in each channel is returned to the global voltage V1 (eg. +180mV, point B for cycle 1 and point D for cycle 2 in figure 17) so that the motor proteins are pushed over the next stall site to continue writing to subsequent units in the DNA. The entire process of passing the desired number of unmodified units, detecting the unit to modify, dropping the voltage (points A and C on figure 17) to stack up the units to be modified and waiting, then irradiating globally, proceeds in loops of [E1/F1, E2/F2, ... En/Fn] as the strands proceed through each nanopore writing the desired pattern of modifications into each strand.

At the end of the writing phase the modified DNA is read back. The modified DNA can be transferred to another reading system, for example another nanopore in another nanopore sequencer. Alternatively, the modified DNA can be read back in situ on the same nanopore in which it was modified by retaining it. For example, the strands can be capped with a blocker (eg. Streptavidin) that prevents total translocation through the nanopore. Then for example the strand can be reversed back through the nanopore by reverse voltage and reloaded with a new motor protein, and then passed back through the pore again to read out the sequence. Alternatively, a motor protein that pulls the DNA out of the nanopore can be loaded using methods known in the art and used to pull the DNA out of the pore through nanopore to read back the signal.

### Example 6

This example demonstrates the encoding of data by modification of a naturally occurring DNA strand by photoirradiation.

A 3.5 kb section of lambda phage DNA (commercially available) is provided. Figure 18 shows representative ionic current vs time data obtained by sequencing the lambda phage DNA using a MinION flow cell (Oxford Nanopore Technologies) operated according to the manufacturer's instructions. Guanine bases are identified using standard base-calling algorithms and are highlighted in Figure 18 (lower panel).

The section of lambda phage DNA is passed under motor protein control through a CsgG nanopore modified to include a photosensitizer such as a quantum dot or gold-based nanoparticle. The system is illuminated with UV light. The sensitizer under illumination creates a highly localised change in conditions that mediates the reaction of the nearby guanine bases, which are more sensitive than other bases to chemical oxidation. The reaction solution contains one or more sensitizer molecules selected from hydrogen peroxide, acridine orange, methylene blue that mediate reduction-oxidation chemistry to more efficiently modify the guanines. The lambda phage DNA is moved through the pore at approximately constant rate under irregular irradiation, thus creating a unique pattern of modifications in the strand (shown schematically in Figure 19). The pattern of modifications (Figure 20) encodes data on the strand.

The following are aspects of the invention:
1. A method of encoding data on a polynucleotide strand, comprising:
   (A) moving the polynucleotide strand with respect to a nanoreactor; and
   (B) selectively modifying portions of the polynucleotide strand as they move through the nanoreactor;
   wherein the pattern of selective modifications on the polynucleotide strand encodes data on the strand.
2. A method according to aspect 1, wherein the portions of the polynucleotide strand which are selectively modified are sequential portions of the polynucleotide strand.
3. A method according to aspect 1 or aspect 2 wherein selectively modifying portions of the polynucleotide strand does not alter the overall length of the polynucleotide strand.
4. A method according to any one of aspects 1 to 3 wherein selectively modifying portions of the polynucleotide strand does not comprise adding or removing monomer units from the polynucleotide strand.
5. A method according to any one of the preceding aspects wherein the portions of the polynucleotide strand which are selectively modified correspond to data units thereby encoded.
6. A method according to any one of the preceding aspects wherein the portions of the polynucleotide strand which are selectively modified in the nanoreactor comprise:
   - from 1 to about 1000 nucleotides; or
   - from 1 to about 100 nucleotides; or
   - from 1 to about 10 nucleotides.
7. A method according to any one of the preceding aspects comprising selectively modifying from about 10¹ to about 10⁹ portions of the polynucleotide strand as they move through the nanoreactor.
6. A method according to any one of the preceding aspects, comprising continuously moving the polynucleotide strand with respect to the nanoreactor whilst selectively modifying the portions of the polynucleotide strand as they move through the nanoreactor.
7. A method according to aspect 6, comprising moving the polynucleotide strand at a constant rate with respect to the nanoreactor, and applying reaction conditions at a regular or irregular frequency to the polynucleotide strand as it moves through the nanoreactor.
8. A method according to aspect 6, comprising moving the polynucleotide strand at a variable rate with respect to the nanoreactor, and applying reaction conditions at a regular or irregular frequency to the polynucleotide strand as it moves through the nanoreactor.
9. A method according to any one of aspects 1 to 5, comprising interrupting the movement of the polynucleotide strand with respect to the nanoreactor whilst selectively modifying the portions of the polynucleotide strand as they move through the nanoreactor.
10. A method according to any one of the preceding aspects wherein selectively modifying portions of the polynucleotide strand comprises selectively controlling the number of nucleotides that are modified within each portion of the polynucleotide strand.
11. A method according to any one of the preceding aspects wherein selectively modifying portions of the polynucleotide strand comprises selectively controlling the extent of the modifications made to the nucleotides that are modified within each portion of the polynucleotide strand.
12. A method according to any one of the preceding aspects wherein the nanoreactor comprises a nanopore.
13. A method according to aspect 12 wherein the nanoreactor comprises the internal volume of the nanopore or a portion of the internal volume of the nanopore.
14. A method according to any one of the preceding aspects wherein the nanoreactor comprises a nanovolume around a nanopore.
15. A method according to aspect 14 wherein the nanovolume comprises a volume extending to about 30 nm from one or more openings of the nanopore.
16. A method according to any one of aspects 12 to 15 wherein the nanopore is a transmembrane protein nanopore, a solid state nanopore, a DNA-origami pore, or a polymer-based plastic pore;
   optionally wherein the nanopore is a transmembrane β-barrel protein pore.
17. A method according to any one of aspects 12 to 16 wherein the nanopore comprises a well, gap, channel, tube, trench or slit in a membrane or across a membrane.
18. A method according to any one of aspects 12 to 17, comprising passing the polynucleotide strand through the nanopore.
19. A method according to any one of the preceding aspects wherein selectively modifying portions of the polynucleotide strand comprises making epigenetic modifications to the polynucleotide strand.
20. A method according to any one of the preceding aspects wherein selectively modifying portions of the polynucleotide strand comprises subjecting the portions of the polynucleotide strand in the nanoreactor to reaction conditions comprising (i) the presence, absence or concentration of one or more chemical reagent(s); (ii) the engagement of an enzyme with the polynucleotide strand under conditions that the enzyme modifies the nucleotides within the polynucleotide strand; (iii) the presence or absence of electromagnetic radiation; and/or (iv) the presence or absence of applied heat.
21. A method according to aspect 20, comprising controlling the presence, absence or concentration of one or more chemical reagent(s) in the nanoreactor by applying an electrical or chemical potential across the nanoreactor.
22. A method according to aspect 20 or aspect 21 wherein said chemical reagents comprise at least a first reagent and a second reagent and the first and second reagents react with the portion of the polynucleotide strand in the nanoreactor.
23. A method according to any one of aspects 20 to 22 wherein the chemical reagents selectively or non-selectively modify the backbone, the sugar, and/or the bases of polynucleotides in the nanoreactor.
24. A method according to any one of aspects 20 to 23 wherein one or more protectant(s) are provided external to the nanoreactor to minimise or prevent modification of the polynucleotide strand by chemical reagent(s) external to the nanoreactor.
25. A method according to aspect 20, comprising contacting the portions of the polynucleotide in the nanoreactor with a polynucleotide-processing enzyme;
   optionally wherein the polynucleotide-processing enzyme modifies the base portion of nucleotides within the polynucleotide strand.
26. A method according to aspect 25, comprising controlling the modification of the polynucleotide strand by the polynucleotide-processing enzyme by (i) controlling the presence, absence or concentration of fuel and/or substrate for the enzyme; and/or (ii) controlling a force exerted on the polynucleotide strand.
27. A method according to aspect 20, comprising contacting the portions of the polynucleotide strand in the nanoreactor with electromagnetic radiation in the form of light, preferably visible or ultraviolet light.
28. A method according to aspect 20 or aspect 27, comprising irradiating a photosensitizer in the nanoreactor for modification of the polynucleotide strand.
29. A method according to aspect 20, 27 or 28, comprising irradiating a sensitizer in the nanoreactor and transferring radiation from the sensitizer to the polynucleotide strand in the nanoreactor for modification of the polynucleotide strand.
30. A method according to aspect 29 wherein the sensitizer is a metal nanoparticle, and/or wherein said radiation is electromagnetic radiation or thermal radiation.
31. A method according to any one of the preceding aspects, further comprising the step of:
   (C) determining the pattern of selective modifications on the polynucleotide strand;
   optionally wherein determining the pattern of selective modifications on the polynucleotide strand comprises determining the presence, absence, extent or properties of modifications made to the polynucleotide strand.
32. A method according to aspect 31, wherein determining the pattern of selective modifications on the polynucleotide strand corresponds to reading data encoded on the polynucleotide strand.
33. A method according to any one of the preceding aspects, comprising characterizing the modified polynucleotide strand.
34. A method according to any one of aspects 31 to 33 wherein determining the pattern of selective modifications on the polynucleotide strand and/or characterizing the modified polynucleotide strand comprises:
   i) contacting a detector with the modified polynucleotide strand such that the polynucleotide strand moves with respect to the detector; and
   ii) taking one or more measurements which are indicative of one or more properties of the modified polynucleotide strand as the polynucleotide strand moves with respect to the detector.
35. A method according to aspect 34 wherein the detector is a transmembrane pore;
   wherein said method optionally comprises:
   a) contacting the pore with the modified polynucleotide strand;
   b) applying a potential difference across the pore; and
   c) taking one or more measurements which are indicative of one or more properties of the modified polynucleotide strand moving with respect to the pore and thereby determining the pattern of selective modifications on the polynucleotide strand and/or characterizing the modified polynucleotide strand.
36. A method according to aspect 35 wherein said method comprises measuring the current passing through the transmembrane pore as the modified polynucleotide strand moves with respect to the transmembrane pore.
37. A method according to aspect 35 or aspect 36, wherein
   a) the nanoreactor comprises a nanopore; and
   b) determining the pattern of selective modifications on the polynucleotide strand and/or characterizing the modified polynucleotide comprises (i) contacting the same nanopore with the modified polynucleotide strand such that the polynucleotide strand moves with respect to the nanopore; and (ii) taking one or more measurements which are indicative of one or more properties of the modified polynucleotide strand as the polynucleotide strand moves with respect to the nanopore.
38. A method of modifying a polynucleotide strand, comprising:
   (A) moving the polynucleotide strand with respect to a nanoreactor; and
   (B) selectively modifying portions of the polynucleotide strand as they move through the nanoreactor;
   wherein optionally the method, polynucleotide strand, nanoreactor and/or selective modifications made are as defined in any one of the preceding aspects.
39. A system for encoding data on a polynucleotide strand, the system comprising a nanoreactor configured to selectively modify portions of a polynucleotide strand as the polynucleotide strand moves through the nanoreactor;
   optionally wherein the system comprises a nanopore for controlling the movement of the polynucleotide strand through the nanoreactor and/or a polynucleotide for modification in the nanoreactor.
40. A system for reading data encoded on a polynucleotide strand; the system comprising a detector configured to determine the pattern of selective modifications on the polynucleotide strand as the polynucleotide strand moves with respect to the detector;
   optionally wherein the system comprises a modified polynucleotide strand for analysis by said detector.
41. A data encoding / data reading platform comprising:
   i) a nanoreactor configured to selectively modify portions of a polynucleotide strand as the polynucleotide strand moves through the nanoreactor;
   ii) optional storage for storing the polynucleotide strand once modified in the nanoreactor; and
   iii) a detector configured to determine the pattern of selective modifications on the polynucleotide strand as the polynucleotide strand moves with respect to the detector;
   wherein preferably the nanoreactor and/or the detector comprise a nanopore, wherein more preferably the nanoreactor and the detector comprise the same nanopore.
42. Use of a nanopore and/or a nanovolume around a nanopore as a nanoreactor for selectively modifying portions of a polynucleotide strand as the polynucleotide strand moves with respect to the nanopore.
43. Use according to aspect 42 wherein selectively modifying the polynucleotide strand is for encoding data on said polynucleotide strand.
44. A data storage medium comprising a selectively modified polynucleotide, wherein said modified polynucleotide is obtainable using a method according to any one of aspects 1 to 30.

## Claims

1. A method of encoding data on a polynucleotide strand, comprising:
(A) moving the polynucleotide strand with respect to a nanopore defining a nanoreactor; wherein the nanoreactor comprises an internal volume within the nanopore and/or a nanovolume extending from one or more openings of the nanopore;
wherein the movement of the polynucleotide strand with respect to the nanopore is controlled using a polynucleotide-handling enzyme;
and
(B) selectively modifying portions of the polynucleotide strand as they move through the nanoreactor;
wherein the pattern of selective modifications on the polynucleotide strand encodes data on the strand;
wherein selectively modifying portions of the polynucleotide strand comprises subjecting the portions of the polynucleotide strand in the nanoreactor to reaction conditions comprising:
(i) the presence, absence or concentration of one or more chemical reagent(s), wherein (a) said chemical reagents comprise at least a first reagent and a second reagent and the first and second reagents react with the portion of the polynucleotide strand in the nanoreactor, and/or (b) the chemical reagents selectively or non-selectively modify the backbone or the sugar of polynucleotides in the nanoreactor;
(ii) the engagement of an enzyme with the polynucleotide strand under conditions that the enzyme modifies the nucleotides within the polynucleotide strand;
(iii) the presence or absence of electromagnetic radiation; and/or
(iv) the presence or absence of applied heat.

2. A method of encoding data on a polynucleotide strand, comprising:
(A) moving the polynucleotide strand with respect to a nanopore defining a nanoreactor; wherein the nanoreactor comprises an internal volume within the nanopore and/or a nanovolume extending from one or more openings of the nanopore;
wherein the movement of the polynucleotide strand with respect to the nanopore is controlled using a polynucleotide-handling enzyme which is a helicase;
and
(B) selectively modifying portions of the polynucleotide strand as they move through the nanoreactor;
wherein the pattern of selective modifications on the polynucleotide strand encodes data on the strand.

3. A method according to claim 2 wherein selectively modifying portions of the polynucleotide strand comprises subjecting the portions of the polynucleotide strand in the nanoreactor to reaction conditions comprising (i) the presence, absence or concentration of one or more chemical reagent(s); (ii) the engagement of an enzyme with the polynucleotide strand under conditions that the enzyme modifies the nucleotides within the polynucleotide strand; (iii) the presence or absence of electromagnetic radiation; and/or (iv) the presence or absence of applied heat.

4. A method according to any one of the preceding claims wherein selectively modifying portions of the polynucleotide strand comprises:
(i) scission of one or more side chains on the polynucleotide strand;
(ii) modification of one or more side chains on the strand; and/or
(iii) addition of pendant chemical groups to the backbone or sidechains of the residues on the polynucleotide strand.

5. A method according to claim 1, 3 or 4, wherein selectively modifying portions of the polynucleotide strand comprises subjecting the portions of the polynucleotide strand in the nanoreactor to reaction conditions comprising the presence, absence or concentration of one or more chemical reagent(s); wherein (a) said chemical reagents comprise at least a first reagent and a second reagent and the first and second reagents react with the portion of the polynucleotide strand in the nanoreactor, and/or (b) the chemical reagents selectively or non-selectively modify the backbone or the sugar of polynucleotides in the nanoreactor;
optionally wherein controlling the presence, absence or concentration of one or more chemical reagent(s) in the nanoreactor comprises applying an electrical or chemical potential across the nanoreactor.

6. A method according to claim 1 or 3 to 5 wherein one or more protectant(s) are provided external to the nanoreactor to minimise or prevent modification of the polynucleotide strand by chemical reagent(s) external to the nanoreactor.

7. A method according to any one of the preceding claims, wherein selectively modifying portions of the polynucleotide strand comprises contacting the portions of the polynucleotide in the nanoreactor with a polynucleotide-processing enzyme;
optionally wherein controlling the modification of the polynucleotide strand by the polynucleotide-processing enzyme comprises (i) controlling the presence, absence or concentration of fuel and/or substrate for the enzyme; and/or (ii) controlling a force exerted on the polynucleotide strand.

8. A method according to any one of the preceding claims, wherein selectively modifying portions of the polynucleotide strand comprises:
(i) contacting the portions of the polynucleotide strand in the nanoreactor with electromagnetic radiation in the form of light, preferably visible or ultraviolet light; and/or
(ii) irradiating a photosensitizer, preferably a metal nanoparticle, in the nanoreactor for modification of the polynucleotide strand;
optionally thereby transferring electromagnetic radiation or thermal radiation from the sensitizer to the polynucleotide strand in the nanoreactor for modification of the polynucleotide strand.

9. A method according to any one of the preceding claims, wherein the polynucleotide strand is single-stranded or double-stranded.

10. A method according to any one of the preceding claims, wherein the portions of the polynucleotide strand which are selectively modified are sequential portions of the polynucleotide strand;wherein the portions of the polynucleotide strand which are selectively modified correspond to data units thereby encoded and/or wherein selectively modifying portions of the polynucleotide strand does not comprise adding or removing monomer units from the polynucleotide strand such that the overall length of the polynucleotide strand is unaltered.

11. A method according to any one of the preceding claims wherein:
(a) the portions of the polynucleotide strand which are selectively modified in the nanoreactor comprise:
- from 1 to about 1000 nucleotides; or
- from 1 to about 100 nucleotides; or
- from 1 to about 10 nucleotides;
and/or
(b) said method comprises selectively modifying from about 10¹ to about 10⁹ portions of the polynucleotide strand as they move through the nanoreactor.

12. A method according to any one of the preceding claims, comprising:
A: continuously moving the polynucleotide strand with respect to the nanoreactor whilst selectively modifying the portions of the polynucleotide strand as they move through the nanoreactor;
optionally wherein said method comprises:
(i) moving the polynucleotide strand at a constant rate with respect to the nanoreactor, and applying reaction conditions at a regular or irregular frequency to the polynucleotide strand as it moves through the nanoreactor;
or
(ii) moving the polynucleotide strand at a variable rate with respect to the nanoreactor, and applying reaction conditions at a regular or irregular frequency to the polynucleotide strand as it moves through the nanoreactor;
or
B: interrupting the movement of the polynucleotide strand with respect to the nanoreactor whilst selectively modifying the portions of the polynucleotide strand as they move through the nanoreactor.

13. A method according to any one of the preceding claims wherein selectively modifying portions of the polynucleotide strand comprises selectively controlling the number of nucleotides that are modified within each portion of the polynucleotide strand and/or selectively controlling the extent of the modifications made to the nucleotides that are modified within each portion of the polynucleotide strand.

14. A method according to any one of the preceding claims wherein the nanopore is a transmembrane protein nanopore, a solid state nanopore, a DNA-origami pore, or a polymer-based plastic pore; or wherein the nanopore comprises a well, gap, channel, tube, trench or slit in a membrane or across a membrane;
optionally wherein the nanopore is a transmembrane β-barrel protein pore.

15. A method according to any one of the preceding claims, further comprising:
(i) the step of:
(C) determining the pattern of selective modifications on the polynucleotide strand; thereby reading data encoded on the polynucleotide strand;
optionally wherein determining the pattern of selective modifications on the polynucleotide strand comprises determining the presence, absence, extent or properties of modifications made to the polynucleotide strand;
and/or
(ii) characterizing the modified polynucleotide strand.

16. A method according to claim 15 wherein determining the pattern of selective modifications on the polynucleotide strand and/or characterizing the modified polynucleotide strand comprises:
i) contacting a detector with the modified polynucleotide strand such that the polynucleotide strand moves with respect to the detector; and
ii) taking one or more measurements which are indicative of one or more properties of the modified polynucleotide strand as the polynucleotide strand moves with respect to the detector;
optionally wherein the detector is a transmembrane pore and said method comprises:
a) contacting the pore with the modified polynucleotide strand;
b) applying a potential difference across the pore; and
c) taking one or more measurements, preferably one or more current measurements, which are indicative of one or more properties of the modified polynucleotide strand moving with respect to the pore and thereby determining the pattern of selective modifications on the polynucleotide strand and/or characterizing the modified polynucleotide strand.

17. A method according to claim 16, wherein
a) the nanoreactor comprises a nanopore; and
b) determining the pattern of selective modifications on the polynucleotide strand and/or characterizing the modified polynucleotide comprises (i) contacting the same nanopore with the modified polynucleotide strand such that the polynucleotide strand moves with respect to the nanopore; and (ii) taking one or more measurements which are indicative of one or more properties of the modified polynucleotide strand as the polynucleotide strand moves with respect to the nanopore.

18. A method of modifying a polynucleotide strand, comprising:
(A) moving the polynucleotide strand with respect to a nanopore defining a nanoreactor; wherein the nanoreactor comprises an internal volume within the nanopore and/or a nanovolume extending from one or more openings of the nanopore; wherein the movement of the polynucleotide strand with respect to the nanopore is controlled using a polynucleotide-handling enzyme; and
(B) selectively modifying portions of the polynucleotide strand as they move through the nanoreactor;
wherein selectively modifying portions of the polynucleotide strand comprises subjecting the portions of the polynucleotide strand in the nanoreactor to reaction conditions comprising:
(i) the presence, absence or concentration of one or more chemical reagent(s), wherein (a) said chemical reagents comprise at least a first reagent and a second reagent and the first and second reagents react with the portion of the polynucleotide strand in the nanoreactor, and/or (b) the chemical reagents selectively or non-selectively modify the backbone or the sugar of polynucleotides in the nanoreactor;
(ii) the engagement of an enzyme with the polynucleotide strand under conditions that the enzyme modifies the nucleotides within the polynucleotide strand;
(iii) the presence or absence of electromagnetic radiation; and/or
(iv) the presence or absence of applied heat;
wherein optionally the method, polynucleotide strand, nanoreactor and/or selective modifications made are as defined in any one of claims 3 to 17.
